# EUROPEAN PATENT APPLICATION

(11) **EP 4 239 068 A1**
(43) Date of publication of application: **06.09.2023**
(21) Application number: 21886298.5
(22) Date of filing: 28.10.2021
(51) Int. Cl.: C12N 15/13, A61K 31/7088, A61K 36/06, A61K 39/395, A61P 31/14, C07K 16/10, C07K 16/46, C12N 1/15, C12N 1/19, C12N 1/21, C12N 5/10, C12N 15/62, C12P 21/08, G01N 33/53, G01N 33/543

(54) **ANTIBODY CAPABLE OF BINDING SPECIFICALLY TO NUCLEOCAPSID PROTEIN OF SEVERE ACUTE RESPIRATORY SYNDROME CORONAVIRUS-2 OR FRAGMENT THEREOF, AND USE OF SAID ANTIBODY OR SAID FRAGMENT**

(30) Priority: 30.10.2020 JP 2020183273
(71) Applicant: TOYOBO CO., LTD., Osaka-shi Osaka 5300001 (JP); National University Corporation University Of Toyama, Toyama-shi, Toyama 930-8555 (JP)
(72) Inventor: KAWAHATA, Takashi, Tsuruga-shi, Fukui 914-8550 (JP); MASUYA, Takahiro, Tsuruga-shi, Fukui 914-8550 (JP); YUHARA, Kosuke, Tsuruga-shi, Fukui 914-8550 (JP); NISHIMURA, Kengo, Otsu-shi, Shiga 520-0292 (JP); KITAZAWA, Hiroaki, Tsuruga-shi, Fukui 914-8550 (JP); KUROITA, Toshihiro, Osaka-shi, Osaka 530-8230 (JP); ISOBE, Masaharu, Toyama-shi, Toyama 930-8555 (JP); KUROSAWA, Nobuyuki, Toyama-shi, Toyama 930-8555 (JP)
(74) Representative: Vossius & Partner Patentanwälte Rechtsanwälte mbB
(86) International application number: PCT/JP2021/039754
(87) International publication number: WO 2022/092181

(57) **Abstract**

Provided are an antibody or fragment thereof specifically bindable to the NTD or CTD of the N protein of SARS-CoV-2, and use of the antibody or fragment thereof. An antibody or fragment thereof specifically bindable to the amino acid sequence of SEQ ID NO: 1 or 2 is disclosed.

Sequence Listing

<110>  TOYOBO CO., LTD
       NATIONAL UNIVERSITY CORPORATION UNIVERSITY OF TOYAMA

<120>  Antibody specifically binding to nucleocapsid protein of Severe
       Acute Respiratory Syndrome Coronavirus 2 or its fragment and use
       of the same

<130>  P21-084WO

<150>  JP 2020-183273
<151>  2020-10-30

<160>  152

<170>  PatentIn version 3.5

<210>  1
<211>  172
<212>  PRT
<213>  Homo sapiens

<400>  1

Met Ser Asp Asn Gly Pro Gln Asn Gln Arg Asn Ala Pro Arg Ile Thr
1               5                   10                  15

Phe Gly Gly Pro Ser Asp Ser Thr Gly Ser Asn Gln Asn Gly Glu Arg
            20                  25                  30

Ser Gly Ala Arg Ser Lys Gln Arg Arg Pro Gln Gly Leu Pro Asn Asn
        35                  40                  45

Thr Ala Ser Trp Phe Thr Ala Leu Thr Gln His Gly Lys Glu Asp Leu
    50                  55                  60

Lys Phe Pro Arg Gly Gln Gly Val Pro Ile Asn Thr Asn Ser Ser Pro
65                  70                  75                  80

Asp Asp Gln Ile Gly Tyr Tyr Arg Arg Ala Thr Arg Arg Ile Arg Gly
                85                  90                  95

Gly Asp Gly Lys Met Lys Asp Leu Ser Pro Arg Trp Tyr Phe Tyr Tyr
            100                 105                 110

Leu Gly Thr Gly Pro Glu Ala Gly Leu Pro Tyr Gly Ala Asn Lys Asp
        115                 120                 125

Gly Ile Ile Trp Val Ala Thr Glu Gly Ala Leu Asn Thr Pro Lys Asp
    130                 135                 140

His Ile Gly Thr Arg Asn Pro Ala Asn Asn Ala Ala Ile Val Leu Gln
145                 150                 155                 160

Leu Pro Gln Gly Thr Thr Leu Pro Lys Gly Phe Tyr
                165                 170

<210>  2

<211>  173
<212>  PRT
<213>  Homo sapiens

<400>  2

Thr Lys Lys Ser Ala Ala Glu Ala Ser Lys Lys Pro Arg Gln Lys Arg
1               5                   10                  15

Thr Ala Thr Lys Ala Tyr Asn Val Thr Gln Ala Phe Gly Arg Arg Gly
            20                  25                  30

Pro Glu Gln Thr Gln Gly Asn Phe Gly Asp Gln Glu Leu Ile Arg Gln
        35                  40                  45

Gly Thr Asp Tyr Lys His Trp Pro Gln Ile Ala Gln Phe Ala Pro Ser
    50                  55                  60

Ala Ser Ala Phe Phe Gly Met Ser Arg Ile Gly Met Glu Val Thr Pro
65                  70                  75                  80

Ser Gly Thr Trp Leu Thr Tyr Thr Ala Ala Ile Lys Leu Asp Asp Lys
                85                  90                  95

Asp Pro Asn Phe Lys Asp Gln Val Ile Leu Leu Asn Lys His Ile Asp
            100                 105                 110

Ala Tyr Lys Thr Phe Pro Pro Thr Glu Pro Lys Lys Asp Lys Lys Lys

        115                 120                 125

Lys Ala Asp Glu Thr Gln Ala Leu Pro Gln Arg Gln Lys Lys Gln Gln
    130                 135                 140

Thr Val Thr Leu Leu Pro Ala Ala Asp Leu Asp Asp Phe Ser Lys Gln
145                 150                 155                 160

Leu Gln Gln Ser Met Ser Ser Ala Asp Ser Thr Gln Ala
                165                 170

<210> 3
<211> 8
<212> PRT
<213> Guinea pig

<400> 3

Gly Phe Thr Phe Asp Asp Tyr Gly
1               5

<210> 4
<211> 8
<212> PRT
<213> Guinea pig

<400> 4

Gly Phe Thr Phe Ser Asp Phe Trp
1               5

<210> 5
<211> 8
<212> PRT
<213> Guinea pig

<400> 5

Gly Phe Thr Phe Asn Asn Tyr Trp
1               5

<210> 6
<211> 8
<212> PRT
<213> Guinea pig

<400> 6

Gly Phe Thr Phe Asp Asp Phe Gly
1               5

<210> 7
<211> 8
<212> PRT
<213> Guinea pig

<400> 7

Gly Phe Thr Phe Ser Asn Tyr Tyr
1               5

<210>  8
<211>  8
<212>  PRT
<213>  Guinca pig

<400>  8

Gly Phe Thr Phe Ser Asp Tyr Trp
1               5

<210>  9
<211>  8
<212>  PRT
<213>  Guinea pig

<400>  9

Gly Phe Ser Leu Thr Ser Tyr Ser
1               5

<210>  10
<211>  8
<212>  PRT
<213>  Guinea pig

<400>  10

Gly Phe Thr Phe Ser Asp Tyr Thr
1               5

<210>  11
<211>  10

<212> PRT
<213> Guinea pig

<400> 11

Gly Phe Ser Ile Thr Thr Asn Gly Tyr Thr
1               5                   10

<210> 12
<211> 8
<212> PRT
<213> Guinea pig

<400> 12

Ile Ser Tyr Ser Gly Gly Arg Thr
1               5

<210> 13
<211> 8
<212> PRT
<213> Guinea pig

<400> 13

Ile Asn Tyr Asp Gly Asp Arg Thr
1               5

<210> 14
<211> 8
<212> PRT
<213> Guinea pig

<400>  14

Ile Asn Gly Asp Ser Thr Lys Ile
1               5

<210>  15
<211>  8
<212>  PRT
<213>  Guinea pig

<400>  15

Ile Asp Tyr Ser Gly Gly Thr Thr
1               5

<210>  16
<211>  8
<212>  PRT
<213>  Guinea pig

<400>  16

Ile Ser Tyr Ser Gly Gly Ser Thr
1               5

<210>  17
<211>  8
<212>  PRT
<213>  Guinea pig

<400>  17

Ile Ser Gly Asp Ser Asn Tyr Ile
1               5

<210>  18
<211>  8
<212>  PRT
<213>  Guinea pig

<400>  18

Ile His Pro Asp Gly Gly Ser Thr
1               5

<210>  19
<211>  8
<212>  PRT
<213>  Guinea pig

<400>  19

Ile Ser Pro Asp Gly Gly Gly Thr
1               5

<210>  20
<211>  8
<212>  PRT
<213>  Guinea pig

<400>  20

Ile Ser Pro Ser Gly Gly Ser Thr

1                 5

<210>  21
<211>  8
<212>  PRT
<213>  Guinea pig

<400>  21

Ile Ser Tyr Ser Gly Gly Asn Thr
1                 5

<210>  22
<211>  8
<212>  PRT
<213>  Guinea pig

<400>  22

Ile Trp Tyr Asp Gly Thr Lys Met
1                 5

<210>  23
<211>  8
<212>  PRT
<213>  Guinea pig

<400>  23

Ile Ser Gly Asp Thr Asn Tyr Ile
1                 5

<210> 24
<211> 7
<212> PRT
<213> Guinea pig

<400> 24

Ile Trp Tyr Asp Ala Asp Thr
1               5

<210> 25
<211> 7
<212> PRT
<213> Guinea pig

<400> 25

Ile Trp Ser Gly Gly Arg Ile
1               5

<210> 26
<211> 15
<212> PRT
<213> Guinea pig

<400> 26

Ala Arg Asp Gly Gly Ser Tyr Tyr Ser Pro Ile Asn Phe Gln Phe
1               5                   10                  15

<210> 27

<211>  15
<212>  PRT
<213>  Guinea pig

<400>  27

Ser Arg Asp Gly Gly Ser Tyr His Ser Pro Ile Ser Cys Gln Tyr
1               5                   10                  15

<210>  28
<211>  15
<212>  PRT
<213>  Guinea pig

<400>  28

Ala Arg Asp Gly Gly Ser Tyr Ser Ser Pro Ile Asn Phe Gln Tyr
1               5                   10                  15

<210>  29
<211>  15
<212>  PRT
<213>  Guinea pig

<400>  29

Ser Arg Asp Gly Gly Ser Tyr Ser Ser Pro Ile Asn Phe Gln Tyr
1               5                   10                  15

<210>  30
<211>  15
<212>  PRT

<213>  Guinea pig

<400>  30

Ala Arg Asp Gly Gly Ser Val Thr Ser Pro Ile Tyr Phe Gln Val
1               5                   10                  15

<210>  31
<211>  15
<212>  PRT
<213>  Guinea pig

<400>  31

Ala Arg Asp Gly Gly Ser Val Lys Ser Pro Ile Tyr Phe Gln Tyr
1               5                   10                  15

<210>  32
<211>  8
<212>  PRT
<213>  Guinea pig

<400>  32

Ala Thr Asn Tyr Asn Phe Gln Tyr
1               5

<210>  33
<211>  8
<212>  PRT
<213>  Guinea pig

<400>  33

Ala Thr Ser Gly Pro Met Asp Val
1               5

<210>  34
<211>  19
<212>  PRT
<213>  Guinea pig

<400>  34

Ala Arg Ser Ile Ile Ala Glu Ile Pro Thr Ile Phe Thr Phe Pro Pro
1               5                   10                  15

Phe Asp Val

<210>  35
<211>  11
<212>  PRT
<213>  Guinea pig

<400>  35

Ala Arg Leu Trp Ser Gly Tyr Ala Leu Asp Ile
1               5                   10

<210>  36
<211>  13
<212>  PRT

<213>  Guinea pig

<400>  36

Ala Lys Tyr Tyr Asp Tyr Asp Tyr Thr Gly Phe Asp Val
1               5                   10

<210>  37
<211>  14
<212>  PRT
<213>  Guinea pig

<400>  37

Val Ser Pro Leu Leu Val Val His Gly Pro Phe Phe Gln Tyr
1               5                   10

<210>  38
<211>  14
<212>  PRT
<213>  Guinea pig

<400>  38

Val Ser Pro Leu Leu Val Tyr His Gly Pro Phe Phe Gln Tyr
1               5                   10

<210>  39
<211>  14
<212>  PRT
<213>  Guinea pig

<400>  39

Val Lys Pro Leu Leu Val Leu His Gly Pro Phe Phe Gln Ser
1               5                   10

<210>  40
<211>  10
<212>  PRT
<213>  Guinea pig

<400>  40

Val Ser Pro Ser Gly Ala Gly Leu Asp Val
1               5                   10

<210>  41
<211>  9
<212>  PRT
<213>  Guinea pig

<400>  41

Ser Pro Ser Ala Val Gly Phe Asp Val
1               5

<210>  42
<211>  7
<212>  PRT
<213>  Guinea pig

<400>  42

Ser Glu Tyr Lys His Tyr Asn
1               5

<210>  43
<211>  7
<212>  PRT
<213>  Guinea pig

<400>  43

Ser Glu His Arg Ser Tyr Tyr
1               5

<210>  44
<211>  7
<212>  PRT
<213>  Guinea pig

<400>  44

Ser Asp Tyr Ser His Tyr Ser
1               5

<210>  45
<211>  7
<212>  PRT
<213>  Guinea pig

<400>  45

Ser Gln Gly Ile Asn Lys Trp
1               5

<210> 46
<211> 7
<212> PRT
<213> Guinea pig

<400> 46

Ser Gly His Ser Ser Tyr Tyr
1               5

<210> 47
<211> 7
<212> PRT
<213> Guinea pig

<400> 47

Ser Glu His Ser Ser Tyr Tyr
1               5

<210> 48
<211> 7
<212> PRT
<213> Guinea pig

<400> 48

Ser Asp His Ser Ser Tyr Tyr
1               5

<210>  49
<211>  13
<212>  PRT
<213>  Guinca pig

<400>  49

Ser Gln Ser Leu Phe Tyr Ser Gly Lys Asn Lys Asp Leu
1               5                   10

<210>  50
<211>  6
<212>  PRT
<213>  Guinea pig

<400>  50

Asn Ile Gly Gly Lys Thr
1               5

<210>  51
<211>  7
<212>  PRT
<213>  Guinea pig

<400>  51

Leu Lys Ser Asp Gly Ser His
1               5

<210>  52
<211>  7

<212>  PRT
<213>  Guinea pig

<400>  52

Val Lys Ser Asp Gly Ser His
1               5

<210>  53
<211>  7
<212>  PRT
<213>  Guinea pig

<400>  53

Ile Glu Ser Asp Gly Ser His
1               5

<210>  54
<211>  7
<212>  PRT
<213>  Guinea pig

<400>  54

Val Lys Ser Asp Gly Ser Leu
1               5

<210>  55
<211>  7
<212>  PRT
<213>  Guinea pig

<400> 55

Leu Asn Ser Tyr Gly Ser His
1               5

<210> 56
<211> 7
<212> PRT
<213> Guinea pig

<400> 56

Leu Asn Ser Lys Gly Ser His
1               5

<210> 57
<211> 7
<212> PRT
<213> Guinea pig

<400> 57

Ile Glu Ser Lys Gly Ser His
1               5

<210> 58
<211> 8
<212> PRT
<213> Guinea pig

<400> 58

Val Gly Thr Ser Asp Ile Val Gly
1               5

<210>  59
<211>  10
<212>  PRT
<213>  Guinea pig

<400>  59

Gly Val Ser Tyr Ser Gly Gly His Asn Ile
1               5                   10

<210>  60
<211>  10
<212>  PRT
<213>  Guinea pig

<400>  60

Gly Val Ser Phe Ser Gly Gly His Gly Val
1               5                   10

<210>  61
<211>  10
<212>  PRT
<213>  Guinea pig

<400>  61

Gly Val Ser Tyr Ser Gly Gly His Asn Val

1                   5                   10

<210>  62
<211>  10
<212>  PRT
<213>  Guinea pig

<400>  62

Gly Val Ser Val Ser Gly Gly His Asn Leu
1                   5                   10

<210>  63
<211>  10
<212>  PRT
<213>  Guinea pig

<400>  63

Gly Val Ser Leu Lys Gly Gly His Asn Leu
1                   5                   10

<210>  64
<211>  11
<212>  PRT
<213>  Guinea pig

<400>  64

Gly Ala Asp Tyr Ser Gly Ala Ser Ser Tyr Val
1                   5                   10

<210> 65
<211> 13
<212> PRT
<213> Guinea pig

<400> 65

Gly Ala Asn His Asn Ile Gly Glu Ser Tyr Gly Tyr Val
1               5                   10

<210> 66
<211> 9
<212> PRT
<213> Guinea pig

<400> 66

Gly Val Gly Tyr Asn Asn Lys Tyr Val
1               5

<210> 67
<211> 9
<212> PRT
<213> Guinea pig

<400> 67

Gly Val Gly Tyr Ser Gly Gly Tyr Val
1               5

<210> 68

<211>  9
<212>  PRT
<213>  Guinea pig

<400>  68

Gly Val Ser Tyr Ser Gly Gly Tyr Val
1               5

<210>  69
<211>  9
<212>  PRT
<213>  Guinea pig

<400>  69

Gly Val Lys Tyr Ser Gly Gly Val Val
1               5

<210>  70
<211>  9
<212>  PRT
<213>  Guinea pig

<400>  70

Gly Val Ser Tyr Ser Gly Gly Tyr Tyr
1               5

<210>  71
<211>  9
<212>  PRT

<213>  Guinea pig

<400>  71

Gln Tyr Asp Ser Thr Pro Pro Leu Thr
1               5

<210>  72
<211>  9
<212>  PRT
<213>  Guinea pig

<400>  72

Gln Val Trp Asp Ser Tyr Thr Tyr Val
1               5

<210>  73
<211>  8
<212>  PRT
<213>  Guinea pig

<400>  73

Gln Phe Ile Asn Gly Pro Leu Thr
1               5

<210>  74
<211>  8
<212>  PRT
<213>  Guinea pig

<400> 74

Gly Phe Ile Phe Ser Asn Tyr Phe
1               5

<210> 75
<211> 8
<212> PRT
<213> Guinea pig

<400> 75

Gly Phe Thr Phe Asn Thr Tyr Thr
1               5

<210> 76
<211> 8
<212> PRT
<213> Guinea pig

<400> 76

Gly Phe Thr Phe Ser Asn Tyr Tyr
1               5

<210> 77
<211> 8
<212> PRT
<213> Guinea pig

<400> 77

Gly Leu Ser Leu Thr Asn Tyr Thr
1               5

<210>  78
<211>  8
<212>  PRT
<213>  Guinea pig

<400>  78

Gly Phe Ser Phe Ser Asn Ser Trp
1               5

<210>  79
<211>  8
<212>  PRT
<213>  Guinea pig

<400>  79

Gly Phe Thr Phe Ser Asn Tyr Trp
1               5

<210>  80
<211>  8
<212>  PRT
<213>  Guinea pig

<400>  80

Gly Phe Thr Phe Arg Asn His Trp
1               5

<210> 81
<211> 8
<212> PRT
<213> Guinea pig

<400> 81

Gly Phe Thr Phe Ser Asn His Trp
1               5

<210> 82
<211> 8
<212> PRT
<213> Guinea pig

<400> 82

Gly Phe Phe Phe Ser Asn Ser Trp
1               5

<210> 83
<211> 8
<212> PRT
<213> Guinea pig

<400> 83

Gly Phe Thr Phe Asp Asp Tyr Gly
1               5

<210> 84
<211> 6
<212> PRT
<213> Guinca pig

<400> 84

Lys Leu Ser Glu Arg Tyr
1               5

<210> 85
<211> 8
<212> PRT
<213> Guinea pig

<400> 85

Ile Ser Gly Asp Ser Thr Tyr Ile
1               5

<210> 86
<211> 8
<212> PRT
<213> Guinea pig

<400> 86

Ile Gly Asn Ser Gly Ser Lys Thr
1               5

<210> 87
<211> 8

<212>  PRT
<213>  Guinea pig

<400>  87

Ile Ser Gly Asp Gly Ser Asn Ile
1               5

<210>  88
<211>  8
<212>  PRT
<213>  Guinea pig

<400>  88

Ile Ser Gly Asp Ser Ser Asn Ile
1               5

<210>  89
<211>  8
<212>  PRT
<213>  Guinea pig

<400>  89

Ile Lys Gly Asp Ser Ser Ile Ile
1               5

<210>  90
<211>  8
<212>  PRT
<213>  Guinea pig

<400>  90

Ile Asn Pro Asp Asp Gly Ser Pro
1               5

<210>  91
<211>  8
<212>  PRT
<213>  Guinea pig

<400>  91

Ile Lys Gly Asp Ser Ser Ile Thr
1               5

<210>  92
<211>  8
<212>  PRT
<213>  Guinea pig

<400>  92

Ile Asn Ser Asp Gly Ser Asn Thr
1               5

<210>  93
<211>  8
<212>  PRT
<213>  Guinea pig

<400>  93

Ile Asn Thr Asp Gly Ser Ser Thr
1               5

<210>  94
<211>  8
<212>  PRT
<213>  Guinea pig

<400>  94

Ile Lys Gly Asp Ser Arg Thr Ile
1               5

<210>  95
<211>  8
<212>  PRT
<213>  Guinea pig

<400>  95

Ile Ser Gly Ala Ser Ser Asn Ile
1               5

<210>  96
<211>  8
<212>  PRT
<213>  Guinea pig

<400>  96

Ile Ser Gly Asp Ser Ser Asn Val

1                   5

<210>  97
<211>  8
<212>  PRT
<213>  Guinea pig

<400>  97

Ile Lys Gly Asp Ser Ser Thr Ile
1                   5

<210>  98
<211>  8
<212>  PRT
<213>  Guinea pig

<400>  98

Ile Asn Ser Asp Gly Ser Ser Thr
1                   5

<210>  99
<211>  8
<212>  PRT
<213>  Guinea pig

<400>  99

Ile Gly Tyr Ser Gly Ala Ser Ile
1                   5

<210> 100
<211> 8
<212> PRT
<213> Guinea pig

<400> 100

Ile Ser Gly Thr Ser Ser Asn Ile
1               5

<210> 101
<211> 7
<212> PRT
<213> Guinea pig

<400> 101

Met Trp Ser Asp Gly Asp Thr
1               5

<210> 102
<211> 7
<212> PRT
<213> Guinea pig

<400> 102

Thr Ile Asn Trp Gly Asp Val
1               5

<210> 103

<211>  7
<212>  PRT
<213>  Guinea pig

<400>  103

Ser Thr Leu Gly Val Gly Val
1               5

<210>  104
<211>  7
<212>  PRT
<213>  Guinea pig

<400>  104

Thr Thr Leu Gly Val Gly Val
1               5

<210>  105
<211>  7
<212>  PRT
<213>  Guinea pig

<400>  105

Ser Thr Thr Gly Phe Asp Val
1               5

<210>  106
<211>  7
<212>  PRT

<213>  Guinea pig

<400>  106

Ser Thr Leu Gly Asn Phe Leu
1               5

<210>  107
<211>  7
<212>  PRT
<213>  Guinea pig

<400>  107

Thr Ile Thr Trp Gly Asp Val
1               5

<210>  108
<211>  7
<212>  PRT
<213>  Guinea pig

<400>  108

Thr Ile Asp Trp Gly Asn Val
1               5

<210>  109
<211>  19
<212>  PRT
<213>  Guinea pig

<400>  109

Ala Arg Thr Ser Arg Ile Val Ala Asp Leu Val Thr Met Gly Tyr Ala
1               5                   10                  15

Leu Asp Phe

<210>  110
<211>  16
<212>  PRT
<213>  Guinea pig

<400>  110

Ala Arg Asp Val Val Lys Thr Gly Thr Thr Gly Leu Pro Phe Asp Leu
1               5                   10                  15

<210>  111
<211>  14
<212>  PRT
<213>  Guinea pig

<400>  111

Ala Arg Glu Gly Val Ile Thr Val Gly Thr Trp Gly Asp Val
1               5                   10

<210>  112
<211>  5
<212>  PRT

<213>  Guinea pig

<400>  112

Ala Thr Ser Glu Tyr
1               5

<210>  113
<211>  6
<212>  PRT
<213>  Guinea pig

<400>  113

Thr Thr Ala Thr Asp Val
1               5

<210>  114
<211>  6
<212>  PRT
<213>  Guinea pig

<400>  114

Thr Pro Ala Thr Asp Val
1               5

<210>  115
<211>  10
<212>  PRT
<213>  Guinea pig

<400> 115

Ser Pro Met Thr Ile His Gly Leu Asp Thr
1               5                   10

<210> 116
<211> 5
<212> PRT
<213> Guinea pig

<400> 116

Gly Ser Gly Trp Val
1               5

<210> 117
<211> 5
<212> PRT
<213> Guinea pig

<400> 117

Gly Ser Gly Trp Tyr
1               5

<210> 118
<211> 10
<212> PRT
<213> Guinea pig

<400> 118

Leu Ser Ser Glu Ser Asp Asp Ala Arg Val
1               5                   10

<210>  119
<211>  6
<212>  PRT
<213>  Guinea pig

<400>  119

Lys Leu Ser Asn Gln Tyr
1               5

<210>  120
<211>  6
<212>  PRT
<213>  Guinea pig

<400>  120

Lys Leu Ser Glu Arg Tyr
1               5

<210>  121
<211>  6
<212>  PRT
<213>  Guinea pig

<400>  121

Lys Leu Ser Lys Glu Tyr
1               5

<210> 122
<211> 6
<212> PRT
<213> Guinea pig

<400> 122

Ala Leu Pro Lys Lys Tyr
1               5

<210> 123
<211> 6
<212> PRT
<213> Guinea pig

<400> 123

Lys Leu Ser Thr Gln Tyr
1               5

<210> 124
<211> 6
<212> PRT
<213> Guinea pig

<400> 124

Lys Leu Ser Asp Gln Tyr
1               5

<210> 125
<211> 12
<212> PRT
<213> Guinca pig

<400> 125

Ser Glu Ser Leu Leu His Ser Asp Gly Asn Thr Tyr
1               5                   10

<210> 126
<211> 12
<212> PRT
<213> Guinea pig

<400> 126

Ser Gln Ser Leu Leu His Ser Asp Gly Lys Thr Tyr
1               5                   10

<210> 127
<211> 12
<212> PRT
<213> Guinea pig

<400> 127

Ser Gln Ser Leu Val His Ser Asp Gly Lys Thr Tyr
1               5                   10

<210> 128
<211> 12

<212>  PRT
<213>  Guinea pig

<400>  128

Ser Gln Ser Leu Leu Asp Ser Asp Gly Tyr Thr Tyr
1               5                   10

<210>  129
<211>  12
<212>  PRT
<213>  Guinea pig

<400>  129

Ser Gln Ser Leu Leu Tyr Ser Asp Gly Tyr Thr Tyr
1               5                   10

<210>  130
<211>  12
<212>  PRT
<213>  Guinea pig

<400>  130

Ser Gln Ser Leu Leu Tyr Ser Asp Gly Tyr Thr Phe
1               5                   10

<210>  131
<211>  12
<212>  PRT
<213>  Guinea pig

<400> 131

Ser Gln Ser Leu Leu His Ser Asp Gly Asn Thr Tyr
1               5                   10

<210> 132
<211> 12
<212> PRT
<213> Guinea pig

<400> 132

Ser Gln Ser Leu Leu His Gly Asp Gly Lys Thr Tyr
1               5                   10

<210> 133
<211> 6
<212> PRT
<213> Guinea pig

<400> 133

Asn Ile Gly Gly Lys Ala
1               5

<210> 134
<211> 11
<212> PRT
<213> Guinea pig

<400> 134

Leu Ser Arg Gln Ser Asp Asp Ala Ala Ser Val
1               5                   10

<210>  135
<211>  11
<212>  PRT
<213>  Guinea pig

<400>  135

Leu Ser Ser Glu Ser Asp Asp Ala Ala Arg Val
1               5                   10

<210>  136
<211>  11
<212>  PRT
<213>  Guinea pig

<400>  136

Leu Ser Arg Glu Ser Asn Asp Ala Ala Trp Val
1               5                   10

<210>  137
<211>  11
<212>  PRT
<213>  Guinea pig

<400>  137

Leu Ser Ala Asp Ser Ser Gly Thr Tyr Arg Val

1                   5                       10

<210>  138
<211>  11
<212>  PRT
<213>  Guinea pig

<400>  138

Leu Ser Arg Gln Ser Asp Thr Ala Ala Ser Leu
1                   5                       10

<210>  139
<211>  11
<212>  PRT
<213>  Guinea pig

<400>  139

Leu Ser Arg Gln Gly Asp Thr Ala Ala Ser Tyr
1                   5                       10

<210>  140
<211>  10
<212>  PRT
<213>  Guinea pig

<400>  140

Leu Ser Gly Glu Ser Asp Asp Ala Trp Val
1                   5                       10

<210> 141
<211> 9
<212> PRT
<213> Guinea pig

<400> 141

Leu Gln Ala Thr His Asp Pro Tyr Thr
1               5

<210> 142
<211> 9
<212> PRT
<213> Guinea pig

<400> 142

Val Gln Thr Thr His Asp Pro Tyr Thr
1               5

<210> 143
<211> 9
<212> PRT
<213> Guinea pig

<400> 143

Leu Gln Ala Thr His Asn Pro Val Ser
1               5

<210> 144

<211>  9
<212>  PRT
<213>  Guinea pig

<400>  144

Leu Gln Ala Thr His Asp Pro Ile Thr
1               5

<210>  145
<211>  9
<212>  PRT
<213>  Guinea pig

<400>  145

Val Gln Thr Ser His Val Pro Leu Thr
1               5

<210>  146
<211>  9
<212>  PRT
<213>  Guinea pig

<400>  146

Val Gln Thr Thr His Val Pro Leu Thr
1               5

<210>  147
<211>  8
<212>  PRT

<213>  Guinea pig

<400>  147

Gln Thr Thr Asn Asp Pro Tyr Thr
1               5

<210>  148
<211>  9
<212>  PRT
<213>  Guinea pig

<400>  148

Gln Val Tyr Asp Ser Ser Ser Phe Val
1               5

<210>  149
<211>  8
<212>  PRT
<213>  Guinea pig

<400>  149

Trp Gln Gly Thr Asp Phe Pro Arg
1               5

<210>  150
<211>  12
<212>  PRT
<213>  Homo sapiens

<400> 150

Leu Leu Pro Ala Ala Asp Leu Asp Asp Phe Ser Lys
1               5                   10

<210> 151
<211> 9
<212> PRT
<213> Homo sapiens

<400> 151

Lys Lys Ala Asp Glu Thr Gln Ala Leu
1               5

<210> 152
<211> 8
<212> PRT
<213> Homo sapiens

<400> 152

Ser Ala Ala Glu Ala Ser Lys Lys
1               5

## Description

### Technical Field

Techniques related to antibodies or fragments thereof specifically bindable to the nucleocapsid protein of severe acute respiratory syndrome coronavirus-2 (SARS-CoV-2), and use of the antibodies or fragments thereof are provided.

### Background Art

SARS-CoV-2 is the causative virus of novel coronavirus infection (COVID-19), which has been rapidly spreading worldwide since early 2020. SARS-CoV-2 is composed of a nucleocapsid and an envelope surrounding the nucleocapsid, which is similar to typical coronaviruses. The nucleocapsid contains the viral genome (RNA) and the nucleocapsid protein (N protein) binding to the viral genome. The envelope contains a lipid and a spike protein (S protein), a membrane protein (M protein), and an envelope protein (E protein) that bind to the lipid.

The N protein is involved in viral core formation, as well as in packaging and transcription of the viral genome. The N protein has a structure in which the N-terminal domain (NTD) and C-terminal domain (CTD) are joined via a linker with a serine (S)/arginine (R)-rich region. NPL 1 discloses that the N protein is heavily phosphorylated, showing mapping of phosphorylated sites. Due to the conservation of the amino acid sequence of the N protein among strains, the N protein is usable as a diagnostic marker.

### Citation List

### Non-patent Literature

NPL 1: Klann et al., Molecular Cell, 80(1), 2020, pp. 164-174

### Summary of Invention

### Technical Problem

The inventors found that because the linker with an S/R-rich region (a large amount of phosphorylated sites) of the N protein of SARS-CoV-2 can be cleaved by apoptosis-inducing cysteine proteases (e.g., caspase 3 and caspase 6) or by autolytic action, even an antibody specifically bindable to the N protein of SARS-CoV-2 may not enable the detection of the N protein of SARS-CoV-2, depending on the site to which the antibody binds.

Thus, the primary object of the present invention is to provide an antibody or fragment thereof specifically bindable to the NTD or CTD of the N protein of SARS-CoV-2, and use of the antibody or fragment thereof.

The present inventors also found that in the method for detecting the N protein of SARS-CoV-2 by forming a sandwich structure with two different antibodies specifically bindable to the N protein, the detection sensitivity for the N protein of SARS-CoV-2 is decreased due to cleavage of the linker with an S/R-rich region hindering sandwich structure formation, for example, if two antibodies for use are an antibody specifically binding to the NTD and an antibody specifically bindable to the CTD.

Thus, the secondary object of the present invention is to provide a kit and a method for detecting the N protein of SARS-CoV-2 with high sensitivity.

### Solution to Problem

The present inventors conducted extensive research to achieve the objects and then found many kinds of antibodies and fragments thereof specifically bindable to the NTD or CTD of the N protein of SARS-CoV-2. The inventors also found that the use of two or more antibodies or fragments thereof that specifically bind to the NTD and that each recognize a different epitope in the NTD and/or two or more antibodies or fragments thereof that specifically bind to the CTD and that each recognize a different epitope in the CTD enables the detection of the N protein with high sensitivity. The inventors conducted further research based on these findings and completed the present invention.

The present invention includes the following aspects.

### Item 1.

An antibody or fragment thereof specifically bindable to the amino acid sequence of SEQ ID NO: 1 or 2 (antigen-binding fragment).

### Item 2.

An antibody or fragment thereof (antigen-binding fragment), comprising
a heavy-chain CDR1 containing the amino acid sequence represented by the following formula (A-1) or (A-2): GFX^{A31}X^{A41}X^{A51}X^{A61}X^{A71}X^{A81} (A-1)
   wherein
   X^{A31} represents T or S,
   X^{A41} represents F, L, or I,
   X^{A51} represents D, S, N, or T,
   X^{A61} represents D, N, S, I, or T,
   X^{A71} represents Y, F, or N,
   X^{A81} represents G, W, Y, S, or T, and
   GFX^{A33}X^{A43}X^{A53}X^{A63}X^{A73}X^{A83}X^{A93}X^{A103} (A-2)
   wherein
   X^{A33} represents T or S,
   X^{A43} represents F, L, or I,
   X^{A53} represents D, S, N, or T,
   X^{A63} represents D, N, S, or T,
   X^{A73} represents N or S,
   X^{A83} represents G or Y,
   X^{A93} represents Y, F, or N,
   X^{A103} represents T, G, W, or Y;
a heavy-chain CDR2 containing the amino acid sequence represented by the following formula (B-1) or (B-2): IX^{B21}X^{B31}X^{B41}X^{B51}X^{B61}X^{B71}X^{B81} (B-1)
   wherein
   X^{B21} represents S, N, D, W, or H,
   X^{B31} represents Y, G, P, or S,
   X^{B41} represents S, D, or G,
   X^{B51} represents G, S, T, or A,
   X^{B61} represents G, D, T, N, or R,
   X^{B71} represents R, K, T, S, N, G, Y, or D,
   X^{B81} represents T, I, or M, and
   IX^{B23}X^{B33}X^{B43}X^{B53}X^{B63}X^{B73} (B-2)
   wherein
   X^{B23} represents S, N, D, W, or H,
   X^{B33} represents Y, G, P, or S,
   X^{B43} represents S, D, or G,
   X^{B53} represents G, S, T, or A,
   X^{B63} represents R, K, T, S, N, G, Y, or D,
   X^{B73} represents T, I, or M;
a heavy-chain CDR3 containing the amino acid sequence represented by any one of the following formulas (C-1) to (C-8) :
   X^{C11}X^{C21}X^{C31}X^{C41}X^{C51}X^{C61}X^{C71}X^{C81}X^{C91}X^{C101}X^{C111}X^{C121}X^{C131}X^{C141}X^{C151} (C-1)
   wherein
   X^{G11} represents A or S,
   X^{C21} represents R, T, or K,
   X^{C31} represents D, N, S, or Y,
   X^{C41} represents G, S, or Y,
   X^{C51} represents G, I, or D,
   X^{C61} represents S or I,
   X^{C71} represents Y, V, or A,
   X^{C81} represents Y, H, S, T, K, or E,
   X^{C91} represents S, I, or D,
   X^{C101} represents P or Y,
   X^{C111}represents I, Y, G, P, T, A, or V,
   X^{C121} represents N, S, Y, P, G, or F,
   X^{C131} represents F, C, M, or L,
   X^{C141} represents Q or D,
   X^{C151} represents F, Y, V, or S,
   X^{C13}X^{C23}X^{C33}X^{C43}X^{C53}X^{C63}X^{C73}X^{C83} (C-2)
   wherein
   X^{C13} represents A or S,
   X^{C23} represents R, T, or K,
   X^{C33} represents D, N, S, or Y,
   X^{C43} represents I, Y, G, P, T, A, or V,
   _{X}^{C53} represents N, S, Y, P, G, or F,
   X^{C63} represents F, C, M, or L,
   X^{C73} represents Q or D,
   X^{C83} represents F, Y, V, or S,
   X^{C15}X^{C25}X^{C35}X^{C45}X^{C55}X^{C65}X^{C75}X^{C85}X^{C95}TIFTFX^{C155}X^{C165}XC¹⁷⁵X^{C185}X^{c195} (C-3 )
   wherein
   X^{C15} represents A or S,
   X^{C25} represents R, T, or K,
   X^{C35} represents G, S, or Y,
   X^{C45} represents G, I, or D,
   X^{C55} represents S or I,
   X^{C65} represents Y, V, or A,
   X^{C75} represents Y, H, S, T, K, or E,
   X^{C85} represents S, I, or D,
   X^{C95} represents P or Y,
   X^{C155} represents I, Y, G, P, T, A, or V,
   X^{C165} represents N, S, Y, P, G, or F,
   X^{C175} represents F, C, M, or L,
   X^{C185} represents Q or D,
   X^{C195} represents F, Y, V, or S,
   ARLWSGYALDI (C-4),
   X^{C16}X^{C26}X^{C36}X^{C46}X^{C56}X^{C66}X^{C76}X^{C86}X^{C96}X^{C106}X^{C116}X^{C126}X^{C136} (C-5)
   wherein
   X^{C16} represents A or S,
   X^{C26} represents R, T, or K,
   X^{C36} represents D, N, S, or Y,
   X^{C46} represents G, S, or Y,
   X^{C56} represents G, I, or D,
   X^{C66} represents Y, H, S, T, K, or E,
   X^{C76} represents S, I, or D,
   X^{C86} represents P or Y,
   X^{C96} represents I, Y, G, P, T, A, or V,
   X^{C106} represents N, S, Y, P, G, or F,
   X^{C116} represents F, C, M, or L,
   X^{C126} represents Q or D,
   X^{C136} represents F, Y, V, or S,
   VX^{C27}PLLVX^{C77}HGX^{C107}X^{C1117}X^{C127}X^{C137}X^{C147} (C₋6)
   wherein
   X^{C27} represents S or K,
   X^{C77} represents V, Y, or L,
   X^{C107} represents I, Y, G, P, T, A, or V,
   X^{C117} represents N, S, Y, P, G, or F,
   X^{C127} represents F, C, M, or L,
   X^{C137} represents Q or D,
   X^{C147} represents F, Y, V, or S,
   VSPSX^{C58}X^{C68}X^{C78}X^{C88}X^{C98}X^{C108} (C-7)
   wherein
   X^{C58} represents G or A,
   X^{C68} represents I, Y, G, P, T, A, or V,
   X^{C78} represents N, S, Y, P, G, or F,
   X^{C88} represents F, C, M, or L,
   X^{C98} represents Q or D,
   X^{C108} represents F, Y, V, or S, and
   SPSX^{C49}X^{C59}X^{C69}X^{C79}X^{C89}X^{C99} (C-8)
   wherein
   X^{C49} represents G or A,
   X^{C59} represents I, Y, G, P, T, A, or V,
   X^{C69} represents N, S, Y, P, G, or F,
   X^{C79} represents F, C, M, or L,
   X^{C89} represents Q or D,
   X^{C99} represents F, Y, V, or S;
a light-chain CDR1 containing the amino acid sequence represented by any one of the following formulas (D-1) to (D-3) :
   SX^{D21}X^{D31}X^{D41}X^{D51}X^{D61}X^{D71} (D-1)
   wherein
   X^{D21} represents E, D, Q, or G,
   X^{D31} represents H, Y, G, or S,
   X^{D41} represents R, S, I, K, or L,
   X^{D51} represents S, H, N, or F,
   X^{D61} represents Y or K,
   X^{D71} represents Y, S, W, or N,
   SX^{D23}X^{D33}X^{D43}X^{D53}X^{D63}X^{D73}GKNKDL (D-2)
   wherein
   X^{D23} represents E, D, Q, or G,
   X^{D33} represents H, Y, G, or S,
   X^{D43} represents R, S, I, K, or L,
   X^{D53} represents S, H, N, or F,
   X^{D63} represents Y or K,
   X^{D73} represents Y, S, W, or N, and
   NIGGKT (D-3);
a light-chain CDR2 containing the amino acid sequence represented by any one of the following formulas (E-1) to (E-3) :
   X^{E11}X^{E21}SX^{E41}GSX^{E71} (E-1)
   wherein
   X^{E11} represents L, V, or I,
   X^{E21} represents K, N, or E,
   X^{E41} represents D, Y, or K,
   X^{E71} represents H or L,
   VGTSDIVG (E-2), and
   X^{E12}X^{E22}X^{E32} (E-3)
   wherein
   X^{E12} represents K, S, or W,
   X^{E22} represents A or H,
   X^{E32} represents N or S; and
a light-chain CDR3 containing the amino acid sequence represented by any one of the following formulas (F-1) to (F-7):
   GX^{F21}X^{F31}X^{F41}X^{F51}X^{F61}X^{F71}X^{F81}X^{F91}X^{F101} (F-1)
   wherein
   X^{F21} represents V or A,
   X^{F31} represents S, D, N, G, or K,
   X^{F41} represents Y, F, V, L, or H,
   X^{F51} represents S, K, or N,
   X^{F61} represents G or N,
   X^{F71} represents G, A, E, or K,
   X^{F81} represents H, S, Y, or V,
   X^{F91} represents N, G, or S,
   X^{P101} represents I, V, L, or Y,
   GX^{F23}X^{F33}X^{F43}X^{F53}X^{F63}X^{F73}X^{F83}X^{F93}YX^{F113} (F-2)
   wherein
   X^{F23} represents V or A,
   X^{F33} represents S, D, N, or G,
   X^{F43} represents Y, F, V, L, or H,
   X^{F53} represents S, K, or N,
   X^{F63} represents G or N,
   X^{F73} represents G, A, E, or K,
   X^{F83} represents H, S, or Y,
   X^{F93} represents N, G, or S,
   X^{F113} represents I, V, L, or Y,
   GX^{F24}X^{F34}X^{F44}X^{F54}IX^{F74}X^{F84}X^{F94}YX^{F114}YX^{F134} (F-3)
   wherein
   X^{F24} represents V or A,
   X^{F34} represents S, D, N, or G,
   X^{F44} represents Y, F, V, L, or H,
   X^{F54} represents S, K, or N,
   X^{F74} represents G or N,
   X^{F84} represents G, A, E, or K,
   X^{F94} represents H, S, or Y,
   X^{F114} represents N, G, or S,
   X^{F134} represents I, V, L, or Y,
   GX^{F25}X^{F35}X^{F45}X^{F55}X^{F65}X^{F75}X^{F85}X^{F95} (F-4)
   wherein
   X^{F25} represents V or A,
   X^{F35} represents S, D, N, G, or K,
   X^{F45} represents Y, F, V, L, or H,
   X^{F55} represents S, K, or N,
   X^{F65} represents G or N,
   X^{F75} represents G, A, E, or K,
   X^{F85} represents H, S, Y, or V,
   X^{F105} represents I, V, L, or Y,
   QYDSTPPLT (F-5),
   QVWDSYTYV (F-6), and
   QFINGPLT (F-7).

### Item 3.

The antibody or fragment thereof according to Item 2, comprising
a heavy-chain CDR1 containing the amino acid sequence represented by formula (A-1),
a heavy-chain CDR2 containing the amino acid sequence represented by formula (B-1) or (B-2),
a heavy-chain CDR3 containing the amino acid sequence represented by any one of formulas (C-1), (C-2), (C-4), and (C-6) to (C-8),
a light-chain CDR1 containing the amino acid sequence represented by formula (D-1),
a light-chain CDR2 containing the amino acid sequence represented by any one of formulas (E-1) to (E-3), and
a light-chain CDR3 containing the amino acid sequence represented by any one of formulas (F-1) to (F-5).

### Item 4.

An antibody or fragment thereof (antigen-binding fragment), comprising
a heavy-chain CDR1 containing the amino acid sequence represented by the following formula (A-1-1): GFX^{A32}X^{A42}X^{A52}X^{A62}X^{A72}X^{A82} (A-1-1)
   wherein
   X^{A32} represents T or S,
   X^{A42} represents F or L,
   X^{A52} represents D, S, N, or T,
   X^{A62} represents D, S, or N,
   X^{A72} represents Y or F, and
   X^{A82} represents G, W, S, or Y;
a heavy-chain CDR2 containing the amino acid sequence represented by the following formula (B-1-1) or (B-2-1): IX^{B22}X^{B32}X^{B42}X^{B52}X^{B62}X^{B72}X^{B82} (B-1-1)
   wherein
   X^{B22} represents S, N, D, or H,
   X^{B32} represents Y, G, or P,
   X^{B42} represents S or D,
   X^{B52} represents G or S,
   X^{B62} represents G, D, T, or N,
   X^{B72} represents R, K, T, S, G, or Y,
   X^{B82} represents T or I, and
   IWX^{B34}X^{B44}X^{B54}X^{B64}X^{B74} (B-2-1)
   wherein
   X^{B34} represents Y or S,
   X^{B44} represents D or G,
   X^{B54} represents G or A,
   X^{B64} represents R or D, and
   X^{B74} represents T or I,
a heavy-chain CDR3 containing the amino acid sequence represented by any one of the following formulas (C-1-1), (C-2-1), (C-6-1), (C-7-1), and (C-8-1): X^{C12}RDGGSYX^{C82}SPIX^{C122}X^{C132}QX^{C152} (C-1-1)
   wherein
   X^{C12} represents A or S,
   X^{C82} represents Y, H, or S,
   X^{C122} represents N or S,
   X^{C132} represents F or C,
   X^{C152} represents F or Y, and
   ATX^{C34}X^{C44}X^{C54}X^{C64}X^{C74}X^{C84} (C-2-1)
   wherein
   X^{C34} represents N or S,
   X^{C44} represents Y or G,
   X^{C54} represents N or P,
   X^{C64} represents F or M,
   X^{C74} represents Q or D,
   X^{C84} represents Y or V,
   VSPLLVVHGPFFQY (C-6-1),
   VSPSGAGLDV (C-7-1), and
   SPSAVGFDV (C-8-1);
a light-chain CDR1 containing the amino acid sequence represented by any one of the following formulas (D-1-1) to (D-1-7):
   SEYKHYN (D-1-1),
   SEHRSYY (D-1-2),
   SDYSHYS (D-1-3),
   SQGINKW (D-1-4),
   SGHSSYY (D-1-5),
   SEHSSYY (D-1-6), and
   SDHSSYY (D-1-7);
a light-chain CDR2 containing the amino acid sequence represented by any one of the following formulas (E-1-1), (E-2), and (E-3-1):
   X^{E12}X^{E22}SDGSX^{E72} (E-1-1)
   wherein
   X^{E12} represents L, V, or I,
   X^{E22} represents K, or E,
   X^{E72} represents H or L,
   VGTSDIVG (E-2), and
   KAN (E-3-1); and
a light-chain CDR3 containing the amino acid sequence represented by any one of the following formulas (F-1-1), (F-2-1), (F-3-1), (F-4-1), and (F-5):
   GVSX^{F42}SGGHX^{F92}X^{F102} (F-1-1)
   wherein
   X^{F42} represents Y or F,
   X^{F92} represents N or G,
   X^{F102} represents I or V,
   GADYSGASSYV (F-2-1),
   GANHNIGESYGYV (F-3-1),
   GVX^{F36}YX^{F56}X^{F66}X^{F76}X^{F86}X^{F96} (F-4-1)
   wherein
   X^{F36} represents S, G, or K,
   X^{F56} represents S or N,
   X^{F66} represents G or N,
   X^{F76} represents G or K,
   X^{F86} represents Y or V,
   X^{F96} represents V or Y; and
   QYDSTPPLT (F-5).

### Item 5.

The antibody or fragment thereof according to Item 2, comprising
a heavy-chain CDR1 containing the amino acid sequence of any one of SEQ ID NOs: 3 to 11 or an amino acid sequence formed of any of these amino acid sequences with one to three amino acid mutations,
a heavy-chain CDR2 containing the amino acid sequence of any one of SEQ ID NOs: 12 to 25 or an amino acid sequence formed of any of these amino acid sequences with one to three amino acid mutations,
a heavy-chain CDR3 containing the amino acid sequence of any one of SEQ ID NOs: 26 to 41 or an amino acid sequence formed of any of these amino acid sequences with one to three amino acid mutations,
a light-chain CDR1 containing the amino acid sequence of any one of SEQ ID NOs: 42 to 50 or an amino acid sequence formed of any of these amino acid sequences with one to three amino acid mutations,
a light-chain CDR2 containing the amino acid sequence of any one of SEQ ID NOs: 51 to 58 or an amino acid sequence formed of any of these amino acid sequences with one to three amino acid mutations, or the amino acid sequence represented by KAN, SHN, or WAS, and
a light-chain CDR3 containing the amino acid sequence of any one of SEQ ID NOs: 59 to 73 or an amino acid sequence formed of any of these amino acid sequences with one to three amino acid mutations.

### Item 5a.

The antibody or fragment thereof according to Item 2, comprising
a heavy-chain CDR1 containing the amino acid sequence of any one of SEQ ID NOs: 3 to 9 or an amino acid sequence formed of any of these amino acid sequences with one to three amino acid mutations,
a heavy-chain CDR2 containing the amino acid sequence of any one of SEQ ID NOs: 12 to 19 and 25 or an amino acid sequence formed of any of these amino acid sequences with one to three amino acid mutations,
a heavy-chain CDR3 containing the amino acid sequence of any one of SEQ ID NOs: 26 to 29, 32, 33, 37, 40, and 41 or an amino acid sequence formed of any of these amino acid sequences with one to three amino acid mutations,
a light-chain CDR1 containing the amino acid sequence of any one of SEQ ID NOs: 42 to 48 or an amino acid sequence formed of any of these amino acid sequences with one to three amino acid mutations,
a light-chain CDR2 containing the amino acid sequence of any one of SEQ ID NOs: 51 to 54 and 57 or an amino acid sequence formed of any of these amino acid sequences with one to three amino acid mutations, or the amino acid sequence represented by KAN, and
a light-chain CDR3 containing the amino acid sequence of any one of SEQ ID NOs: 59 to 61, 64 to 68, and 71 or an amino acid sequence formed of any of these amino acid sequences with one to three amino acid mutations.

### Item 6.

The antibody or fragment thereof according to Item 5, wherein the mutations are conservative substitutions.

### Item 6a.

The antibody or fragment thereof according to Item 5a, wherein the mutations are conservative substitutions.

### Item 7.

The antibody or fragment thereof according to any one of Items 2 to 6, which is specifically bindable to the amino acid sequence of SEQ ID NO: 1.

### Item 7a.

The antibody or fragment thereof according to Item 5a or 6a, which is specifically bindable to the amino acid sequence of SEQ ID NO: 1.

### Item 8.

An antibody or fragment thereof (antigen-binding fragment), comprising
a heavy-chain CDR1 containing the amino acid sequence represented by the following formula (G-1) or (G-2): GX^{G21}X^{G31}X^{G41}X^{GS1}X^{G61}X^{G71}X^{G81} (G-1)
   wherein
   X^{G21} represents F or L,
   X^{G31} represents I, T, S, or F,
   X^{G41} represents F or L,
   X^{G51} represents S, N, T, R, or D,
   X^{G61} represents N, T, or D,
   X^{G71} represents Y, S, or H,
   X^{C81} represents F, T, Y, W, or G,
   KLSERY (G-2);
a heavy-chain CDR2 containing the amino acid sequence represented by any one of the following formulas (H-1) to (H-3):
   IX^{H21}X^{H31}X^{H41}X^{H51}X^{H61}X^{H71}X^{H81} (H-1)
   wherein
   X^{H21} represents S, G, K, or N,
   X^{H31} represents G, N, S, Y, T, or P,
   X^{H41} represents D, S, T, or A,
   X^{H51} represents S, G, or D,
   X^{H61} represents T, S, A, G, or R,
   X^{H71} represents Y, K, N, T, I, or S,
   X^{H81} represents I, T, P, or V,
   MWSDGDT (H-2), and
   NDS (H-3);
a heavy-chain CDR3 containing the amino acid sequence represented by any one of the following formulas (I-1) to (I-11): X^{I11}X^{I21}X^{I31}X^{I41}X^{I51}X^{I61}X^{I71} (I-1)
   wherein
   X^{I11} represents S or T,
   X^{I21} represents I or T,
   X^{I31} represents N, L, D, or T,
   X^{I41} represents W or G,
   X^{I51} represents G, V, F, or N,
   X^{I61} represents D, G, F, or N,
   X^{I71} represents V or L,
   ARTSRIVADLVTMGYALDF (I-2),
   ARDVVKTGTTGLPFDL (I-3),
   AREGVITVGTWGDV (I-4),
   ATSEY (I-5),
   TTATDV (I-6),
   TPATDV (I-7),
   SPMTIHGLDT (I-8),
   GSGWV (I-9),
   GSGWY (I-10), and
   LSSESDDARV (I-11);
a light-chain CDR1 containing the amino acid sequence represented by any one of the following formulas (J-1) to (J-3): X^{J11}LX^{J31}X^{J41}X^{J51}Y (J-1)
   wherein
   X^{J11} represents K or A,
   X^{J31} represents S or P,
   X^{J41} represents N, E, K, T, or D,
   X^{J51} represents Q, R, K, or E,
   SX^{J23}SLX^{J53}X^{J63}X^{J73}DGX^{J103}TX^{J123} (J-2),
   wherein
   X^{J23} represents E or Q,
   X^{J53} represents L or V,
   X^{J63} represents H, D, or Y,
   X^{J73} represents S or G,
   X^{J103} represents N, K, or Y,
   X^{J123} represents Y or F, and
   NIGGKA (J-3),
a light-chain CDR2 containing the amino acid sequence represented by the following formula (K-1): X^{K11}X^{K21}X^{K31} (K-1)
   wherein
   X^{K11} represents K, N, L, or E,
   X^{K21} represents D, G, V, or I,
   X^{K31} represents S, N, or D, and
a light-chain CDR3 containing the amino acid sequence represented by any one of the following formulas (L-1) to (L-6):
   LSX^{L31}X^{L41}X^{L51}X^{L61}X^{L71}X^{L81}X^{L91}X^{L101}X^{L111} (L-1)
   wherein
   X^{L31} represents R, S, A, or G,
   X^{L41} represents Q, E, or D,
   X^{L51} represents S or G,
   X^{L61} represents D, S, or N,
   X^{L71} represents D, G, or T,
   X^{L81} represents A, T, or Y,
   X^{L91} represents A, T, or Y,
   X^{L101} represents S, R, or W,
   X^{L111} represents V, L, or Y,
   LSX^{L33}X^{L43}X^{L53}X^{L63}X^{L73}X^{L83}X^{L93}X^{L103} (L-2),
   wherein
   X^{L33} represents R, S, A, or G,
   X^{L43} represents Q, E, or D,
   X^{L53} represents S or G,
   X^{L63} represents D, S, or N,
   X^{L73} represents D, G, or T,
   X^{L83} represents A, T, or Y,
   X^{L93} represents S, R, or W,
   X^{L103} represents V, L, or Y,
   X^{L14}QX^{L34}X^{L44}X^{L54}X^{L64}PX^{L84}X^{L94} (L-3),
   wherein
   X^{L14} represents L or V,
   X^{L34} represents A or T,
   X^{L44} represents T or S,
   X^{L54} represents H or N,
   X^{L64} represents D, N, or V,
   X^{L84} represents Y, V, I, or L,
   X^{L94} represents T or S,
   QX^{L27}X^{L37}X^{L47}X^{L57}PX^{L77}X^{L87} (L-4)
   wherein
   X^{L27} represents A or T,
   X^{L37} represents T or S,
   X^{L47} represents H or N,
   X^{L57} represents D, N, or V,
   X^{L77} represents Y, V, I, or L,
   X^{L87} represents T or S,
   QVYDSSSFV (L-5), and
   WQGTDFPR (L-6).

### Item 9.

The antibody or fragment thereof according to Item 8, comprising
a heavy-chain CDR1 containing the amino acid sequence represented by formula (G-1),
a heavy-chain CDR2 containing the amino acid sequence represented by formula (H-1) or (H-2),
a heavy-chain CDR3 containing the amino acid sequence represented by any one of formulas (I-1) to (I-3) and (I-5) to (I-9),
a light-chain CDR1 containing the amino acid sequence represented by any one of formulas (J-1) to (J-3),
a light-chain CDR2 containing the amino acid sequence represented by formula (K-1), and
a light-chain CDR3 containing the amino acid sequence represented by any one of formulas (L-1) to (L-3), (L-5), and (L-6).

### Item 10.

An antibody or fragment thereof (antigen-binding fragment), comprising
a heavy-chain CDR1 containing the amino acid sequence represented by the following formula (G-1-1):
   GX^{G22}X^{G32}X^{G42}X^{G52}X^{G62}X^{G72}X^{G82} (G-1-1)
   wherein
   X^{G22} represents F or L,
   X^{G32} represents I, T, or S,
   X^{G42} represents F or L,
   X^{G52} represents S, N, T, or R,
   X^{G62} represents N or T,
   X^{G72} represents Y, S, or H,
   X^{G82} represents F, T, Y, or W,
a heavy-chain CDR2 containing the amino acid sequence represented by the following formula (H-1-1) or (H-2): IX^{H22}X^{H32}X^{H42}X^{H52}X^{H62}X^{H72}X^{H82} (H-1-1)
   wherein
   X^{H22} represents S, G, K, or N,
   X^{H32} represents G, N, S, T, or P,
   X^{H42} represents D, S, or A,
   X^{H52} represents S, G, or D,
   X^{H62} represents T, S, G, or R,
   X^{H72} represents Y, K, N, T, I, or S,
   X^{H82} represents I, T, P, or V, and
   MWSDGDT (H-2),
a heavy-chain CDR3 containing the amino acid sequence represented by any one of the following formulas (I-1-1), (1-2), (1-3), and (1-5) to (1-9): X^{I12}X^{I22}X^{I32}X^{I42}X^{I52}X^{I62}X^{I72} (I-1-1)
   wherein
   X^{I12} represents S or T,
   X^{I22} represents I or T,
   X^{I32} represents N, L, or T,
   X^{I42} represents W or G,
   X^{I52} represents G, V, F, or N,
   X^{I62} represents D, F, or G,
   X^{I72} represents V or L,
   ARTSRIVADLVTMGYALDF (I-2),
   ARDVVKTGTTGLPFDL (I-3),
   ATSEY (I-5),
   TTATDV (I-6),
   TPATDV (I-7),
   SPMTIHGLDT (I-8), and
   GSGWV (I-9),
a light-chain CDR1 containing the amino acid sequence represented by any one of the following formulas (J-1-1), (J-2-1), and (J-3):
   KLSX^{J42}X^{J52}Y (J-1-1)
   wherein
   X^{J42} represents N, E, or K,
   X^{J52} represents Q, R, or E,
   SX^{J24}SLX^{J54}X^{J64}SDGX^{J104}TX^{J124} (J-2-1)
   wherein
   X^{J24} represents E or Q,
   X^{J54} represents L or V,
   X^{J64} represents H, D, or Y,
   X^{J104} represents N, K, or Y,
   X^{J124} represents Y or F, and
   NIGGKA (J-3),
a light-chain CDR2 containing the amino acid sequence represented by the following formulas (K-1-1) to (K-1-7) :
   NDD (K-1-1),
   KDS (K-1-2),
   NGN (K-1-3),
   NDS (K-1-4),
   KVS (K-1-5),
   LIS (K-1-6), and
   EVS (K-1-7), and
a light-chain CDR3 containing the amino acid sequence represented by any one of the following formulas (L-1-1), (L-2-1), (L-3-1), (L-5), and (L-6): LSX^{L32}X^{L42}SX^{L62}DAAX^{L102}V (L-1-1)
   wherein
   X^{L32} represents R or S,
   X^{L42} represents Q or E,
   X^{L62} represents D or N,
   X^{L102} represents S, R, or W,
   LSGESDDAWV (L-2-1),
   X^{L15}QX^{L35}THDPYT (L-3-1)
   wherein
   X^{L15} represents L or V,
   X^{L35} represents A or T,
   QVYDSSSFV (L-5), and
   WQGTDFPR (L-6).

### Item 11.

The antibody or fragment thereof according to Item 8, comprising
a heavy-chain CDR1 containing the amino acid sequence of any one of SEQ ID NOs: 74 to 84 or an amino acid sequence formed of any of these amino acid sequences with one to three amino acid mutations,
a heavy-chain CDR2 containing the amino acid sequence of any one of SEQ ID NOs: 85 to 101 or an amino acid sequence formed of any of these amino acid sequences with one to three amino acid mutations, or the amino acid sequence represented by NDS,
a heavy-chain CDR3 containing the amino acid sequence of any one of SEQ ID NOs: 102 to 118 or an amino acid sequence formed of any of these amino acid sequences with one to three amino acid mutations,
a light-chain CDR1 containing the amino acid sequence of any one of SEQ ID NOs: 119 to 133 or an amino acid sequence formed of any of these amino acid sequences with one to three amino acid mutations,
a light-chain CDR2 containing the amino acid sequence represented by NDD, KDS, NGN, NDS, KVS, LIS, or EVS, and a light-chain CDR3 containing the amino acid sequence of any one of SEQ ID NOs: 134 to 149 or an amino acid sequence formed of any of these amino acid sequences with one to three amino acid mutations.

### Item 11a.

The antibody or fragment thereof according to Item 8, comprising
a heavy-chain CDR1 containing the amino acid sequence of any one of SEQ ID NOs: 74 to 81 or an amino acid sequence formed of any of these amino acid sequences with one to three amino acid mutations,
a heavy-chain CDR2 containing the amino acid sequence of any one of SEQ ID NOs: 85 to 90, 92 to 95, and 101 or an amino acid sequence formed of any of these amino acid sequences with one to three amino acid mutations,
a heavy-chain CDR3 containing the amino acid sequence of any one of SEQ ID NOs: 102 to 106, 109, 110, and 112 to 116 or an amino acid sequence formed of any of these amino acid sequences with one to three amino acid mutations,
a light-chain CDR1 containing the amino acid sequence of any one of SEQ ID NOs: 119 to 121, 125 to 129, and 133 or an amino acid sequence formed of any of these amino acid sequences with one to three amino acid mutations,
a light-chain CDR2 containing the amino acid sequence represented by NDD, KDS, NGN, NDS, KVS, LIS, or EVS, and
a light-chain CDR3 containing the amino acid sequence of any one of SEQ ID NOs: 134 to 136, 140 to 142, 148, and 149 or an amino acid sequence formed of any of these amino acid sequences with one to three amino acid mutations.

### Item 12.

The antibody or fragment thereof according to Item 11, wherein the mutations are conservative substitutions.

### Item 12a.

The antibody or fragment thereof according to Item 11a, wherein the mutations are conservative substitutions.

### Item 13.

The antibody or fragment thereof according to any one of Items 8 to 12, which is specifically bindable to the amino acid sequence of SEQ ID NO: 2.

### Item 13a.

The antibody or fragment thereof according to Item 11a or 12a, which is specifically bindable to the amino acid sequence of SEQ ID NO: 2.

### Item 14.

An antibody or fragment thereof (antigen-binding fragment) against a nucleocapsid protein of severe acute respiratory syndrome coronavirus-2 (SARS-CoV-2), whose epitope is present in a region formed of the amino acid sequence of any of SEQ ID NOs: 150 to 152 of the nucleocapsid protein.

### Item 15.

The antibody or fragment thereof according to any one of Items 1 to 14, wherein the antibody is a monoclonal antibody.

### Item 15a.

The antibody or fragment thereof according to any one of Items 5a, 6a, 11a, and 12a, wherein the antibody is a monoclonal antibody.

### Item 16.

A polynucleotide comprising a coding sequence of the antibody or fragment thereof of any one of Items 1 to 15.

### Item 16a.

A polynucleotide comprising a coding sequence of the antibody or fragment thereof of any one of Items 5a, 6a, 11a, 12a, and 15a.

### Item 17.

A cell comprising the polynucleotide of Item 16.

### Item 17a.

A cell comprising the polynucleotide of Item 16a.

### Item 18.

A reagent or drug comprising the antibody or fragment thereof of any one of Items 1 to 15, the polynucleotide of Item 16, or the cell of Item 17.

### Item 18a.

A reagent or drug comprising the antibody or fragment thereof of any one of Items 5a, 6a, 11a, 12a, and 15a, the polynucleotide of Item 16a, or the cell of Item 17a.

### Item 19.

A kit for detecting the nucleocapsid protein of severe acute respiratory syndrome coronavirus-2 (SARS-CoV-2), comprising the antibody or fragment thereof of any one of Items 1 to 15.

### Item 19a.

A kit for detecting the nucleocapsid protein of severe acute respiratory syndrome coronavirus-2 (SARS-CoV-2), comprising the antibody or fragment thereof of any one of Items 5a, 6a, 11a, 12a, and 15a.

### Item 20.

A kit for detecting the nucleocapsid protein of severe acute respiratory syndrome coronavirus-2 (SARS-CoV-2), comprising
two or more antibodies or fragments thereof specifically bindable to the amino acid sequence of SEQ ID NO: 1, the two or more antibodies or fragments thereof each recognizing a different epitope in the amino acid sequence of SEQ ID NO: 1, and/or
two or more antibodies or fragments thereof specifically bindable to the amino acid sequence of SEQ ID NO: 2, the two or more antibodies or fragments thereof each recognizing a different epitope in the amino acid sequence of SEQ ID NO: 2.

### Item 21.

The kit according to Item 20,
wherein
the two or more antibodies or fragments thereof specifically bindable to the amino acid sequence of SEQ ID NO: 1 are each the antibody or fragment thereof of any one of Items 2 to 7 or an antibody or fragment thereof containing an amino acid sequence with at least 90% identity to the amino acid sequence of the antibody or fragment thereof of any one of Items 2 to 7, and
the two or more antibodies or fragments thereof specifically bindable to the amino acid sequence of SEQ ID NO: 2 are each the antibody or fragment thereof of any one of Items 8 to 14 or an antibody or fragment thereof containing an amino acid sequence with at least 90% identity to the amino acid sequence of the antibody or fragment thereof of any one of Items 8 to 14.

### Item 21a.

The kit according to Item 20,
wherein
the two or more antibodies or fragments thereof specifically bindable to the amino acid sequence of SEQ ID NO: 1 are each the antibody or fragment thereof of Item 5a or 6a, and
the two or more antibodies or fragments thereof specifically bindable to the amino acid sequence of SEQ ID NO: 2 are each the antibody or fragment thereof of Item 11a or 12a.

### Item 22.

The kit according to any one of Items 19 to 21, for detecting the nucleocapsid protein of severe acute respiratory syndrome coronavirus-2 (SARS-CoV-2) by immunochromatography, enzyme immunoassay, chemiluminescent immunoassay, chemiluminescent enzyme immunoassay, radioimmunoassay, electrochemiluminescent immunoassay, immunonephelometry, or latex agglutination.

### Item 22a.

The kit according to Item 19a or 21a, for detecting the nucleocapsid protein of severe acute respiratory syndrome coronavirus-2 (SARS-CoV-2) by immunochromatography, enzyme immunoassay, chemiluminescent immunoassay, chemiluminescent enzyme immunoassay, radioimmunoassay, electrochemiluminescent immunoassay, immunonephelometry, or latex agglutination.

### Item 23.

The kit according to any one of Items 19 to 22, wherein a portion of two or more antibodies or fragments thereof specifically bindable to the amino acid sequence of SEQ ID NO: 1 or 2 is immobilized, and the remaining portion is labeled.

### Item 23a.

The kit according to any one of Items 19a, 21a, and 22a, wherein a portion of two or more antibodies or fragments thereof specifically bindable to the amino acid sequence of SEQ ID NO: 1 or 2 are immobilized, and the remaining portion is labeled.

### Item 24.

A method for detecting the nucleocapsid protein of severe acute respiratory syndrome coronavirus-2 (SARS-CoV-2), comprising the following steps (1) to (3):
(1) immobilizing a primary antibody or fragment thereof specifically bindable to the amino acid sequence of SEQ ID NO: 1 or 2 onto a solid phase,
(2) applying a secondary antibody or fragment thereof that is specifically bindable to a nucleocapsid protein of SARS-CoV-2, and that is specifically bindable to the amino acid sequence of SEQ ID NO: 1 or 2 to the solid phase, wherein the secondary antibody or fragment thereof recognizes a different epitope in an amino acid sequence to which the primary antibody or fragment thereof is specifically bindable, and the secondary antibody or fragment thereof is labeled with a labeling substance, and
(3) detecting a label derived from the labeling substance of the secondary antibody or fragment thereof bound to the solid phase via the nucleocapsid protein.

### Item 25.

The method according to Item 24, wherein the primary antibody or fragment thereof and the secondary antibody or fragment thereof are selected from the group consisting of the antibody or fragment thereof of any one of Items 2 to 15, and an antibody or fragment thereof containing an amino acid sequence with at least 90% identity to the amino acid sequence of the antibody or fragment thereof of any one of Items 2 to 15.

### Item 25a.

The method according to Item 24, wherein the primary antibody or fragment thereof and the secondary antibody or fragment thereof are selected from the group consisting of the antibody or fragment thereof of any one of Items 5a, 6a, 11a, 12a, and 15a, and an antibody or fragment thereof containing an amino acid sequence with at least 90% identity to the amino acid sequence of the antibody or fragment thereof of any one of Items 5a, 6a, 11a, 12a, and 15a.

### Advantageous Effects of Invention

The present invention provides an antibody or fragment thereof specifically bindable to the NTD or CTD of the N protein of SARS-CoV-2. The present invention also provides a kit and a method for detecting the N protein of SARS-CoV-2 with high sensitivity.

### Description of Embodiments

### 1. Definition

In the present specification, an amino acid may be a natural or non-natural amino acid. Examples of non-natural amino acids include, but are not limited to, citrulline, ornithine, ε-acetyl-lysine, β-alanine, aminobenzoic acid, 6-aminocaproic acid, aminobutyric acid, hydroxyproline, mercaptopropionic acid, 3-nitrotyrosine, norleucine, and pyroglutamic acid. An amino acid may be, for example, L-amino acid, D-amino acid, or DL-amino acid.

In the present specification, the "identity" of amino acid sequences refers to the degree to which two or more contrastable amino acid sequences match each other in optimal alignment. The identity of amino acid sequences can be calculated by using analysis tools available commercially or via telecommunication lines (Internet), such as commercial software GENETYX (Genetyx Corporation) or using default parameters in the homology algorithm BLAST (basic local alignment search tool) of the National Center for Biotechnology Information (NCBI) (http://www.ncbi.nlm.nih.gov/BLAST/).

The amino acid sequences disclosed in the present specification may have one or more (e.g., one, two, or three) amino acids deleted, substituted, or modified, and may have one or more (e.g., one, two, or three) amino acids inserted or added as long as its bindability to the N protein of SARS-CoV-2 is not interfered with.

The substitution of an amino acid is preferably a substitution of an amino acid to another amino acid similar in structure and/or properties (conservative substitution). Conservative substitution includes, for example, substitutions within the groups shown in Table 1.

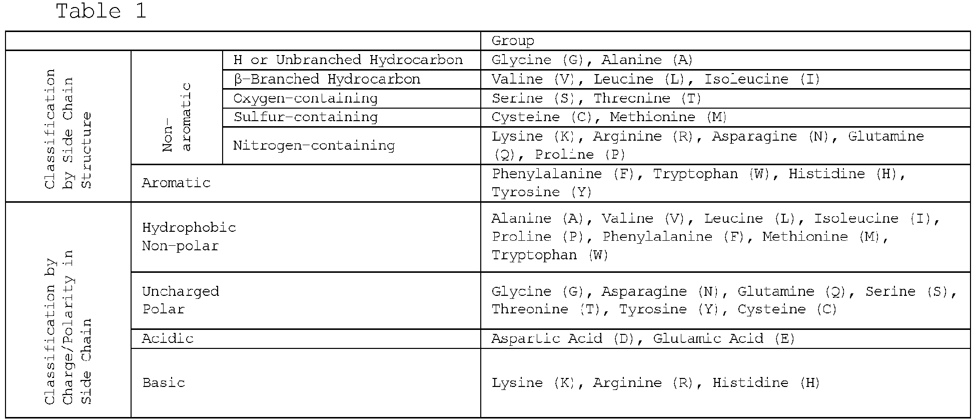

Modification of amino acids includes, for example, modification of functional groups such as amino, carboxyl, hydroxyl, and sulfhydryl (SH) groups. Examples of modification of the functional groups include glycosylation; methylation; esterification; amidation; PEGylation; phosphorylation; hydroxylation; binding to protective groups such as a t-butoxycarbonyl (Boc) group or a 9-fluorenylmethyloxycarbonyl (Fmoc) group; biotinylation; binding to a fluorescent dye such as fluorescein isothiocyanate (FITC); and binding to an enzyme such as peroxidase (HRP) or alkaline phosphatase (ALP).

In the present specification, antibodies may be either a monoclonal antibody or a polyclonal antibody, and are preferably a monoclonal antibody. Antibodies can be of any isotype, such as IgG, IgA, IgD, IgE, or IgM. Antibodies may be either a human antibody or a non-human antibody. Non-human antibodies include, but are not limited to, mouse antibodies, rat antibodies, and guinea pig antibodies. Antibodies may be a chimeric antibody, such as a mouse-human chimeric antibody. Antibodies can be a partially or fully humanized antibody.

In the present specification, antibody fragments are not particularly limited as long as they include heavy-chain CDR1 to CDR3 and light-chain CDR1 to CDR3. Examples of antibody fragments include Fv, Fab, Fab', (Fab')₂, scFv, scFv-Fc, diabody, triabody, tetrabody, and minibody. Antibody fragments are preferably a fragment with an antigen-binding capacity (antigen-binding fragment).

In the present specification, the heavy-chain CDR1 to CDR3 and the light-chain CDR1 to CDR3 are defined according to IMGT.

In the present specification, nucleotides such as DNA and RNA may have known chemical modifications as illustrated below. To prevent degradation by hydrolytic enzymes such as nucleases, the phosphate residue (phosphate) of each nucleotide may be replaced with a chemically modified phosphate residue, such as phosphorothioate (PS), methyl phosphonate, or phosphorodithionate. The hydroxyl group at the 2-position of the ribose of each ribonucleotide may be replaced with -OR (R is, for example, -CH₃, -CH₂CH₂OCH₃, -CH₂CH₂NHC(NH)NH₂, -CH₂CONHCH₃, and -CH₂CH₂CN). Additionally, the base moiety (pyrimidine, purine) may be chemically modified, for example, by introducing a methyl group or cationic functional group at the 5-position of the pyrimidine base, or by replacing the carbonyl group at the 2-position with thiocarbonyl. Furthermore, the phosphate and hydroxyl moieties may be modified with, for example, biotin, an amino group, a lower alkylamine group, or an acetyl group. However, modifications are not limited to these.

In the present specification, the N protein of SARS-CoV-2 (antigen) has the amino acid sequence disclosed in GenBank accession number MN908947. The N protein of SARS-CoV has the amino acid sequence disclosed in GenBank accession number AY278741.

### 2. Antibody or fragment thereof specifically bindable to the NTD of the N protein of SARS-CoV-2

The NTD of the N protein of SARS-CoV-2 (simply "NTD") means the amino acid region at positions 1 to 172 from the N-terminus of the N protein and has the following amino acid sequence:

MSDNGPQNQRNAPRITFGGPSDSTGSNQNGERSGARSKQRRPQGLPNNTASWFTALTQHGKEDLK
FPRGQGVPINTNSSPDDQIGYYRRATRRIRGGDGKMKDLSPRWYFYYLGTGPEAGLPYGANKDGI
IWVATEGALNTPKDHIGTRNPANNAAIVLQLPQGTTLPKGFY (SEQ ID NO: 1)

The antibody or fragment thereof specifically bindable to the NTD of SARS-CoV-2 preferably does not bind to the NTD of SARS-CoV or has low bindability to the NTD of SARS-CoV. In the amino acid sequence of SEQ ID NO: 1, for example, the 63rd D, 65th K, 79th S, 94th I, 103rd D, 120th G, 128th D, 131st I, 152nd A, and 157th I differ from their corresponding amino acid in the NTD of SARS-CoV. The antibody or fragment thereof specifically bindable to the NTD of SARS-CoV-2 preferably binds to a region (or an epitope) containing one or more amino acids selected from the group consisting of the 63rd D, 65th K, 79th S, 94th I, 103rd D, 120th G, 128th D, 131st I, 152nd A, and 157th I in SEQ ID NO: 1.

In an embodiment, the antibody or fragment thereof specifically bindable to the NTD of SARS-CoV-2 may bind to the amino acid region at positions 1 to 180 from the N-terminus of the N protein, preferably to the RNA-binding domain of the NTD, and preferably to the amino acid region at positions 44 to 180 or 44 to 172 from the N-terminus of the N protein.

The antibody or fragment thereof specifically bindable to the NTD of SARS-CoV-2 can be produced according to known methods in combination, such as by a method including the following steps (P1) to (P6):
(P1) obtaining a cell producing an antibody that specifically binds to the N protein of SARS-CoV-2 from antibody-producing cells of an animal immunized with the N protein of SARS-CoV-2 by using the N protein of SARS-CoV-2 labeled with a fluorescent label whose staining selectivity for the endoplasmic reticulum is higher than that for cell organelles other than the endoplasmic reticulum;
(P2) obtaining an antibody variable region gene fragment from the cell obtained in step (P1);
(P3) obtaining a gene expression unit from the antibody variable region gene fragment obtained in step (P2);
(P4) introducing the gene expression unit obtained in step (P3) into a cell to express antibodies that bind to the N protein of SARS-CoV-2;
(P5) recovering the antibodies expressed in step (P4); and
(P6) obtaining antibodies that specifically bind to the NTD from the antibodies recovered in step (P5).

Step (P1) can be performed according to the method (ERIAA method) described, for example, in U.S. Patent Application Publication No. 2013/0029325 (incorporated by reference in its entirety into this specification).

Step (P2) can be performed according to the method (MAGrahd method) described, for example, in the following literature: Rapid production of antigen-specific monoclonal antibodies from a variety of animals, BMC Biology 2012, 10:80.

Step (P3) can be performed according to the method (TS-jPCT method) described, for example, in U.S. Patent Application Publication No. 2013/0023009 (incorporated by reference in its entirety into this specification).

Step (P4) can be performed using known gene transfer methods, such as calcium phosphate transfection, lipofection, DEAE dextran, electroporation, or microinjection. The cells into which the gene expression unit is introduced are not particularly limited to specific species as long as the cells can transiently express antibodies, and include mammalian cells such as CHO cells, HEK293 cells, Expi293 cells, 293F cells, 293T cells, and 293FT cells.

Step (P6) can be performed by selecting antibodies that specifically bind to the NTD according to a known screening method, such as enzyme-linked immunosorbent assay (ELISA), western blotting, or immunostaining.

The antibodies may optionally be purified according to a known method, such as salting out using ammonium sulfate, sodium sulfate, etc.; affinity chromatography using protein A etc.; ion-exchange chromatography using DEAE cellulose etc.; gel filtration; or a purification method using His-tag, FLAG-tag, etc.

The method including steps (P1) to (P6) can provide multiple types of antibodies or fragments thereof that specifically bind to the NTD of SARS-CoV-2. (The antibodies or fragments thereof differ from each other in terms of the amino acid sequence of at least one CDR of the heavy-chain CDR1 to CDR3 and the light-chain CDR1 to CDR3.)

In an embodiment, the heavy-chain CDR1 of the antibody or fragment thereof specifically bindable to the NTD of SARS-CoV-2 preferably contains the following amino acid sequence:
· the amino acid sequence represented by either formula (A-1) or (A-2) shown in Table 2
· an amino acid sequence having at least 90% (preferably at least 95%) identity to the amino acid sequence represented by either formula (A-1) or (A-2), or
· an amino acid sequence formed of the amino acid sequence represented by either formula (A-1) or (A-2) with 1 to 3 (preferably 1 or 2, more preferably 1) amino acid mutations (preferably conservative substitution).

**Table 2**

| | | |
|---|---|---|
| | | GFTFDDYG (A-1-2, SEQ ID NO: 3) |
| GFX^{A31}X^{A41}X^{A51}X^{A61}X^{A71}X^{A81} | | GFTFSDFW (A 1-3, SEQ ID NO: 4) |
| (A-1) | GFX^{A32}X^{A42}X^{AS2}X^{A62}X^{A72}X^{A82} (A-1-1) | GFTFNNYW (A-1-4, SEQ ID NO: 5) |
| wherein | X^{A32} represents T or S, | |
| X^{A41} represents F, L, or I, | X^{A32} represents F or L, | |
| X^{A31} represents T or S, | X^{A52} represents D, S, N, or T, | GFTFDDFG (A 1-5, SEQ ID NO: 6) |
| X^{A51} represents D, S, N, or T, | X^{A62} represents D, S, or N, | GFTFSNYY (A 1-6, SEQ ID NO: 7) |
| X^{A61} represents D, N, S, I, or T, | X^{A72} represents Y or F, | |
| X^{A71} represents Y, F, or N, | X^{A82} represents G, W, S, or Y | GFTFSDYW (A 1-7, SEQ ID NO: 8) |
| X^{A81} represents G, W, Y, S, or T. | | |
| | | GFSLTSYS (A 1-8, SEQ ID NO: 9) |
| | GFTFSDYT (A-1-9, SEQ ID NO: 10) | |
| GFX^{A33}X^{A43}X^{AS3}X^{A63}X^{A73}X^{A83}X^{A93}X^{A103} (A-2) | | GFSITTNGYT (A-2-2, SEQ ID NO: 11) |
| wherein | GFSITTX^{A74} | |
| X^{A33} represents T or S, | X^{A84}YX^{A104} | |
| X^{A43} represents F, L, or I, | (A-2-1) | |
| X^{A53} represents D, S, N, or T, | wherein | |
| X^{A63} represents D, N, S, or T, | X^{A74} represents N or S, | |
| X^{A73} represents N or S, | X^{A84} represents G or Y, | |
| X^{A83} represents G or Y, | X^{A104} represents T or G. | |
| X^{A93} represents Y, F, or N, | | |
| X^{A103} represents T, G, W, or Y | | |

In formula (A-1), X^{A41} is preferably F or L; X^{A51} is preferably D, S, N, or T; X^{A61} is preferably D, N, S, or I, and more preferably D, N, or S; X^{A71} is preferably Y or F; and X^{A81} is preferably G, W, S, or Y.

Formula (A-1) is preferably formula (A-1-1) or one selected from the group consisting of formulas (A-1-2) to (A-1-9), and more preferably one selected from the group consisting of formulas (A-1-2) to (A-1-8).

In formula (A-2), X^{A33} is preferably S; X^{A43} is preferably I; X^{A53} is preferably T; X^{A63} is preferably T; X^{A73} is preferably N or S; X^{A83} is preferably G or Y; X^{A93} is preferably Y or F, and more preferably Y; and X^{A103} is preferably T, G, or W, and more preferably is T or G.

Formula (A-2) is preferably formula (A-2-1), and more preferably formula (A-2-2).

The heavy-chain CDR1 of the antibody or fragment thereof specifically bindable to the NTD of SARS-CoV-2 preferably contains the amino acid sequence of formula (A-1); more preferably the amino acid sequence of formula (A-1-1) or one amino acid sequence selected from the group consisting of the amino acid sequences of formulas (A-1-2) to (A-1-9); and still more preferably one amino acid sequence selected from the group consisting of the amino acid sequences of formulas (A-1-2) to (A-1-8); or an amino acid sequence having at least 90% identity to any of these amino acid sequences. The identity is preferably at least 91%, at least 92%, at least 93%, at least 94%, or at least 95%.

In an embodiment, the heavy-chain CDR2 of the antibody or fragment thereof specifically bindable to the NTD of SARS-CoV-2 preferably contains the following amino acid sequence:
· the amino acid sequence represented by either formula (B-1) or (B-2) shown in Table 3,
· an amino acid sequence having at least 90% (preferably at least 95%) identity to the amino acid sequence represented by either formula (B-1) or (B-2), or
· an amino acid sequence formed of the amino acid sequence represented by either formula (B-1) or (B-2) with 1 to 3 (preferably 1 or 2, more preferably 1) amino acid mutations (preferably conservative substitution).

**Table 3**

| | | |
|---|---|---|
| | | ISYSGGRT (B-1-2, SEQ ID NO: 12) |
| | | INYDGDRT (B-1-3, SEQ ID NO: 13) |
| | | INGDSTKI (B-1-4, SEQ ID NO: 14) |
| | IX^{B22}X^{B32}X^{B42}X^{B32}X^{B62}X^{B72}X^{B82} (B-1-1) | IDYSGGTT (B-1-5, SEQ ID NO: 15) |
| | wherein | |
| IX^{B21}X^{B31}X^{B41}X^{B51}X^{B61}X^{B71}X^{B81} (B-1) wherein | X^{B22} represents S, N, D, or H, | |
| X^{B21} represents S, N, D, W, or H, | X^{B32} represents Y, G, or P, | ISYSGGST (B-1-6, SEQ ID NO: 16) |
| X^{B31} represents Y, G, P, or S, | X^{B42} represents S or D, | |
| X^{B41} represents S, D, or G, | X^{B52} represents G or S, | |
| X^{B51} represents G, S, T, or A, | X^{B62} represents G, D, T, or N, | ISGDSNYI (B-1-7, SEQ ID NO: 17) |
| X^{B61} represents G, D, T, N, or R, | X^{B72} represents R, K, T, S, G, or Y, | |
| X^{B71} represents R, K, T, S, N, G, Y, or D, | X^{B82} represents T or I. | |
| X^{B81} represents T, I, or M. | | IHPDGGST (B-1-8, SEQ ID NO: 18) |
| | | ISPDGGGT (B-1-9, SEQ ID NO: 19) |
| | | ISPSGGST (B-1-10, SEQ ID NO: 20) |
| | ISYSGGNT (B-1-11, SEQ ID NO: 21) | |
| | IWYDGTKM (B-1-12, SEQ ID NO: 22) | |
| | ISGDTNYI (B-1-13, SEQ ID NO: 23) | |
| IX^{B23}X^{B33}X^{B43}X^{B53}X^{B63}X^{B73} (B-2) | IWX^{B34}X^{B44}X^{B54}X^{B64}X^{B74} (B-2-1) | IWYDADT (B-2-2, SEQ ID NO: 24) |
| wherein | wherein | |
| X^{B23} represents S, N, D, W, or H, | X^{B34} represents Y or S, | |
| X^{B33} represents Y, G, P, or S, | X^{B44} D represents or G, | |
| X^{B43} represents S, D, or G, | X^{B54} represents G or A, | IWSGGRI (B-2-3, SEQ ID NO: 25) |
| X^{B53} represents G, S, T, or A, | X^{B64} represents R or D, | |
| X^{B63} represents R, K, T, S, N, G, Y, or D, | X^{B74} represents T or I. | |
| X^{B73} represents T, I, or M. | | |

In formula (B-1), X^{B21} is preferably S, N, D, or H; X^{B31} is preferably Y, G, or P; X^{B41} is preferably S or D; X^{B51} is preferably G or S; X^{B61} is preferably G, D, T, or N; X^{B71} is preferably R, K, T, S, G, or Y; and X^{B81} is preferably is T or I.

Formula (B-1) is preferably formula (B-1-1) or one selected from the group consisting of formulas (B-1-2) to (B-1-13), more preferably one selected from the group consisting of formulas (B-1-2) to (B-1-10), and still more preferably one selected from the group consisting of formulas (B-1-2) to (B-1-9) .

In formula (B-2), X^{B23} is preferably S, N, D, or W, more preferably W; X^{B33} is preferably Y, G, or S, more preferably Y or S; X^{B43} is preferably D or G; X^{B53} is preferably G, S, or A, more preferably G or A; X^{B63} is preferably R, K, T, or D, more preferably R or D; and X^{B73} is preferably T or I.

Formula (B-2) is preferably formula (B-2-1), more preferably formula (B-2-2) or (B-2-3), and still more preferably formula (B-2-3).

The heavy-chain CDR2 of the antibody or fragment thereof specifically bindable to the NTD of SARS-CoV-2 preferably contains the amino acid sequence selected from the group consisting of formulas (B-1-1) and (B-2-1) or from the group consisting of the formulas (B-1-2) to (B-1-13), (B-2-2), and (B-2-3), more preferably the amino acid sequence selected from the group consisting of formulas (B-1-2) to (B-1-10), (B-2-2) and (B-2-3); particularly more preferably the amino acid sequence selected from the group consisting of formulas (B-1-2) to (B-1-9) and (B-2-3), or an amino acid sequence having at least 90% identity to any of these amino acid sequences. The identity is preferably at least 91%, at least 92%, at least 93%, at least 94%, or at least 95%.

In an embodiment, the heavy-chain CDR3 of the antibody or fragment thereof specifically bindable to the NTD of SARS-CoV-2 preferably contains the following amino acid sequence:
· the amino acid sequence represented by any of formulas (C-1) to (C-8) shown in Table 4,
· an amino acid sequence having at least 90% (preferably at least 95%) identity to the amino acid sequence represented by any of formulas (C-1) to (C-8), or
· an amino acid sequence formed of the amino acid sequence represented by any of formulas (C-1) to (C-8) with 1 to 3 (preferably 1 or 2, more preferably 1) amino acid mutations (preferably conservative substitution).

**Table 4**

| | | |
|---|---|---|
| X^{C11}X^{C21}X^{C31}X^{C41}X^{C51}X^{C61}X^{C71}X^{C82}X^{C91}X^{C101}X^{C111}X^{C121}X^{C131}X^{C141}X^{C151} (C-1) | X^{C12}RDGGSYX^{C82}SPIX^{C122}X^{C132}QX^{C152} (C-1-1) | ARDGGSYYSPINFQF (C-1-2, SEQ ID NO: 26) |
| wherein | | SRDGGSYHSPISCQY (C-1-3, SEQ ID NO: 27) |
| X^{C11} represents A or S, | wherein | |
| X^{C21} represents R, T, or K, | X^{C12} represents A or S, | |
| X^{C31} represents D, N, S, or Y, | X^{C82} represents Y, H, or S, | ARDGGSYSSPINFQY (C-1-4, SEQ ID NO: 28) |
| X^{C41} represents G, S, or Y, | X^{C122} represents N or S, | |
| X^{C51} represents G, I, or D, | X^{C132} represents F or C, | |
| X^{C61} represents S or I, | X^{C152} represents F or Y. | SPDGGSYSSPINFQY (C-1-5, SEQ ID NO: 29) |
| X^{C71} represents Y, V, or A, | | |
| X^{C81} represents Y, H, S, T, K, or E, | | |
| X^{C91} represents S, I, or D, | | |
| X^{C101} represents P or Y, | ARDGGSVTSPIYFQV (C-1-6, SEQ ID NO: 30) | |
| X^{C111} represents I, Y, G, P, T, A, or V, | | |
| X^{C121} represents N, S, Y, P, G, or F, | | |
| X^{C131} represents F, C, M, or L, | ARDGGSVKSPIYFQY (C-1-7, SEQ ID NO: 31) | |
| X^{C141} represents Q or D, | | |
| X^{C151} represents F, Y, V, or S. | | |
| X^{C13}X^{C23}X^{C33}X^{C43}X^{C53}X^{C63}X^{C73}X^{C83} (C-2) | | |
| wherein | ATX^{C34}X^{C44}X^{C54}X^{C64}X^{C74}X^{C84} (C-2-1) | ATNYNFQY (C-2-2, SEQ ID NO: 32) |
| X^{C13} represents A or S, | wherein | |
| X^{C23} represents R, T, or K, | X^{C34} represents N or S, | |
| X^{C33} represents D, N, S, or Y, | X^{C44} represents Y or G, | |
| X^{C43} represents I, Y, G, P, T, A, or V, | X^{C54} represents N or P, | ATSGPMDV (C-2-3, SEQ ID NO: 33) |
| x^{C53} represents N, S, Y, P, G, or F, | X^{C64} represents F or M, | |
| X^{C63} represents F, C, M, or L, | X^{C74} represents Q or D, | |
| X^{C73} represents Q or D, | X^{C84} represents Y or V. | |
| X^{C83} represents F, Y, V, or S. | | |
| X^{C15}X^{C25}X^{C35}X^{C45}X^{C55}X^{C65}X^{C75}X^{C85}X^{C95}TIFTFX^{C155}X^{C165}X^{C175}X^{C185}X^{C195} (C-3) | ARSIIAEIPTIFTFPPFDV (C-3-1, SEQ ID NO: 34) | |
| wherein | | |
| X^{C15} represents A or S, | | |
| X^{C25} represents R, T, or K, | | |
| X^{C35} represents G, S, or Y, | | |
| X^{C45} represents G, I, or D, | | |
| X^{C55} represents S or I, | | |
| X^{C65} represents Y, V, or A, | | |
| X^{C75} represents Y, H, S, T, K, or E, | | |
| X^{C85} represents S, I, or D, | | |
| X^{C95} represents P or Y, | | |
| X^{C155} represents I, Y, G, P, T, A, or V, | | |
| X^{C165} represents N, S, Y, P, G, or F, | | |
| X^{C175} represents F, C, M, or L, | | |
| X^{C185} represents Q or D, | | |
| X^{C195} represents F, Y, V, or S. | | |
| ARLWSGYALDI (C-4, SEQ ID NO: 35) | | |
| X^{C16}X^{C26}X^{C36}X^{C46}X^{C56}X^{C66}X^{C76}X^{C86}X^{C96}X^{C106}X^{C116}X^{C126}X^{C136} (C-5) | AKYYDYDYTGFDV (C-5-1, SEQ ID NO: 36) | |
| wherein | | |
| X^{C16} represents A or S, | | |
| X^{C26} represents R, T, or K, | | |
| X^{C36} represents D, N, S, or Y, | | |
| X^{C46} represents G, S, or Y, | | |
| X^{C56} represents G, I, or D, | | |
| X^{C66} represents Y, H, S, T, K, or E, | | |
| X^{C76} represents S, I, or D, | | |
| X^{C66} represents P or Y, | | |
| X^{C96} represents I, Y, G, P, T, A, or V, | | |
| X^{C106} represents N, S, Y, P, G, or F, | | |
| X^{C116} represents F, C, M, or L, | | |
| X^{C126} represents Q or D, | | |
| X^{C136} represents F, Y, V, or S. | | |
| VX^{C27}PLLVX^{C77}HGX^{C107}X^{C117}X^{C127}X^{C137}X^{C147} (C-6) | | |
| wherein | VSPLLVVHGPFFQY (C-6-1, SEQ ID NO: 37) | |
| X^{C27} represents S or K, | | |
| X^{C77} represents V, Y, or L, | | |
| X^{C117} represents I, Y, G, P, T, A, or V, | VSPLLVYHGPFFQY (C-6-2, SEQ ID NO: 38) | |
| X^{C117} represents N, S, Y, P, G, or F, | VKPLLVLHGPFFQS (C-6-3, SEQ ID NO: 39) | |
| X^{C117} represents F, C, M, or L, | | |
| x^{C137} represents Q or D, | | |
| X^{C117} represents F, Y, V, or S. | | |
| VSPSX^{C58}X^{C68}X^{C78}X^{C88}X^{C98}X^{C108} (C-7) | VSPSGAGLDV (C-7-1, SEQ ID NO: 40) | |
| wherein | | |
| X^{C58} represents G or A, | | |
| X^{C68} represents I, Y, G, P, T, A, or V, | | |
| X^{C78} represents N, S, Y, P, G, or F, | | |
| X^{C88} represents F, C, M, or L, | | |
| X^{C98} represents Q or D, | | |
| X^{C108} represents F, Y, V, or S. | | |
| SPSX^{C49}X^{C59}X^{C69}X^{C79}X^{C89}X^{C99} (C-8) | SPSAVGFDV (C-8-1, SEQ ID NO: 41) | |
| wherein | | |
| X^{C49} represents G or A, | | |
| X^{C59} represents I, Y, G, P, T, A, or V, | | |
| X^{C69} represents N, S, Y, P, G, or F, | | |
| X^{C79} represents F, C, M, or L, | | |
| X^{C89} represents Q or D, | | |
| X^{C99} represents F, Y, V, or S. | | |

In formula (C-1), X^{C21} is preferably R; X^{C31} is preferably D; X^{C41} is preferably G; X^{C51} is preferably G; X^{C61} is preferably S; X^{C71} is preferably Y or V, more preferably Y; X^{C81} is preferably Y, H, S, T, or K, more preferably Y, H, or S; X^{C91} is preferably S; X^{C101} is preferably P; X^{C111} is preferably I; X^{C121} is preferably N, S, or Y, more preferably N or S; X^{C131} is preferably F or C; X^{C141} is preferably Q; and X^{C151} is preferably F, Y or V, more preferably F or Y.

Formula (C-1) is preferably formula (C-1-1) or one selected from the group consisting of formulas (C-1-2) to (C-1-7), and more preferably one selected from the group consisting of formulas (C-1-2) to (C-1-4).

In formula (C-2), X^{C13} is preferably A; X^{C23} is preferably T; X^{C33} is preferably N or S; X^{C43} is preferably Y or G; X^{C53} is preferably N, S, or P, more preferably N or P; X^{C63} is preferably F, C, or M, more preferably F or M; X^{C73} is preferably Q or D; and X^{C83} is preferably F, Y, or V, more preferably Y or V.

Formula (C-2) is preferably formula (C-2-1), and more preferably formula (C-2-2) or (C-2-3).

In formula (C-3), X^{C15} is preferably A; X^{C25} is preferably R; X^{C35} is preferably S; X^{C45} is preferably I; X^{C55} is preferably I; X^{C65} is preferably A; X^{C75} is preferably E; X^{C85} is preferably I; X^{C95} is preferably P; X^{C155} is preferably P; X^{C165} is preferably N, S, or P, more preferably P; X^{C175} is preferably F, C, or M, more preferably F; X^{C185} is preferably Q or D, more preferably D; and X^{C195} is preferably F, Y, or V, more preferably V.

Formula (C-3) is preferably formula (C-3-1).

In formula (C-5), X^{C16} is preferably A; X^{C26} is preferably K; X^{C36} is preferably Y; X^{C46} is preferably Y; X^{C56} is preferably D; X^{C66} is preferably Y; X^{C76} is preferably D; X^{C86} is preferably Y; X^{C96} is preferably T; X^{C106} is preferably G; X^{C116} is preferably F, C, or M, more preferably F; X^{C126} is preferably Q or D, more preferably D; and X^{C136} is preferably F, Y, or V, and more preferably V.

Formula (C-5) is preferably formula (C-5-1).

In formula (C-6), X^{C107} is preferably P; X^{C117} is preferably F; X^{C127} is preferably F, C, or M, more preferably F; X^{C137} is preferably Q or D, more preferably Q, and X^{C147} is preferably Y or S.

Formula (C-6) is preferably one selected from the group consisting of formulas (C-6-1) to (C-6-3).

In formula (C-7), X^{C58} is preferably G; X^{C68} is preferably A; X^{C78} is preferably G; X^{C88} is preferably L; X^{C98} is preferably Q or D, more preferably D; and X^{C108} is preferably F, Y, or V, more preferably V.

Formula (C-7) is preferably formula (C-7-1).

In formula (C-8), X^{C49} is preferably A; X^{C59} is preferably V; X^{C69} is preferably G; X^{C79} is preferably F, C, or M, more preferably F; X^{C89} is preferably Q or D, more preferably D; and X^{C99} is preferably F, Y, or V, more preferably V.

Formula (C-8) is preferably formula (C-8-1).

The heavy-chain CDR3 of the antibody or fragment thereof specifically bindable to the NTD of SARS-CoV-2 preferably contains the amino acid sequence selected from the group consisting of formulas (C-1), (C-2), (C-4), and (C-6) to (C-8), more preferably the amino acid sequence selected from the group consisting of formulas (C-1-1), (C-2-1), and (C-6) to (C-8), the amino acid sequence selected from the group consisting of formulas (C-1-2) to (C-1-7), (C-2-2), (C-2-3), (C-4), (C-6-1) to (C-6-3), (C-7-1), and (C-8-1), and more preferably the amino acid sequence selected from the group consisting of formulas (C-1-2) to (C-1-5), (C-2-2), (C-2-3), (C-4), (C-6-1), (C-7-1), and (C-8-1), or an amino acid sequence having at least 90% identity to any of these amino acid sequences. The identity is preferably at least 91%, at least 92%, at least 93%, at least 94%, or at least 95%.

In an embodiment, the light-chain CDR1 of the antibody or fragment thereof specifically bindable to the NTD of SARS-CoV-2 preferably contains the following amino acid sequence:
· the amino acid sequence represented by any of formulas (D-1) to (D-3) shown in Table 5,
· an amino acid sequence having at least 90% (preferably at least 95%) identity to the amino acid sequence represented by any of formulas (D-1) to (D-3), or
· an amino acid sequence formed of the amino acid sequence represented by any of formulas (D-1) to (D-3) with 1 to 3 (preferably 1 or 2, more preferably 1) amino acid mutations (preferably conservative substitution).

**Table 5**

| | |
|---|---|
| SX^{D21}X^{D31}X^{D41}X^{D51}X^{D61}X^{D71} (D-1) | SEYKHYN (D-1-1, SEQ ID NO: 42) |
| wherein | |
| X^{D21} represents E, D, Q, or G, | SEHRSYY (D-1-2, SEQ ID NO: 43) |
| X^{D31} represents H, Y, G, or S, | SDYSHYS (D-1-3, SEQ ID NO: 44) |
| X^{D41} represents R, S, I, K, or L, | SQGINKW (D-1-4, SEQ ID NO: 45) |
| X^{D51} represents S, H, N, or F, | SGHSSYY (D-1-5, SEQ ID NO: 46) |
| | SEHSSYY (D-1-6, SEQ ID NO: 47) |
| X^{D61} represents Y or K, | SDHSSYY (D-1-7, SEQ ID NO: 48) |
| X^{D71} represents Y, S, W, or N. | |
| SX^{D23}X^{D33}X^{D43}X^{D53}X^{D63}X^{D73}GKNKDL (D-2) | SQSLFYSGKNKDL (D-2-1, SEQ ID NO: 49) |
| wherein | |
| X^{D23} represents E, D, Q, or G, | |
| X^{D33} represents H, Y, G, or S, | |
| X^{D43} represents R, S, I, K, or L, | |
| X^{D53} represents S, H, N, or F, | |
| X^{D63} represents Y or K, | |
| X^{D73} represents Y, S, W, or N. | |
| NIGGKT (D-3, SEQ ID NO: 50) | |

Formula (D-1) is preferably one selected from the group consisting of formulas (D-1-1) to (D-1-7).

In formula (D-2), X^{D23} is preferably Q; X^{D33} is preferably S; X^{D43} is preferably L; X^{D53} is preferably F; X^{D63} is preferably Y or K, more preferably Y; and X^{D73} is preferably Y, S, or W, more preferably S.

Formula (D-2) is preferably formula (D-2-1).

The light-chain CDR1 of the antibody or fragment thereof specifically bindable to the NTD of SARS-CoV-2 preferably contains the amino acid sequence of formula (D-1), more preferably the amino acid sequence selected from the group consisting of formulas (D-1-1) to (D-1-7), or an amino acid sequence having at least 90% identity to any of these amino acid sequences. The identity is preferably at least 91%, at least 92%, at least 93%, at least 94%, or at least 95%.

In an embodiment, the light-chain CDR2 of the antibody or fragment thereof specifically bindable to the NTD of SARS-CoV-2 preferably contains the following amino acid sequence:
· the amino acid sequence represented by any of formulas (E-1) to (E-3) shown in Table 6,
· an amino acid sequence having at least 90% (preferably at least 95%) identity to the amino acid sequence represented by any of formulas (E-1) to (E-3), or
· an amino acid sequence formed of the amino acid sequence represented by any of formulas (E-1) to (E-3) with 1 to 3 (preferably 1 or 2, more preferably 1) amino acid mutations (preferably conservative substitution).

**Table 6**

| | | |
|---|---|---|
| | | LKSDGSH (E-1-2, SEQ ID NO: 51) |
| | X^{E12}X^{E22}SDGSX^{E72} (E-1-1) | VKSDGSH (E-1-3, SEQ ID NO: 52) |
| X^{E11}X^{E21}SX^{E41}GSX^{E71} (E-1) | wherein | |
| | X^{E12} represents L, V, or I, | |
| wherein | X^{E22} represents K, or E, | IESDGSH (E-1-4, SEQ ID NO: 53) |
| X^{E11} represents L, V, or I, | X^{E72} represents H or L. | |
| X^{E21} represents K, N, or E, | | VKSDGSL (E-1-5, SEQ ID NO: 54) |
| X^{E41} represents D, Y, or K, | | |
| X^{E71} represents H or L. | LNSYGSH (E-1-6, SEQ ID NO: 55) | |
| | LNSKGSH (E-1-7, SEQ ID NO: 56) | |
| | IESKGSH (E-1-8, SEQ ID NO: 57) | |
| VGTSDIVG (E-2, SEQ ID NO: 58) | | |
| X^{E13}X^{E23}X^{E33} (E-3) | KAN (E-3-1) | |
| wherein | | |
| X^{E13} represents K, S, or W, | SHN (E-3-2) | |
| X^{E23} represents A or H, | WAS (E-3-3) | |
| X^{E33} represents N or S. | | |

In formula (E-1), X^{E21} is preferably K or E, and X^{E41} is preferably D.

Formula (E-1) is preferably formula (E-1-1) or one selected from the group consisting of formulas (E-1-2) to (E-1-8), and more preferably one selected from the group consisting of formulas (E-1-2) to (E-1-5).

In formula (E-3), X^{E13} is preferably K, X^{E23} is preferably A, and X^{E33} is preferably N.

Formula (E-3) is preferably one selected from the group consisting of formulas (E-3-1) to (E-3-3), and more preferably formula (E-3-1).

The light-chain CDR2 of the antibody or fragment thereof specifically bindable to the NTD of SARS-CoV-2 preferably contains the amino acid sequence selected from the group consisting of formulas (E-1-1), (E-2), and (E-3), or the amino acid sequence selected from the group consisting of formulas (E-1-2) to (E-1-8), (E-2), and (E-3-1) to (E-3-3); more preferably the amino acid sequence selected from the group consisting of formulas (E-1-2) to (E-1-5), (E-2), and (E-3-1); or an amino acid sequence having at least 90% identity to any of these amino acid sequences. The identity is preferably at least 91%, at least 92%, at least 93%, at least 94%, or at least 95%.

In an embodiment, the light-chain CDR3 of the antibody or fragment thereof specifically bindable to the NTD of SARS-CoV-2 preferably contains the following amino acid sequence:
· the amino acid sequence represented by any of formulas (F-1) to (F-7) shown in Table 7,
· an amino acid sequence having at least 90% (preferably at least 95%) identity to the amino acid sequence represented by any of formulas (F-1) to (F-7), or
· an amino acid sequence formed of the amino acid sequence represented by any of formulas (F-1) to (F-7) with 1 to 3 (preferably 1 or 2, more preferably 1) amino acid mutations (preferably conservative substitution).

**Table 7**

| | | |
|---|---|---|
| GX^{F21}X^{F31}X^{F41}X^{F51}X^{F61}X^{F71}X^{F81}X^{F91}XF¹⁰¹ (F-1) | GVSX^{F42}SGGHX^{F92}X^{F102} (F-1-1) | GVSYSGGHNI (F-1-2, SEQ ID NO: 59) |
| | wherein | |
| wherein | X^{F42} represents Y or F, | GVSFSGGHGV (F-1-3, SEQ ID NO: 60) |
| X^{F21} represents V or A, | X^{F92} represents N or G, | |
| X^{F31} represents S, D, N, G, or K, | X^{F102} represents I or V. | GVSYSGGHNV (F-1-4, SEQ ID NO: 61) |
| X^{F41} represents Y, F, V, L, or H, | | |
| X^{F51} represents S, K, or N, | GVSVSGGHNL (F-1-5, SEQ ID NO: 62) | |
| X^{F61} represents G or N, | | |
| X^{F71} represents G, A, E, or K, | | |
| X^{F81} represents H, S, Y, or V, | GVSLKGGHNL (F-1-6, SEQ ID NO: 63) | |
| X^{F91} represents N, G, or S, | | |
| X^{F101} represents I, V, L, or Y. | | |
| GX^{F23}X^{F33}X^{F43}X^{F53}X^{F63}X^{F73}X^{F83}X^{F93}YX^{F113} (F-2) | GADYSGASSYV (F-2-1, SEQ ID NO: 64) | |
| wherein | | |
| X^{F23} represents V or A, | | |
| X^{F33} represents S, D, N, G, or K, | | |
| X^{F43} represents Y, F, V, L, or H, | | |
| X^{F53} represents S, K, or N, | | |
| X^{F63} represents G or N, | | |
| X^{F73} represents G, A, E, or K, | | |
| X^{F83} represents H, S, Y, or V, | | |
| X^{F93} represents N, G, or S, | | |
| X^{F113} represents I, V, L, or Y. | | |
| GX^{F24}X^{F34}X^{F44}X^{F54}IX^{F74}X^{F84}X^{F94}YX^{F114}YX^{F134} (F-3) | GANHNIGESYGYV (F-3-1, SEQ ID NO: 65) | |
| wherein | | |
| X^{F24} represents V or A, | | |
| X^{F34} represents S, D, N, G, or K | | |
| X^{F44} represents Y, F, V, L, or H, | | |
| X^{F54} represents S, K, or N, | | |
| X^{F74} represents G or N, | | |
| X^{F84} represents G, A, E, or K, | | |
| X^{F94} represents H, S, Y, or V, | | |
| X^{F114} represents N, G, or S, | | |
| X^{F134} represents I, V, L, or Y. | | |
| GX^{F25}X^{F35}X^{F45}X^{F55}X^{F65}X^{F75}X^{F85}X^{F95} (F-4) | GVX^{F36}YX^{F56}X^{F66}X^{F76}X^{F86}X^{F96} (F-4-1) | GVGYNNKYV (F-4-2, SEQ ID NO: 66) |
| wherein | | |
| X^{F25} represents V or A, | wherein | GVGYSGGYV (F-4-3, SEQ ID NO: 67) |
| X^{F35} represents S, D, N, G, or K, | X^{F36} represents S, G, or K, | |
| X^{F45} represents Y, F, V, L, or H, | X^{F56} represents S or N, | GVSYSGGYV (F-4-4, SEQ ID NO: 68) |
| X^{F55} represents S, K, or N, | X^{F66} represents G or N, | |
| X^{F65} represents G or N, | X^{F76} represents G or K, | GVKYSGGW (F-4-5, SEQ ID NO: 69) |
| X^{F75} represents G, A, E, or K, | X^{F86} represents Y or V, | |
| X^{F85} represents H, S, Y, or V, | X^{F96} represents V or Y. | GVSYSGGYY (F-4-6, SEQ ID NO: 70) |
| X^{F95} represents I, V, L, or Y. | | |
| QYDSTPPLT (F-5, SEQ ID NO: 71) | | |
| QVWDSYTYV (F-6, SEQ ID NO: 72) | | |
| QFINGPLT (F-7, SEQ ID NO: 73) | | |

In formula (F-1), X^{F21} is preferably V; X^{F31} is preferably S; X^{F41} is preferably Y, F, V, or L, more preferably Y or F; X^{F51} is preferably S or K, more preferably S; X^{F61} is preferably G; X^{F71} is preferably G; X^{F81} is preferably H; X^{F91} is preferably N or G; and X^{F101} is preferably I, V, or L, more preferably I or V.

Formula (F-1) is preferably formula (F-1-1) or one selected from the group consisting of formulas (F-1-2) to (F-1-6), and more preferably one selected from the group consisting of formulas (F-1-2) to (F-1-4).

In formula (F-2), X^{F23} is preferably A; X^{F33} is preferably S or D, more preferably D; X^{F43} is preferably Y, F, V, or L, more preferably Y or F, still more preferably Y; X^{F53} is preferably S or K, more preferably S; X^{F63} is preferably G; X^{F73} is preferably G or A, more preferably A; X^{F83} is preferably H or S, preferably S; X^{F93} is preferably S; and X^{F113} is preferably I, V, or L, more preferably I or V, and still more preferably V.

Formula (F-2) is preferably formula (F-2-1).

In formula (F-3), X^{F24} is preferably A; X^{F34} is preferably S, D, or N, more preferably N; X^{F44} is preferably Y, F, or H, more preferably H; X^{F54} is preferably S or N, more preferably N; X^{F74} is preferably G; X^{F84} is preferably G or E, more preferably E; X^{F94} is preferably H or S, preferably S; X^{F114} is preferably G; and X^{F134} is preferably I, V, or L, more preferably I or V, still more preferably V.

Formula (F-3) is preferably formula (F-3-1).

In formula (F-4), X^{F25} is preferably V; X^{F35} is preferably S, G, or K; X^{F45} is preferably Y; X^{F55} is preferably S or N; X^{F65} is preferably G or N; X^{F75} is preferably G or K; X^{F85} is preferably Y or V; and X^{F95} is preferably V or Y.

Formula (F-4) is preferably formula (F-4-1), more preferably one selected from the group consisting of formulas (F-4-2) to (F-4-6), and still more preferably one selected from the group consisting of formulas (F-4-2) to (F-4-4).

The light-chain CDR3 of the antibody or fragment thereof specifically bindable to the NTD of SARS-CoV-2 preferably contains the amino acid sequence selected from the group consisting of formulas (F-1) to (F-5); more preferably the amino acid sequence selected from the group consisting of formulas (F-1-1), (F-2-1), (F-3-1), (F-4-1), and (F-5); still more preferably the amino acid sequence selected from the group consisting of formulas (F-1-2) to (F-1-6), (F-2-1), (F-3-1), (F-4-2) to (F-4-6), and (F-5); and particularly more preferably the amino acid sequence selected from the group consisting of formulas (F-1-2) to (F-1-4), (F-2-1), (F-3-1), (F-4-2) to (F-4-4), and (F-5); or an amino acid sequence having at least 90% identity to any of these amino acid sequences. The identity is preferably at least 91%, at least 92%, at least 93%, at least 94%, or at least 95%.

Table 8 shows preferable examples of combinations of the heavy-chain CDR1 to CDR3 and the light-chain CDR1 to CDR3 of the antibody or fragment thereof specifically bindable to the NTD of SARS-CoV-2.

**Table 8**

| Combination | Heavy-chain CDR1 | Heavy-chain CDR2 | Heavy-chain CDR3 | Light-chain CDR1 | Light-chain CDR2 | Light-chain CDR3 |
|---|---|---|---|---|---|---|
| N-1 | (A-1) | (B-1) or (B-2) | (C-1), (C-2), (C-4), (C-6), (C-7), or (C-8) | (D-1) | (E-1) , (E-2), or (E-3) | (F-1), (F-2), (F-3), (F-4), or (F-5) |
| N-2 | (A-1-1) | (B-1-1) or (B-2-1) | (C-1-1), (C-2-1), (C-4), (C-6), (C-7), or (C-8) | (D-1-1) | (E-1-1), (E-2), or (E-3) | (F-1-1), (F-2-1), (F-3), (F-4-1), or (F-5) |
| N-3 | (A-1-2), (A-1-3), (A-1-4), (A-1-5), (A-1-6), (A-1-7), or (A-1-8) | (B-1-2), (B-1-3), (B-1-4), (B-1-5), (B-1-6), (B-1-7), (B-1-8), (B-1-9), or (B-2-3) | (C-1-2), (C-1-3), (C-1-4), (C-1-5), (C-2-2), (C-2-3), (C-4), (C-6-1), (C-7-1), or (C-8-1) | (D-1-1), (D-1-2), (D-1-3), (D-1-4), (D-1-5), (D-1-6), or (D-1-7) | (E-1-2), (E-1-3), (E-1-4), (E-1-5), (E-2), or (E-3-1) | (F-1-2), (F-1-3), (F-1-4), (F-2-1), (F-3-1), (F-4-2), (F-4-3), (F-4-4), or (F-5) |
| N-4 | (A-1-2) | (B-1-2) | (C-1-2) | (D-1-2) | (E-1-2) | (F-1-2) |
| N-5 | (A-1-3) | (B-1-3) | (C-2-2) | (D-1-3) | (E-2) | (F-2-1) |
| N-6 | (A-1-4) | (B-1-4) | (C-2-3) | (D-1-4) | (E-3-1) | (F-5) |
| N-7 | (A-1-5) | (B-1-5) | (C-1-3) | (D-1-5) | (E-1-3) | (F-1-3) |
| N-8 | (A-1-8) | (B-2-3) | (C-4) | (D-1-1) | (E-1-5) | (F-3-1) |
| N-9 | (A-1-2) | (B-1-6) | (C-1-4) | (D-1-6) | (E-1-2) | (F-1-4) |
| N-10 | (A-1-7) | (B-1-8) | (C-7-1) | (D-1-6) | (E-1-2) | (F-4-2) |
| N-11 | (A-1-7) | (B-1-9) | (C-8-1) | (D-1-7) | (E-1-2) | (F-4-3) |
| N-12 | (A-1-6) | (B-1-7) | (C-6-1) | (D-1-6) | (E-1-4) | (F-4-4) |
| N-13 | (A-1-2) | (B-1-6) | (C-1-5) | (D-1-6) | (E-1-3) | (F-1-4) |
| N-14 | (A-1-2), (A-1-3), (A-1-4), (A-1-5), (A-1-6), (A-1-7), (A-1-8), (A-1-9), or (A-2-2) | (B-1-2), (B-1-3), (B-1-4), (B-1-5), (B-1-6), (B-1-7), (B-1-8), (B-1-9), (B-1-10), (B-1-11), (B-1-12), (B-1-13), (B-2-2), or (B-2-3) | (C-1-2), (C-1-3), (C-1-4), (C-1-5), (C-1-6), (C-1-7), (C-2-2), (C-2-3), (C-3-1), (C-4), (C-5-1), (C-6-1), (C-6-2), (C-6-3), (C-7-1), or (C-8-1) | (D-1-1), (D-1-2), (D-1-3), (D-1-4), (D-1-5), (D-1-6), (D-1-7), (D-2-1), or (D-3) | (E-1-2), (E-1-3), (E-1-4), (E-1-5), (E-1-6), (E-1-7), (E-1-8), (E-2), (E-3-1), (E-3-2), or (E-3-3) | (F-1-2), (F-1-3), (F-1-4), (F-1-5), (F-1-6), (F-2-1), (F-3-1), (F-4-2), (F-4-3), (F-4-4), (F-4-5), (F-4-6), (F-5), (F-6), or (F-7) |

It is also preferred that in combinations N-3 to N-14, one to three amino acids are mutated (preferably conservatively substituted) in the amino acid sequence of at least one CDR of the heavy-chain CDR1 to CDR3 and the light-chain CDR1 to CDR3.

### 3. Antibody or fragment thereof specifically bindable to the CTD of the N protein of SARS-CoV-2

The CTD (simply "CTD") of the N protein of SARS-CoV-2 means the amino acid region at positions 247 to 419 from the N-terminus of the N protein and has the following amino acid sequence:

TKKSAAEASKKPRQKRTATKAYNVTQAFGRRGPEQTQGNFGDQELIRQGTDYKHWPQIAQFAPSA
SAFFGMSRIGMEVTPSGTWLTYTAAIKLDDKDPNFKDQVILLNKHIDAYKTFPPTEPKKDKKKA
DETQALPQRQKKQQTVTLLPAADLDDFSKQLQQSMSSADSTQA (SEQ ID NO: 2)

The antibody or fragment thereof specifically bindable to the CTD of SARS-CoV-2 preferably does not bind to the CTD of SARS-CoV or has low bindability to the CTD of SARS-CoV. In the amino acid sequence of SEQ ID NO: 2, for example, the 267th A, 290th E, 334th T, 345th N, and 349th Q from the N-terminus of the N protein differ from their corresponding amino acid in the CTD of SARS-CoV. The antibody or fragment thereof against the N protein of SARS-CoV-2 (in particular, the antibody or fragment thereof specifically bindable to the CTD of SARS-CoV-2) preferably binds to a region (or an epitope) containing one or more amino acids selected from the group consisting of the 267th A, 290th E, 334th T, 345th N, and 349th Q from the N-terminus of the N protein in the amino acid sequence of SEQ ID NO: 2.

In an embodiment, the antibody or fragment thereof against the N protein of SARS-CoV-2 (in particular, the antibody or fragment thereof specifically bindable to the CTD of SARS-CoV-2) may bind to the amino acid region at positions 247 to 419 from the N-terminus of the N protein, preferably to the dimeric domain of the CTD, or preferably to the amino acid region at positions 247 to 364 or 256 to 364 from the N-terminus of the N protein.

In an embodiment, the antibody or fragment thereof against the N protein of SARS-CoV-2 (in particular, the antibody or fragment thereof specifically bindable to the CTD of SARS-CoV-2) preferably binds to the amino acid region at positions 250 to 257, 374 to 382, or 394 to 405 from the N terminus of the N protein; i.e., it is preferred that the epitope is present in the region composed of any of the amino acid sequences of SEQ ID Nos: 150 to 152 on the N protein.

The antibody or fragment thereof specifically bindable to the CTD of SARS-CoV-2 can be produced according to known methods in combination, such as by a method including steps (P1) to (P5) and the following step (P6'):
(P6') obtaining antibodies that specifically bind to the CTD from the antibodies recovered in step (P5).

Step (P6') can be performed in the same manner as step (P6) by selecting antibodies that specifically bind to the CTD according to a known screening method, such as enzyme-linked immunosorbent assay (ELISA), western blotting, or immunostaining.

The method including steps (P1) to (P5) and (P6') can provide multiple types of antibodies or fragments thereof that specifically bind to the CTD of SARS-CoV-2. (The antibodies or fragments thereof differ from each other in terms of the amino acid sequence of at least one CDR of the heavy-chain CDR1 to CDR3 and the light-chain CDR1 to CDR3.)

In an embodiment, the heavy-chain CDR1 of the antibody or fragment thereof specifically bindable to the CTD of SARS-CoV-2 preferably contains the following amino acid sequence:
· the amino acid sequence represented by either formula (G-1) or (G-2) shown in Table 9,
· an amino acid sequence having at least 90% (preferably at least 95%) identity to the amino acid sequence represented by formula (G-1) or (G-2), or
· an amino acid sequence formed of the amino acid sequence represented by either formula (G-1) or (G-2) with 1 to 3 (preferably 1 or 2, more preferably 1) amino acid mutations (preferably conservative substitution).

**Table 9**

| | | |
|---|---|---|
| | | GFIFSNYF (G-1-2, SEQ ID NO: 74) |
| | | GFTFNTYT (G-1-3, SEQ ID NO: 75) |
| | GX^{G22}X^{G32}X^{G42}X^{G52}X^{G62}X^{G72}X^{G82} (G-1-1) | GFTFSNYY (G-1-4, SEQ ID NO: 76) |
| GX^{G21}X^{G31}X^{G41}X^{G51}X^{G61}X^{G71}X^{G81} (G-1) | wherein | |
| wherein | X^{G22} F represents or L, | GLSLTNYT (G-1-5, SEQ ID NO: 77) |
| X^{G21} represents F or L, | X^{G32} represents I, T, or S, | |
| X^{C31} represents I, T, S, or F, | X^{G42} represents F or L, | GFSFSNSW (G-1-6, SEQ ID NO: 78) |
| X^{G41} represents F or L, | X^{G52} represents S, N, T, or R, | |
| X^{G51} represents S, N, T, R, or D, | X^{G62} represents N or T, | GFTFSNYW (G-1-7, SEQ ID NO: 79) |
| X^{G61} represents N, T, or D, | X^{G72} represents Y, S, or H, | |
| X^{G71} represents Y, S, or H, | X^{G82} represents F, T, Y, or W. | GFTFRNHW (G-1-8, SEQ ID NO: 80) |
| X^{G81} represents F, T, Y, W, or G. | | |
| | | GFTFSNHW (G-1-9, SEQ ID NO: 81) |
| | GFFFSNSW (G-1-10, SEQ ID NO: 82) | |
| | GFTFDDYG (G-1-11, SEQ ID NO: 83) | |
| KLSERY (G-2, SEQ ID NO: 84) | | |

In formula (G-1), X^{G31} is preferably I, T, or S; X^{G51} is preferably S, N, T, or R; X^{G61} is preferably N or T; X^{G71} is preferably Y, S, or H; and X^{C81} is preferably F, T, Y, or W.

Formula (G-1) is preferably formula (G-1-1), more preferably one selected from the group consisting of formulas (G-1-2) to (G-1-11), and still more preferably one selected from the group consisting of formulas (G-1-2) to (G-1-9).

The heavy-chain CDR1 of the antibody or fragment thereof specifically bindable to the CTD of SARS-CoV-2 preferably contains the amino acid sequence of formula (G-1-1), more preferably the amino acid sequence selected from the group consisting of formulas (G-1-2) to (G-1-11), still more preferably the amino acid sequence selected from the group consisting of formulas (G-1-2) to (G-1-9), or an amino acid sequence having at least 90% identity to any of these amino acid sequences. The identity is preferably at least 91%, at least 92%, at least 93%, at least 94%, or at least 95%.

In an embodiment, the heavy-chain CDR2 of the antibody or fragment thereof specifically bindable to the CTD of SARS-CoV-2 preferably contains the following amino acid sequence:
· the amino acid sequence represented by any of formulas (H-1) to (H-3) shown in Table 10,
· an amino acid sequence having at least 90% (preferably at least 95%) identity to the amino acid sequence represented by any of formulas (H-1) to (H-3), or
· an amino acid sequence formed of the amino acid sequence represented by any of formulas (H-1) to (H-3) with 1 to 3 (preferably 1 or 2, more preferably 1) amino acid mutations (preferably conservative substitution).

**Table 10**

| | | |
|---|---|---|
| | | ISGDSTYI (H-1-2, SEQ ID NO: 85) |
| | | IGNSGSKT (H-1-3, SEQ ID NO: 86) |
| | | ISGDGSNI (H-1-4, SEQ ID NO: 87) |
| | | ISGDSSNI (H-1-5, SEQ ID NO: 88) |
| | IX^{H22}X^{H32}X^{H42}X^{H52}X^{H62}X^{H72}X^{HB2} (H-1-1) | IKGDSSII (H-1-6, SEQ ID NO: 89) |
| IX^{H21}X^{H31}X^{H41}X^{H51}X^{H61}X^{H71}X^{H81} (H-1) | wherein | INPDDGSP (H-1-7, SEQ ID NO: 90) |
| wherein | X^{H22} represents S, G, K, or N, | |
| X^{H21} represents S, G, K, or N, | X^{H32} represents G, N, S, T, or P, | IKGDSSII (H-1-8, SEQ ID NO: 91) |
| X^{H31} represents G, N, S, Y, T, or P, | X^{H42} represents D, S, or A, | |
| X^{H41} represents D, S, T, or A, | X^{H52} represents S, G, or D, | INSDGSNT (H-1-9, SEQ ID NO: 92) |
| X^{H51} represents S, G, or D, | X^{H62} represents T, S, G, or R, | INTDGSST (H-1-10, SEQ ID NO: 93) |
| X^{H61} represents T, S, A, G, or R, | X^{H72} represents Y, K, N, T, I, or S, | |
| X^{H71} represents Y, K, N, T, I, or S, | X^{H82} represents I, T, P, or V. | IKGDSRTI (H-1-11, SEQ ID NO: 94) |
| X^{H81} represents I, T, P, or V. | | |
| | | ISGASSNI (H-1-12, SEQ ID NO: 95) |
| | | ISGDSSNV (H-1-13, SEQ ID NO: 96) |
| | | IKGDSSTI (H-1-14, SEQ ID NO: 97) |
| | | INSDGSST (H-1-15, SEQ ID NO: 98) |
| | IGYSGASI (H-1-16, SEQ ID NO: 99) | |
| | ISGTSSNI (H-1-17, SEQ ID NO: 100) | |
| MWSDGDT (H-2, SEQ ID NO: 101) | | |
| NDS (H-3) | | |

In formula (H-1), X^{H31} is preferably G, N, S, T, or P; X^{H41} is preferably D, S, or A; and X^{H61} is preferably T, S, G, or R.

Formula (H-1) is preferably formula (H-1-1) or one selected from the group consisting of formulas (H-1-2) to (H-1-17), more preferably one selected from the group consisting of formulas (H-1-2) to (H-1-15), and still more preferably one selected from the group consisting of formulas (H-1-2) to (H-1-7) and (H-1-9) to (H-1-12).

The heavy-chain CDR2 of the antibody or fragment thereof specifically bindable to the CTD of SARS-CoV-2 preferably contains the amino acid sequence selected from the group consisting of formulas (H-1) and (H-2), more preferably the amino acid sequence selected from the group consisting of formulas (H-1-1) and (H-2), or from the group consisting of formulas (H-1-2) to (H-1-17) and (H-2), still more preferably the amino acid sequence selected from the group consisting of formulas (H-1-2) to (H-1-15) and (H-2), and particularly preferably the amino acid sequence selected from the group consisting of formulas (H-1-2) to (H-1-7), (H-1-9) to (H-1-12), and (H-2), or an amino acid sequence having at least 90% identity to any of these amino acid sequences. The identity is preferably at least 91%, at least 92%, at least 93%, at least 94%, or at least 95%.

In an embodiment, the heavy-chain CDR3 of the antibody or fragment thereof specifically bindable to the CTD of SARS-CoV-2 preferably contains the following amino acid sequence:
. the amino acid sequence represented by any of formulas (I-1) to (I-11) shown in Table 11,
· an amino acid sequence having at least 90% (preferably at least 95%) identity to the amino acid sequence represented by any of formulas (I-1) to (I-11), or
· an amino acid sequence formed of the amino acid sequence represented by any of formulas (I-1) to (I-11) with 1 to 3 (preferably 1 or 2, more preferably 1) amino acid mutations (preferably conservative substitution).

**Table 11**

| | | |
|---|---|---|
| X^{I11}X^{I21}X^{I31}X^{I41}X^{I51}X^{I61}X^{I71} (I-1) | X^{I12}X^{I22}X^{I32}X^{I42}X^{I32}X^{I62}X¹⁷² (I-1-1) | TINWGDV (I-1-2, SEQ ID NO: 102) STLGVGV (I-1-3, SEQ ID NO: 103) TTLGVGV (I-1-4, SEQ ID NO: 104) STTGFDV (I-1-5, SEQ ID NO: 105) STLGNFL (I-1-6, SEQ ID NO: 106) TITWGDV (I-1-7, SEQ ID NO: 107) |
| wherein | wherein | |
| X^{I11} represents S or T, X^{I21} represents I or T, X^{I31} represents N, L, D, or T, X^{I41} represents W or G, X^{I51} represents G, V, F, or N, X^{I61} represents D, G, F, or N, X^{I71} represents V or L. | X^{I12} represents S or T, X^{I22} represents I or T, X^{I32} represents N, L, or T, X^{I42} represents W or G, X^{I52} represents G, V, F, or N, X^{I62} represents D, F, or G, X^{I72} represents V or L. | |
| | TIDWGNV (I-1-8, SEQ ID NO: 108) | |
| ARTSRIVADLVTMGYALDF (I-2, SEQ ID NO: 109) | | |
| ARDWKTGTTGLPFDL (I-3, SEQ ID NO: 110) | | |
| AREGVITVGTWGDV (I-4, SEQ ID NO: 111) | | |
| ATSEY (I-5, SEQ ID NO: 112) | | |
| TTATDV (I-6, SEQ ID NO: 113) | | |
| TPATDV (I-7, SEQ ID NO: 114) | | |
| SPMTIHGLDT (I-8, SEQ ID NO: 115) | | |
| GSGWV (I-9, SEQ ID NO: 116) | | |
| GSGWY (I-10, SEQ ID NO: 117) | | |
| LSSESDDARV (I-11, SEQ ID NO: 118) | | |

In formula (I-1), X^{I31} is preferably N, L, or T, and X^{I61} is preferably D, F, or G.

Formula (I-1) is preferably formula (I-1-1) or one selected from the group consisting of formulas (1-1-2) to (I-1-8), more preferably one selected from the group consisting of formulas (1-1-2) to (1-1-7), and still more preferably one selected from the group consisting of formulas (1-1-2) to (I-1-6) .

The heavy-chain CDR3 of the antibody or fragment thereof specifically bindable to the CTD of SARS-CoV-2 preferably contains the amino acid sequence selected from the group consisting of formulas (I-1) to (I-3) and (I-5) to (I-9), more preferably the amino acid sequence selected from the group consisting of formulas (1-1-1), (1-2), (1-3), and (1-5) to (I-9), or the amino acid sequence selected from the group consisting of formulas (1-1-2) to (1-1-8), (1-2), (1-3), and (1-5) to (I-9), still more preferably the amino acid sequence selected from the group consisting of formulas (1-1-2) to (1-1-7), (1-2), (1-3), and (1-5) to (I-9), particularly preferably the amino acid sequence selected from the group consisting of formulas (1-1-2) to (1-1-6), (1-2), (1-3), and (1-5) to (1-9), or an amino acid sequence having at least 90% identity to any of these amino acid sequences. The identity is preferably at least 91%, at least 92%, at least 93%, at least 94%, or at least 95%.

In an embodiment, the light-chain CDR1 of the antibody or fragment thereof specifically bindable to the CTD of SARS-CoV-2 preferably contains the following amino acid sequence:
· the amino acid sequence represented by any of formulas (J-1) to (J-3) shown in Table 12,
· an amino acid sequence having at least 90% (preferably at least 95%) identity to the amino acid sequence represented by any of formulas (J-1) to (J-3), or
· an amino acid sequence formed of the amino acid sequence represented by any of formulas (J-1) to (J-3) with 1 to 3 (preferably 1 or 2, more preferably 1) amino acid mutations (preferably conservative substitution).

**Table 12**

| | | |
|---|---|---|
| | KLSX^{J42}X^{J52}Y (J-1-1) | KLSNQY (J-1-2, SEQ ID NO: 119) |
| X^{J11}LX^{J31}X^{J41}X^{J51}Y (J-1) | wherein | KLSERY (J-1-3, SEQ ID NO: 120) |
| wherein | X^{J42} represents N, E, or K, | |
| X^{J11} represents K or A, | X^{J52} represents Q, R, or E. | KLSKEY (J-1-4, SEQ ID NO: 121) |
| X^{J31} represents S or P, | | |
| X^{J41} represents N, E, K, T, or D, | ALPKKY (J-1-5, SEQ ID NO: 122) | |
| X^{J51} represents Q, R, K, or E. | | |
| | KLSTQY (J-1-6, SEQ ID NO: 123) | |
| | KLSDQY (J-1-7, SEQ ID NO: 124) | |
| | | SESLLHSDGNTY (J-2-2, SEQ ID NO: 125) |
| | | SQSLLHSDGKTY (J-2-3, SEQ ID NO: 126) |
| SX^{J23}SLX^{J53}X^{J63}X^{J73}DGX^{J103}TX^{J123} (J-2), | SX^{J24}SLX^{J54}X^{J64}SDGX^{J104}TX^{J124} (J-2-1) | |
| wherein | wherein | SQSLVHSDGKTY (J-2-4, SEQ ID NO: 127) |
| X^{J23} represents E or Q, | X^{J24} represents E or Q, | SQSLLDSDGYTY (J-2-5, SEQ ID NO: 128) |
| X^{J53} represents L or V, | X^{J54} represents L or V, | |
| X^{J63} represents H, D, or Y, | X^{J64} represents H, D, or Y, | |
| X^{J73} represents S or G, | X^{J104} represents N, K, or Y, | SQSLLYSDGYTY (J-2-6, SEQ ID NO: 129) |
| X^{J103} represents N, K, or Y, | X^{J114} represents Y or F. | SQSLLYSDGYTF (J-2-7, SEQ ID NO: 130) |
| X^{J123} represents Y or F. | | |
| | | SQSLLHSDGNTY (J-2-8, SEQ ID NO: 131) |
| | SQSLLHGDGKTY (J-2-9, SEQ ID NO: 132) | |
| NIGGKA (J-3, SEQ ID NO: 133) | | |

In formula (J-1), X^{J11} is preferably K; X^{J31} is preferably S; X^{J41} is preferably N, E, or K; and X^{J51} is preferably Q, R, or E.

Formula (J-1) is preferably formula (J-1-1) or one selected from the group consisting of formulas (J-1-2) to (I-1-7), and more preferably one selected from the group consisting of formulas (J-1-2) to (J-1-4).

In formula (J-2), X^{J73} is preferably S.

Formula (J-2) is preferably formula (J-2-1) or one selected from the group consisting of formulas (J-2-2) to (J-2-9), more preferably one selected from the group consisting of formulas (J-2-2) to (J-2-8), and still more preferably one selected from the group consisting of formulas (J-2-2) to (J-2-6) .

The heavy-chain CDR3 of the antibody or fragment thereof specifically bindable to the CTD of SARS-CoV-2 preferably contains the amino acid sequence selected from the group consisting of formulas (J-1-1), (J-2-1), and (J-3), or the amino acid sequence selected from the group consisting of formulas (J-1-2) to (J-1-7), (J-2-2) to (J-2-9), and (J-3), more preferably, the amino acid sequences selected from the group consisting of formulas (J-1-2) to (J-1-4), (J-2-2) to (J-2-6), and (J-3), or an amino acid sequence having at least 90% identity to any of these amino acid sequences. The identity is preferably at least 91%, at least 92%, at least 93%, at least 94%, or at least 95%.

In an embodiment, the light-chain CDR2 of the antibody or fragment thereof specifically bindable to the CTD of SARS-CoV-2 preferably contains the following amino acid sequence:
- the amino acid sequence represented by formula (K-1) shown in Table 13,
- an amino acid sequence having at least 90% (preferably at least 95%) identity to the amino acid sequence represented by formula (K-1), or
- an amino acid sequence formed of the amino acid sequence represented by formula (K-1) with 1 to 3 (preferably 1 or 2, more preferably 1) amino acid mutations (preferably conservative substitution).

**Table 13**

| | |
|---|---|
| | NDD (K-1-1) |
| X^{K11}X^{K21}X^{K31} (K-1) | KDS (K-1-2) |
| wherein | NGN (K-1-3) |
| X^{K11} represents K, N, L, or E, | NDS (K-1-4) |
| X^{K21} represents D, G, V, or I, | KVS (K-1-5) |
| X^{K31} represents S, N, or D. | LIS (K-1-6) |
| | EVS (K-1-7) |

Formula (K-1) is preferably one selected from the group consisting of formulas (K-1-1) to (K-1-7).

The light-chain CDR2 of the antibody or fragment thereof specifically bindable to the CTD of SARS-CoV-2 preferably contains the amino acid sequence selected from the group consisting of formulas (K-1-1) to (K-1-7), or an amino acid sequence having at least 90% identity to any of these amino acid sequences. The identity is preferably at least 91%, at least 92%, at least 93%, at least 94%, or at least 95%.

In an embodiment, the light-chain CDR3 of the antibody or fragment thereof specifically bindable to the CTD of SARS-CoV-2 preferably contains the following amino acid sequence:
· the amino acid sequence represented by any of formulas (L-1) to (L-6) shown in Table 14,
· an amino acid sequence having at least 90% (preferably at least 95%) identity to the amino acid sequence represented by any of formulas (L-1) to (L-6), or
· an amino acid sequence formed of the amino acid sequence represented by any of formulas (L-1) to (L-6) with 1 to 3 (preferably 1 or 2, more preferably 1) amino acid mutations (preferably conservative substitution).

**Table 14**

| | | |
|---|---|---|
| LSX^{L31}X^{L41}X^{L51}X^{L61}X^{L71}X^{L81}X^{L91}X^{L101}X^{L111} (L-1) | LSX^{L32}X^{L42}SX^{L62}DAAX^{L102}V (L-1-1) | LSRQSDDAASV (L-1-2, SEQ ID NO: 134) |
| wherein | wherein | |
| X^{L31} represents R, S, A, or G, | X^{L32} represents R or S, | LSSESDDAARV (L-1-3, SEQ ID NO: 135) |
| X^{L41} represents Q, E, or D, | X^{L42} represents Q or E, | |
| X^{L51} represents S or G, | X^{L61} represents D or N, | LSRESNDAAWV (L-1-4, SEQ ID NO: 136) |
| X^{L61} represents D, S, or N, | X^{L102} represents S, R, or W. | |
| X^{L71} represents D, G, or T, | | |
| X^{L81} represents A, T, or Y, | | |
| X^{L91} represents A, T, or Y, | LSADSSGTYRV (L-1-5, SEQ ID NO: 137) | |
| X^{L101} represents S, R, or W, | LSRQSDTAASL (L-1-6, SEQ ID NO: 138) | |
| X^{L111} represents V, L, or Y. | LSRQGDTAASY (L-1-7, SEQ ID NO: 139) | |
| LSX^{L33}X^{L43}X^{L53}X^{L63}X^{L73}X^{L83}X^{L93}X^{L103} (L-2) | | LSGESDDAWV (L-2-1, SEQ ID NO: 140) |
| wherein | | |
| X^{L33} represents R, S, A, or G, | | |
| X^{L43} represents Q, E, or D, | | |
| X^{L53} represents S or G, | | |
| X^{L63} represents D, S, or N, | | |
| X^{L73} represents D, G, or T, | | |
| X^{L83} represents A, T, or Y, | | |
| X^{L93} represents S, R, or W, | | |
| X^{L103} represents V, L, or Y. | | |
| | | |
| X^{L14}QX^{L34}X^{L44}X^{L54}X^{L64}PX^{L84}X^{L94} (L-3) | X^{L15}QX^{L35}THDPYT (L-3-1) | LQATHDPYT (L-3-2, SEQ ID NO: 141) |
| wherein | wherein | |
| X^{L14} represents L or V, | X^{L15} represents L or V, | VQTTHDPYT (L-3-3, SEQ ID NO: 142) |
| X^{L34} represents A or T, | X^{L35} represents A or T. | |
| X^{L44} represents T or S, | | |
| X^{L54} represents H or N, | LQATHNPVS (L-3-4, SEQ ID NO: 143) | |
| X^{L64} represents D, N, or V, | | |
| | LQATHDPIT (L-3-5, SEQ ID NO: 144) | |
| X^{L84} represents Y, V, I, or L, | | |
| | VQTSHVPLT (L-3-6, SEQ ID NO: 145) | |
| X^{L94} represents T or S. | | |
| | VQTTHVPLT (L-3-7, SEQ ID NO: 146) | |
| | | QTTNDPYT (L-4-1, SEQ ID NO: 147) |
| QX^{L27}X^{L37}X^{L47}X^{L57} PX^{L77}X^{L87} (L-4) | | |
| wherein | | |
| X^{L27} represents A or T, | | |
| X^{L37} represents T or S, | | |
| X^{L47} represents H or N, | | |
| X^{L57} represents D, N, or V, | | |
| X^{L77} represents Y, V, I, or L, | | |
| X^{L87} represents T or S. | | |
| QVYDSSSFV (L-5, SEQ ID NO: 148) | | |
| WQGTDFPR (L-6, SEQ ID NO: 149) | | |

In formula (L-1), X^{L31} is preferably R or S; X^{L41} is preferably Q or E; X^{L51} is preferably S; X^{L61} is preferably D or N; X^{L71} is preferably D; X^{L81} is preferably A; X^{L91} is preferably A; X^{L101} is preferably S, R, or W; and X^{L111} is preferably V.

Formula (L-1) is preferably formula (L-1-1) or one selected from the group consisting of formulas (L-1-2) to (L-1-7), and more preferably one selected from the group consisting of formula (L-1-2) to (L-1-4).

In formula (L-2), X^{L33} is preferably R, S, or G, more preferably G; X^{L43} is preferably Q or E, more preferably E; X^{L53} is preferably S; X^{L63} is preferably D; X^{L73} is preferably D; X^{L83} is preferably A; X^{L93} is preferably W; and X^{L103} is preferably V.

Formula (L-2) is preferably formula (L-2-1).

In formula (L-3), X^{L54} is preferably H; X^{L64} is preferably D or N, or D or V; and X^{L84} is preferably Y, V, or I, or Y or L.

Formula (L-3) is preferably formula (L-3-1) or one selected from the group consisting of formulas (L-3-2) to (L-3-7), and more preferably formula (L-3-2) or (L-3-3).

In formula (L-4), X^{L27} is preferably T; X^{L37} is preferably T; X^{L47} is preferably N; X^{L57} is preferably D; X^{L77} is preferably Y; and X^{L87} is preferably T.

Formula (L-4) is preferably formula (L-4-1).

The light-chain CDR3 of the antibody or fragment thereof specifically bindable to the CTD of SARS-CoV-2 preferably contains the amino acid sequence selected from the group consisting of formulas (L-1) to (L-3), (L-5), and (L-6), more preferably the amino acid sequence selected from the group consisting of formulas (L-1-1), (L-2-1), (L-3-1), (L-5), and (L-6), or the amino acid sequence selected from the group consisting of formulas (L-1-2) to (L-1-7), (L-2-1), (L-3-2), (L-3-3), (L-5), and (L-6), still more preferably the amino acid sequence selected from the group consisting of formulas (L-1-2) to (L-1-4), (L-2-1), (L-3-2), (L-3-3), (L-5), and (L-6), or an amino acid sequence having at least 90% identity to any of these amino acid sequences. The identity is preferably at least 91%, at least 92%, at least 93%, at least 94%, or at least 95%.

Table 15 shows preferable examples of combinations of the heavy-chain CDR1 to CDR3 and the light-chain CDR1 to CDR3 of the antibody or fragment thereof specifically bindable to the CTD of SARS-CoV-2.

**Table 15**

| Combination | Heavy-chain CDR1 | Heavy-chain CDR2 | Heavy-chain CDR3 | Light-chain CDR1 | Light-chain CDR2 | Light-chain CDR3 |
|---|---|---|---|---|---|---|
| C-1 | (G-1) | (H-1) or (H-2) | (I-1), (I-2), (I-3), (I-5), (I-6), (I-7), (1-8), or (I-9) | (J-1), (J-2), or (J-3) | (K-1) | (L-1), (L-2), (L-3), (L-5), or (L-6) |
| C-2 | (G-1-1) | (H-1-1) or (H-2) | (I-1-1), (I-2), (I-3), (I-5), (I-6), (I-7), (1-8), or (I-9) | (J-1-1), (J-2-1), or (J-3) | (K-1) | (L-1-1), (L-2), (L-3-1), (L-5), or (L-6) |
| C-3 | (G-1-2), (G-1-3), (G-1-4), (G-1-5), (G-1-6), (G-1-7), (G-1-8), or (G-1-9) | (H-1-2), (H-1-3), (H-1-4), (H-1-5), (H-1-6), (H-1-7), (H-1-9), (H-1-10), (H-1-11), (H-1-12), (H-1-13), or (H-2) | (I-1-2), (I-1-3), (I-1-4), (I-1-5), (I-1-6), (I-2), (I-3), (I-5), (I-6), (I-7), (1-8), or (I-9) | (J-1-2), (J-1-3), (J-1-4), (J-2-2), (J-2-3), (J-2-4), (J-2-5), (J-2-6), (J-2-7), or (J-3) | (K-1-1), (K-1-2), (K-1-3), (K-1-4), (K-1-5), (K-1-6), or (K-1-7) | (L-1-2), (L-1-3), (L-1-4), (L-2-1), (L-3-2), (L-3-3), (L-5), or (L-6) |
| C-4 | (G-1-2) | (H-1-2) | (I-1-2) | (J-1-2) | (K-1-2) | (L-1-2) |
| C-5 | (G-1-3) | (H-1-3) | (I-2) | (J-3) | (K-1-3) | (L-5) |
| C-6 | (G-1-4) | (H-1-4) | (I-1-3) | (J-1-3) | (K-1-4) | (L-1-3) |
| C-7 | (G-1-5) | (H-2) | (I-5) | (J-2-2) | (K-1-5) | (L-6) |
| C-8 | (G-1-6) | (H-1-6) | (I-9) | (J-2-3) | (K-1-6) | (L-3-2) |
| C-9 | (G-1-6) | (H-1-6) | (I-9) | (J-2-4) | (K-1-6) | (L-3-3) |
| C-10 | (G-1-8) | (H-1-9) | (I-6) | (J-2-5) | (K-1-7) | (L-3-3) |
| C-11 | (G-1-9) | (H-1-10) | (I-7) | (J-2-6) | (K-1-7) | (L-3-3) |
| C-12 | (G-1-7) | (H-1-7) | (I-3) | (J-1-3) | (K-1-4) | (L-2-1) |
| C-13 | (G-1-7) | (H-1-11) | (I-1-5) | (J-2-7) | (K-1-7) | (L-3-6) |
| C-14 | (G-1-4) | (H-1-5) | (I-8) | (J-1-4) | (K-1-2) | (L-1-5) |
| C-15 | (G-1-4) | (H-1-12) | (I-1-4) | (J-1-3) | (K-1-4) | (L-1-3) |
| C-16 | (G-1-4) | (H-1-13) | (I-1-6) | (J-1-3) | (K-1-1) | (L-1-3) |
| C-17 | (G-1-2), (G-1-3), (G-1-4), (G-1-5), (G-1-6), (G-1-7), (G-1-8), (G-1-9), (G-1-10), (G-1-11), or (G-2) | (H-1-2), (H-1-3), (H-1-4), (H-1-5), (H-1-6), (H-1-7), (H-1-8), (H-1-9), (H-1-10), (H-1-11), (H-1-12), (H-1-13), (H-1-14), (H-1-15), (H-1-16), (H-1-17), (H-2), or (H-3) | (I-1-2), (I-1-3), (I-1-4), (I-1-5), (I-1-6), (I-1-7), (I-1-8), (I-2), (I-3), (I-4), (I-5), (I-6), (I-7), (I-8), (I-9) , (I-10), or (I-11) | (J-1-2), (J-1-3), (J-1-4), (J-1-5), (J-1-6), (J-1-7), (J-2-2), (J-2-3), (J-2-4), (J-2-5), (J-2-6), (J-2-7), (J-2-8), (J-2-9), or (J-3) | (K-1-1), (K-1-2), (K-1-3), (K-1-4), (K-1-5), (K-1-6), or (K-1-7) | (L-1-2), (L-1-3), (L-1-4), (L-1-5), (L-1-6), (L-1-7), (L-2-1), (L-3-2), (L-3-3), (L-3-4), (L-3-5), (L-3-6), (L-3-7), (L-4-1), (L-5), or (L-6) |

It is also preferred that in combinations C-3 to C-8, one to three amino acids are mutated (preferably conservatively substituted) in the amino acid sequence of at least one CDR of the heavy-chain CDR1 to CDR3 and the light-chain CDR1 to CDR3.

The regions other than the heavy-chain CDR1 to CDR3 and the light-chain CDR1 to CDR3 are not particularly limited and can be of any amino acid sequence as long as the regions are usable in antibodies. For example, the constant region for use can be, but is not limited to, the constant region of IgG1, IgG2, IgG3, IgA1, IgA2, or IgM.

The antibody or fragment thereof specifically bindable to the NTD or CTD of SARS-CoV-2 has high bindability to the N protein of SARS-CoV-2; the bindability is, for example, 0.2 or higher, preferably 0.3 or higher, more preferably 0.4 or higher, and still more preferably 0.5 or higher, as indicated by absorbance measured according to the enzyme-linked immunosorbent assay (ELISA), described in the Examples below.

The antibody or fragment thereof specifically bindable to the NTD or CTD of SARS-CoV-2 has a binding affinity (Kd) for the N protein of SARS-CoV-2 of, for example, 50 nM or lower, preferably 10 nM or lower, and, for example, 1 pM or higher. The binding affinity Kd can be measured by using Biacore (trademark) T200 (Cytiva). Specifically, an amine coupling reaction using N'-(3-dimethylaminopropyl) carbodiimide hydrochloride (NHS) and N-hydroxysuccinimide (EDC) is used to immobilize an anti-guinea pig IgG antibody to carboxymethyl dextran on a CM5 sensor chip through a covalent bond. Then, an antibody or fragment thereof specifically bindable to the NTD or CTD of SARS-CoV-2 is injected into the sensor chip to bind the antibody or fragment thereof, and the N protein of SARS-CoV-2 is further injected into the sensor chip to allow the N protein to react with the antibody or fragment thereof specifically bindable to the NTD or CTD of SARS-CoV-2. The Kd is then calculated by analyzing the reaction signal.

### 4. Polynucleotide

The polynucleotide according to the present invention preferably contains the coding sequence of the antibody or fragment thereof specifically bindable to the NTD or CTD of SARS-CoV-2. An example of the coding sequence is, but is not limited to, the base sequence encoding the antibody or fragment thereof containing the heavy-chain CDR1 to CDR3 and the light-chain CDR1 to CDR3 described in section 3 above.

In an embodiment, the polynucleotide according to the present invention preferably contains an expression cassette of the antibody or fragment thereof specifically bindable to the NTD or CTD of SARS-CoV-2. The expression cassette is not particularly limited to a specific one as long as the expression cassette allows for expression in a host cell, and contains, for example, a promoter and coding sequences placed under control of the promoter.

The promoter is not limited and can be suitably selected according to the type of the host cell. For example, various pol II promoters are usable. Examples of pol II promoters include, but are not limited to, the CMV promoter, the EF1 promoter, the SV40 promoter, and the MSCV promoter. Examples of promoters also include other promoters, such as tryptophan promoters (e.g., trc and tac), the lac promoter, the T7 promoter, the T5 promoter, the T3 promoter, the SP6 promoter, the arabinose-induced promoter, the cold-shock promoter, and the tetracycline-induced promoter.

The expression cassette may contain other elements as needed. Examples of other elements include a multiple cloning site (MCS), a drug-resistant gene, a replication origin, an enhancer sequence, a repressor sequence, an insulator sequence, a reporter protein coding sequence, and a drug-resistant gene coding sequence. These may be one type alone or a combination of two or more.

For example, the polynucleotide according to the present invention can be in the form of a vector. An appropriate vector is selected according to the intended use, host cell type, etc. For example, *E. coli* vectors include M13 phage or variants thereof, λ phage or variants thereof, and pBR322 or variants thereof (e.g. pB325, pAT153, pUC8); yeast vectors include pYepSec1, pMFa, pYES2, and pPIC3.5K; insect vectors include pAc, and pVL; and mammalian vectors include pcDNA, pCDM8, and pMT2PC.

### S. Cell

The cell according to the present invention preferably contains the polynucleotide described in section 4 above. The cell includes, for example, *Escherichia coli* such as *Escherichia coli* K12, bacteria of the genus *Bacillus,* such as *Bacillus subtilis* MI114, yeast, such as *Saccharomyces cerevisiae* AH22, Sf cell lines derived from *Spodoptera frugiperda,* High Five cell lines derived from *Trichoplusia ni,* insect cells, such as olfactory nerve cells, and animal cells. Animal cells are preferably cultured cells of mammalian origin, and specifically include COS7 cells, CHO cells, HEK293 cells, Expi293 cells, 293F cells, 293T cells, 293FT cells, Hela cells, PC12 cells, N1E-115 cells, and SH-SY5Y cells.

In an embodiment, the cell according to the present invention preferably expresses the antibody or fragment thereof specifically bindable to the NTD or CTD of SARS-CoV-2.

In an embodiment, the cell according to the present invention preferably secretes the antibody or fragment thereof specifically bindable to the NTD or CTD of SARS-CoV-2 or preferably has the antibody or fragment thereof specifically bindable to the NTD or CTD of SARS-CoV-2 on the cell surface.

### 6. Reagent or drug

The reagent or drug according to the present invention preferably contains an antibody or fragment thereof specifically bindable to the NTD or CTD of SARS-CoV-2, a polynucleotide containing the coding sequence of the antibody or fragment thereof, or a cell containing the polynucleotide. The reagent or drug according to the present invention preferably further contains a pharmaceutically acceptable excipient or carrier and/or additives.

Examples of excipients or carriers include starch, lactose, crystalline cellulose, sorbitol, calcium hydrogen phosphate, water, ethanol, (poly)ethylene glycol, (poly)propylene glycol, glycerol, and vegetable oil. These can be used singly or in combination of two or more.

Examples of additives include buffers, tonicity agents, thickeners, chelating agents, emulsifiers, colorants, and preservatives. These can be used singly or in a combination of two or more.

The reagent or drug according to the present invention may be an antibody or fragment thereof specifically bindable to the NTD or CTD of SARS-CoV-2 that is dissolved or dispersed in a solvent or lyophilized.

### 7. Kit for detecting the N protein of SARS-CoV-2_

The kit for detecting the N protein of SARS-CoV-2 according to the invention preferably contains the antibody or fragment thereof specifically bindable to the NTD or CTD of SARS-CoV-2. The antibody or fragment thereof may be in the form of a reagent, such as described in section 6 above.

The kit preferably contains the following:
· two or more antibodies or fragments thereof specifically bindable to the NTD of SARS-CoV-2, and the two or more antibodies or fragments thereof each recognize a different epitope of the NTD, and/or
· two or more antibodies or fragments thereof specifically bindable to the CTD of SARS-CoV-2, and the two or more antibodies or fragments thereof each recognize a different epitope of the CTD.

The two or more antibodies or fragments thereof specifically bindable to the NTD preferably differ from each other in terms of the amino acid sequence of at least one CDR of the heavy-chain CDR1 to CDR3 and the light-chain CDR1 to CDR3. In an embodiment, a portion of the two or more antibodies or fragments thereof specifically bindable to the NTD ("the primary anti-NTD antibody or fragment thereof" below) is preferably immobilized, and the remaining portion ("the secondary anti-NTD antibody or fragment thereof" below) is preferably labeled. Examples of the solid phase on which the primary anti-NTD antibody or fragment thereof is immobilized include, but are not limited to, beads, wells, chips, and strips. The method for immobilizing the primary anti-NTD antibody or fragment thereof is not particularly limited. An example is a method of bringing a biotinylated primary anti-NTD antibody or fragment thereof into contact with the surface of a solid phase containing avidin. The labeling substance for labeling the secondary anti-NTD antibody or fragment thereof is not particularly limited, and includes, for example, enzymes such as peroxidase (HRP), and alkaline phosphatase (ALP).

The primary and secondary anti-NTD antibodies or fragments thereof can be selected, for example, from the antibodies or fragments thereof listed in section 2 above, preferably from N-1 in Table 8, more preferably from N-2 in Table 8, still more preferably from N-3 in Table 8, and particularly preferably from combinations of N-4 and N-5, combinations of N-4 and N-6, combinations of N-4 and N-7, combinations of N-5 and N-6, combinations of N-5 and N-7, combinations of N-6 and N-7, combinations of N-6 and N-13, combinations of N-10 and N-12, combinations of N-5 and N-8, and combinations of N-9 and N-10 combinations in Table 8 (wherein the amino acid sequences of the heavy-chain CDR1 to CDR3 and the light-chain CDR1 to CDR3 of N-4 to N-10, N-12, and N-13 may be amino acid sequences having at least 90% identity to these amino acid sequences, or amino acid sequences formed of these amino acid sequences with 1 to 3 amino acid mutations).

The two or more antibodies or fragments thereof specifically bindable to the CTD preferably differ from each other in terms of the amino acid sequence of at least one CDR of the heavy-chain CDR1 to CDR3 and the light-chain CDR1 to CDR3. In an embodiment, a portion of the two or more antibodies or fragments thereof specifically bindable to the CTD ("the primary anti-CTD antibody or fragment thereof" below) is preferably immobilized, and the remaining portion ("the secondary anti-CTD antibody or fragment thereof" below) is preferably labeled. Examples of the solid phase on which the primary anti-CTD antibody or fragment thereof is immobilized include, but are not limited to, beads, wells, chips, and strips. The method for immobilizing the primary anti-CTD antibody or fragment thereof is not particularly limited. An example is a method of bringing a biotinylated primary anti-CTD antibody or fragment thereof into contact with the surface of a solid phase containing avidin. The labeling substance for labeling the secondary anti-CTD antibody or fragment thereof is not limited, and includes, for example, enzymes such as peroxidase (HRP), and alkaline phosphatase (ALP).

The primary and secondary anti-CTD antibodies or fragments thereof can be selected, for example, from the antibodies or fragments thereof listed in section 3 above, preferably from C-1 in Table 15, more preferably from C-2 in Table 15, still more preferably from C-3 in Table 15, and particularly preferably from combinations of C-4 and C-5, combinations of C-4 and C-6, combinations of C-4 and C-7, combinations of C-5 and C-6, combinations of C-5 and C-7, combinations of C-6 and C-7, combinations of C-4 and C-12, combinations of C-9 and C-11, and combinations of C-7 and C-16 in Table 15 (wherein the amino acid sequences of the heavy-chain CDR1 to CDR3 and the light-chain CDR1 to CDR3 of C-4 to C-7, C-9, C-11, C-12, and C-16 may be amino acid sequences having at least 90% identity to these amino acid sequences, or amino acid sequences formed of these amino acid sequences with 1 to 3 amino acid mutations).

The kit is preferably a kit for detecting the N protein of SARS-CoV-2 in a test sample. The test sample may be a biological or non-biological sample. The test sample includes not only the samples as collected but also those samples that have undergone pretreatment, such as removal of foreign substances.

Examples of biological samples include, but are not limited to, blood, serum, plasma, bone marrow fluid, lymph fluid, tears, nasal discharge, nasal lavage fluid, nasal swab fluid, saliva, gargle fluid, sputum, throat swab fluid, sweat, tracheal aspirate, bronchus lavage fluid, pleural effusion, ascites fluid, amniotic fluid, intestinal lavage fluid, urine, feces, cell extracts, tissue extracts, and organ extracts.

Examples of non-biological samples include, but are not limited to, samples taken from environmental surfaces such as faucets, handles, handrails, suspension straps, switches, walls, floors, desks, chairs, and toilet seats.

Examples of the method for detecting the N protein of SARS-CoV-2 by using the kit include, but are not limited to, immunochromatography, enzyme immunoassay, chemiluminescent immunoassay, chemiluminescent enzyme immunoassay, radioimmunoassay, electrochemiluminescent immunoassay, immunonephelometry, and latex agglutination.

The kit may further contain other substances (e.g., buffers, enzyme solutions, solutions for dilution, and secondary antibodies), instruments for use, an instruction manual, etc., for example, according to the preparation method for the test sample, or the detection method for the N protein of SARS-CoV-2.

### 8. Method for detecting the N protein of SARS-CoV-2

The method for detecting the N protein of SARS-CoV-2 preferably includes the following steps (1) to (3):
(1) immobilizing the primary antibody or fragment thereof specifically bindable to the NTD or CTD onto a solid phase,
(2) applying the secondary antibody or fragment thereof that is specifically bindable to the N protein of SARS-CoV-2, and that is specifically bindable to the amino acid sequence of SEQ ID NO: 1 or 2 to the solid phase (wherein the secondary antibody or fragment thereof recognizes a different epitope in the amino acid sequence to which the primary antibody or fragment thereof is specifically bindable, and the secondary antibody or fragment thereof is labeled with a labeling substance), and
(3) detecting a label derived from the labeling substance of the secondary antibody or fragment thereof bound to the solid phase via the nucleocapsid protein.

The primary and secondary antibodies or fragments thereof are preferably the primary and secondary anti-NTD antibodies or fragments thereof and/or the primary and secondary anti-CTD antibodies or fragments thereof described in section 7 above.

The solid phase and the labeling substance can be selected, for example, from those described in section 7 above.

### Examples

### Obtainment of antibody-producing cells by animal immunization

195 µg of SARS-CoV-2 N protein suspended in PBS was mixed with an adjuvant, TiterMAX Gold (Funakoshi Co., Ltd.), and the mixture was administered intramuscularly into the left and right hind limbs of guinea pigs as an initial immunization. Fourteen days after the initial immunization, 100 µg of SARS-CoV-2 N protein suspended in PBS was mixed with TiterMAX Gold, booster immunization was intramuscularly performed in the right and left hind limbs of guinea pigs. Thirty-one days after the booster immunization, booster immunization was performed again by intramuscularly administering 100 µg of SARS-CoV-2 N protein suspended in PBS to the right and left hind limbs of guinea pigs. Eight days after the second booster immunization, two guinea pigs were subjected to total blood sampling and euthanasia under anesthesia. Thereafter, lumbar lymph nodes were removed.

### Sorting of plasma cells producing anti-SARS-Cov2 N protein antibody

Sorting was performed according to the method of US patent application publication No. 2013-0029325, i.e., Fluorescent Probe for Plasma Cell Identification and Isolation, and Plasma Cell Identification or Isolation Method Using the Probe (ERIAA method).

Cells obtained from extracted lumbar lymph nodes were immobilized in a formaldehyde PBS solution on ice for 10 minutes. Immobilized cells were stained with Dylight488-labeled SARS-CoV-2 N protein, Dylight 550-labeled SARS-CoV N protein, Dylight 650-labeled anti-guinea pig IgG antibody, and DAPI (4',6-diamidino-2-phenylindole); and plasma cells showing strong positivity for SARS-CoV-2 N protein, showing negativity for SARS-CoV N protein, and showing strong positivity for anti-guinea pig IgG were sorted by flow cytometry.

### Synthesis of antibody variable region gene fragment

For cells obtained by sorting, mRNA extraction, cDNA synthesis, and amplification of antibody variable region gene fragments were performed according to the method of Kurosawa et al. (MAGrahd method) (Rapid production of antigen-specific monoclonal antibodies from a variety of animals, BMC Biology 2012, 10:80).

### Production of antibody gene expression unit

From the amplified antibody variable region gene fragments, antibody gene expression units were produced according to the method of US patent application publication No. 2013-0023009, i.e., Specific Method for Preparing Joined DNA Fragments Including Sequences Derived from Target Genes (TS-jPCR method).

### Transient expression of antibody in 293FT cells

After the prepared antibody gene expression unit, FuGene HD Transfection Reagent (trademark), and Opti-MEM (trademark) were mixed at a liquid volume ratio of 1:1:50 and allowed to stand at ambient temperature for 20 minutes, the mixture was added to 293FT cells suspended in 10% (w/v) FBS/IMDM medium to introduce the antibody gene expression unit. The 293FT cells in which the antibody gene expression unit had been introduced were seeded in a 24-well plate and incubated at 37°C in an 8% (v/v) CO₂ environment for 2 days, after which a culture supernatant was collected. Antibodies released in the culture supernatant were used for subsequent screening.

### Screening of antibody by ELISA

Screening was performed by evaluating the reaction intensity of each antibody with SARS-CoV-2 N protein by ELISA. In ELISA, immobilization was performed on the bottom of a 96-well plate so that SARS-CoV-2 N protein was at 15 ng/well, and 100 µL of the culture supernatant of 293FT cells in which the antibody was transiently expressed was added to perform an antigen-antibody reaction for 2 hours at room temperature. Guinea pig antibodies bonded to antigens were detected using a goat anti-guinea pig antibody conjugated with horseradish peroxidase, and absorbance was measured at 450 nm using a microplate reader.

### Reactivity evaluation screening of antibody by immunostaining

To confirm whether the obtained antibodies recognize the N-terminal domain (NTD) or the C-terminal domain (CTD) of SARS-CoV-2 N protein, a DNA fragment encoding NTD and a DNA fragment encoding CTD were transiently expressed in 293FT cells. By adding obtained antibodies to perform immunostaining, the reactivity of antibodies with the DNA fragment encoding NTD and with the DNA fragment encoding CTD was evaluated.

Table 16 shows the amino acid sequences of the NTD and CTD.

**Table 16**

| | |
|---|---|
| N-Terminal Domain (NTD) | MSDNGPQNQRNAPRITFGGPSDSTGSNQNGERSGARSKQRRPQGLPNNTASWFTALTQH GKEDLKFPRGQGVPINTNSSPDDQIGYYRRATRRIRGGDGKMKDLSPRWYFYYLGTGPE AGLPYGANKDGIIWVATEGALNTPKDHIGTRNPANNAAIVLQLPQGTTLPKGFY  (SEQ ID: No. 1) |
| C-Terminal Domain (CTD) | TKKSAAEASKKPRQKRTATKAYNVTQAFGRRGPEQTQGNFGDQELIRQGTDYKHWPQIA QFAPSASAFFGMSRIGMEVTPSGTWLTYTAAIKLDDKDPNFKDQVILLNKHIDAYKTFP PTEPKKDKKKKADETQALPQRQKKQQTVTLLPAADLDDFSKQLQQSMSSADSTQA (SEQ ID: No.2) |

Using a plasmid containing an ORF domain of SARS-CoV-2 N protein as a template, a DNA fragment encoding NTD and a DNA fragment encoding CTD were produced by PCR. The prepared DNA fragment (50 ng/uL), FuGene HD Transfection Reagent (trademark) (produced by Promega Co., Ltd.), and Opti-MEM (trademark) (Thermo Fisher Scientific Inc.) were mixed at a liquid volume ratio of 1:1:50. The mixture was allowed to stand at ambient temperature for 20 minutes and added to 293FT cells that had been suspended in 10% (w/v) FBS/IMDM medium.

The 293FT cells to which the DNA fragments had been introduced were seeded in a collagen-coated 96-well plate, followed by incubation at 37°C under an 8% (v/v) CO₂ environment for 2 days. Thereafter, the medium was removed, followed by washing with PBS. A 3% (w/v) formaldehyde PBS solution was added in an amount of 100 µL/well and allowed to stand at ambient temperature for 20 minutes to immobilize the cells. Further, a 0.1% (w/v) Triton PBS solution was added in an amount of 200 µL/well and allowed to stand at room temperature for 5 minutes to perform cell membrane permeabilization. After the solution in the wells was removed, 100 µL of culture supernatant of 293FT cells in which the antibody was transiently expressed were added, and allowed to stand at room temperature for 3 hours for reaction. After the culture supernatant was removed, and washing with PBS was performed, a Dylight594-labeled anti-guinea pig IgG antibody was added, and the mixture was allowed to stand at room temperature for 1 hour under shielded light for reaction. After further washing with PBS, the nuclei were stained with PBS containing DAPI.

By observing the reactivity of immobilized SARS-CoV-2 N protein (full length) and the antibody, and the reactivity of the antibody and cells expressing DNA fragments each encoding NTD or CTD using the method described above, it was determined whether each antibody had NTD reactivity or CTD reactivity.

### Transient expression of antibody in Expi293 cells and purification

For antibodies having high reactivity as a result of evaluation by ELISA, antibodies were produced by transfection of the produced gene expression unit into Expi293 cells. Expi293 Expression Medium (GIBCO) and Expi Fectamine 293 Transfection Kit (GIBCO) were used for the transfection. After 50 µg of the produced gene expression unit of each antibody, 3 mL of OptiMEM I, and 0.16 mL of Expi Fectamine 293 were mixed per E250 culture flask, and allowed to stand for 10 min, the resultant was added to Expi293 cells, thus performing gene introduction. After culture for one day, Expi Fectamine 293 Transfection Enhancer was feed-added, followed by culturing for 4 days. Thereafter, the supernatant was collected and purified in a protein A column to obtain antibodies.

Of antibodies obtained by screening 260 clones of the obtained antibodies, Tables 17 and 18 show the amino acid sequences (based on the IMGT method) of heavy-chain CDR 1 to 3 and light-chain CDR 1 to 3 of 16 clones (N1 to N16) of the antibodies specifically conjugated to NTD and 27 clones (C1 to C27) of the antibodies specifically conjugated to CTD.

**Table 17**

| | Heavy chain (H chain) | | | Light chain (L chain) | | |
|---|---|---|---|---|---|---|
| Clone No. | CDR1 | CDR2 | CDR3 | CDR1 | CDR2 | CDR3 |
| N1 | GFTFDDYG (A-1-2) | ISYSGGRT (B-1-2) | ARDGGSYYSPINFQF (C-1-2) | SEHRSYY (D-1-2) | LKSDGSH (E-1-2) | GVSYSGGHNI (F-1-2) |
| N2 | GFTFSDFW (A-1-3) | INYDGDRT (B-1-3) | ATNYNFQY (C-2-2) | SDYSHYS (D-1-3) | VGTSDIVG (E-2) | GADYSGASSYV (F-2-1) |
| N3 | GFTFNNYW (A-1-4) | INGDSTKI (B-1-4) | ATSGPMDV (C-2-3) | SQGINKW (D-1-4) | KAN (E-3-1) | QYDSTPPLT (F-5) |
| N4 | GFTFDDFG (A-1-5) | IDYSGGTT (B-1-5) | SRDGGSYHSPISCQY (C-1-3) | SGHSSYY (D-1-5) | VKSDGSH (E-1-3) | GVSFSGGHGV (F-1-3) |
| N5 | GFSITTNGYT (A-2-2) | IWYDADT (B-2-2) | ARSIIAEIPTIFTFPPFDV (C-3-1) | NIGGKT (D-3) | SHN (E-3-2) | QVWDSYTYV (F-6) |
| N6 | GFSLTSYS (A-1-8) | IWSGGRI (B-2-3) | ARLWSGYALDI (C-4) | SEYKHYN (D-1-1) | VKSDGSL (E-1-5) | GANHNIGESYGYV (F-3-1) |
| N7 | GFTFDDYG (A-1-2) | ISYSGGST (B-1-6) | ARDGGSYSSPINFQY (C-1-4) | SEHSSYY (D-1-6) | LKSDGSH (E-1-2) | GVSYSGGHNV (F-1-4) |
| N8 | GFTFDDYG (A-1-2) | ISPSGGST (B-1-10) | ARDGGSVTSPIYFQV (C-1-6) | SEHSSYY (D-1-6) | LNSYGSH (E-1-6) | GVSVSGGHNL (F-1-5) |
| N9 | GFTFDDYG (A-1-2) | ISYSGGNT (B-1-11) | ARDGGSVKSPIYFQY (C-1-7) | SEHSSYY (D-1-6) | LNSKGSH (E-1-7) | GVSLKGGHNL (F-1-6) |
| N10 | GFTFSDYT (A-1-9) | IWYDGTKM (B-1-12) | AKYYDYDYTGFDV (C-5-1) | SQSLFYSGKNKDL (D-2-1) | WAS (E-3-3) | QFINGPLT (F-7) |
| N11 | GFTFSDYW (A-1-7) | IHPDGGST (B-1-8) | VSPSGAGLDV (C-7-1) | SEHSSYY (D-1-6) | LKSDGSH (E-1-2) | GVGYNNKYV (F-4-2) |
| N12 | GFTFSDYW (A-1-7) | ISPDGGGT (B-1-9) | SPSAVGFDV (C-8-1) | SDHSSYY (D-1-7) | LKSDGSH (E-1-2) | GVGYSGGYV (F-4-3) |
| N13 | GFTFSNYY (A-1-6) | ISGDSNYI (B-1-7) | VSPLLVVHGPFFQY (C-6-1) | SEHSSYY (D-1-6) | IESDGSH (E-1-4) | GVSYSGGYV (F-4-4) |
| N14 | GFTFSNYY (A-1-6) | ISGDTNYI (B-1-13) | VSPLLVYHGPFFQY (C-6-2) | SDHSSYY (D-1-7) | IESDGSH (E-1-4) | GVKYSGGW (F-4-3) |
| N15 | GFTFSNYY (A-1-6) | ISGDTNYI (B-1-13) | VKPLLVLHGPFFQS (C-6-3) | SDHSSYY (D-1-7) | IESKGSH (E-1-8) | GVSYSGGYY (F-4-6) |
| N16 | GFTFDDYG (A-1-2) | ISYSGGST (B-1-6) | SRDGGSYSSPINFQY (C-1-5) | SEHSSYY (D-1-6) | VKSDGSH (E-1-3) | GVSYSGGHNV (F-1-4) |

**Table 18**

| | Heavy chain (H chain) | | | Light chain (L chain) | | |
|---|---|---|---|---|---|---|
| Clone No. | CDR1 | CDR2 | CDR3 | CDR1 | CDR2 | CDR3 |
| C1 | GFIFSNYF (G-1-2) | ISGDSTYI (H-1-2) | TINWGDV (I-1-2) | KLSNQY (J-1-2) | KDS (K-1-2) | LSRQSDDAASV (L-1-2) |
| C2 | GFTFNTYT (G-1-3) | IGNSGSKT (H-1-3) | ARTSRIVADLVTMGYALDF (I-2) | NIGGKA (J-3) | NGN (K-1-3) | QVYDSSSFV (L-5) |
| C3 | GFTFSNYY (G-1-4) | ISGDGSNI (H-1-4) | STLGVGV (I-1-3) | KLSERY (J-1-3) | NDS (K-1-4) | LSSESDDAARV (L-1-3) |
| C4 | GLSLTNYT (G-1-5) | MWSDGDT (H-2) | ATSEY (I-5) | SESLLHSDGNTY (J-2-2) | KVS (K-1-5) | WQGTDFPR (L-6) |
| C5 | GFFFSNSW (G-1-10) | IKGDSSIT (H-1-8) | GSGWV (I-9) | SQSLLHGDGKTY (J-2-8) | LIS (K-1-6) | LQATHDPYT (L-3-2) |
| C6 | GFSFSNSW (G-1-6) | IKGDSSII (H-1-6) | GSGWV (I-9) | SQSLLHSDGKTY (J-2-3) | LIS (K-1-6) | LQATHDPYT (L-3-2) |
| C7 | GFSFSNSW (G-1-6) | IKGDSSII (H-1-6) | GSGWY (I-10) | SQSLLHSDGNTY (J-2-9) | LIS (K-1-6) | LQATHNPVS (L-3-4) |
| C8 | GFSFSNSW (G-1-6) | IKGDSSII (H-1-6) | GSGWY (I-10) | SQSLVHSDGKTY (J-2-4) | LIS (K-1-6) | LQATHDPIT (L-3-5) |
| C9 | GFSFSNSW (G-1-6) | IKGDSSII (H-1-6) | GSGWV (I-9) | SQSLLHSDGKTY (J-2-4) | LIS (K-1-6) | LQATHDPYT (L-3-3) |
| C10 | GFSFSNSW (G-1-6) | IKGDSSTI (H-1-14) | GSGWV (I-9) | SQSLLHSDGKTY (J-2-4) | LIS (K-1-6) | LQATHDPYT (L-3-3) |
| C11 | GFSFSNSW (G-1-6) | IKGDSSII (H-1-6) | GSGWV (I-9) | SQSLLHSDGKTY (J-2-4) | LIS (K-1-6) | LQATHDPYT (L-3-3) |
| C12 | GFTFDDYG (G-1-11) | IGYSGASI (H-1-16) | AREGVITVGTWGDV (I-4) | ALPKKY (J-1-5) | KDS (K-1-2) | LSADSSGTYRV (L-1-5) |
| C13 | GFTFRNHW (G-1-8) | INSDGSNT (H-1-9) | TTATDV (I-6) | SQSLLDSDGYTY (J-2-5) | EVS (K-1-7) | VQTTHDPYT (L-3-3) |
| C14 | GFTFSNHW (G-1-9) | INTDGSST (H-1-10) | TPATDV (I-7) | SQSLLYSDGYTY (J-2-6) | EVS (K-1-7) | VQTTHDPYT (L-3-3) |
| C15 | GFTFSNHW (G-1-9) | INSDGSST (H-1-15) | TTATDV (I-6) | SQSLLYSDGYTY (J-2-7) | EVS (K-1-7) | QTTNDPYT (L-4-1) |
| C16 | GFTFSNYW (G-1-7) | INPDDGSP (H-1-7) | ARDVVKTGTTGLPFDL (I-3) | KLSERY (J-1-3) | NDS (K-1-4) | LSGESDDAWV (L-2-1) |
| C17 | GFTFSNYW (G-1-7) | INPDDGSP (H-1-7) | ARDVVKTGTTGLPFDL (I-3) | KLSERY (J-1-3) | NDS (K-1-4) | LSGESDDAWV (L-2-1) |
| C18 | GFTFSNYW (G-1-7) | IKGDSRTI (H-1-11) | STTGFDV (I-1-5) | SQSLLYSDGYTF (J-2-7) | EVS (K-1-7) | VQTSHVPLT (L-3-6) |
| C19 | GFTFSNYW (G-1-7) | IKGDSSTI (H-1-14) | STTGFDV (I-1-5) | SQSLLYSDGYTF (J-2-7) | EVS (K-1-7) | VQTTHVPLT (L-3-7) |
| C20 | GFTFSNYY (G-1-4) | ISGDSSNI (H-1-5) | SPMTIHGLDT (I-8) | KLSKEY (J-1-4) | KDS (K-1-2) | LSRESNDAAWV (L-1-4) |
| C21 | GFTFSNYY (G-1-4) | ISGTSSNI (H-1-17) | TTLGVGV (I-1-4) | KLSERY (J-1-3) | NDS (K-1-4) | LSSESDDAARV (L-1-3) |
| C22 | GFTFSNYY (G-1-4) | ISGTSSNI (H-1-17) | TTLGVGV (I-1-4) | KLSERY (J-1-3) | NDS (K-1-4) | LSSESDDAARV (L-1-3) |
| C23 | GFTFSNYY (G-1-4) | ISGASSNI (H-1-12) | TTLGVGV (I-1-4) | KLSERY (J-1-3) | NDS (K-1-4) | LSSESDDAARV (L-1-3) |
| C24 | GFTFSNYY (G-1-4) | ISGDSTYI (H-1-2) | TIDWGNV (I-1-8) | KLSTQY (J-1-6) | KDS (K-1-2) | LSRQSDTAASL (L-1-6) |
| C25 | GFIFSNYF (G-1-2) | ISGDGSNI (H-1-4) | TITWGDV (I-1-7) | KLSDQY (J-1-7) | KDS (K-1-2) | LSRQGDTAASY (L-1-7) |
| C26 | KLSERY (G-2) | NDS (H-3) | LSSESDDAARV (I-11) | KLSERY (J-1-3) | NDS (K-1-4) | LSSESDDAARV (L-1-3) |
| C27 | GFTFSNYY (G-1-4) | ISGDSSNV (H-1-13) | STLGNFL (I-1-6) | KLSERY (J-1-3) | NDD (K-1-1) | LSSESDDAARV (L-1-3) |

The effects of the present invention are explained below using some clones having a reactivity with NTD or CTD; however, the present invention is not limited to these clones. Table 19 shows the results of immunostaining and ELISA for typical clones. The use of other clones can also attain similar activity effects.

**Table 19**

| Clone No. | Immunostaining | | ELISA Absorbance value |
|---|---|---|---|
| | NTD | CTD | |
| N1 | + | - | 0.477 |
| N2 | + | - | 0.184 |
| N3 | + | - | 0.121 |
| N16 | + | - | 0.212 |
| N13 | + | - | 0.860 |
| N11 | + | - | 0.921 |
| N6 | + | - | 0.429 |
| N7 | + | - | 0.672 |
| N12 | + | - | 0.959 |
| N4 | + | - | 0.796 |
| C1 | - | + | 1.985 |
| C2 | - | + | 1.402 |
| C3 | - | + | 0.688 |
| C4 | - | + | 0.737 |
| C9 | - | + | 0.443 |
| C27 | - | + | 1.249 |
| C16 | - | + | 0.331 |
| C14 | - | + | 1.301 |
| C20 | - | + | 1.686 |
| C6 | - | + | 0.443 |
| C18 | - | + | 1.338 |
| C13 | - | + | 1.040 |
| C23 | - | + | 0.777 |

### Sandwich ELISA for NP antigen with progressive degradation

Each biotin-labelled clone (immobilized antibody) was diluted with TBS to 5 pg/mL, and added to a 96-well microplate in an amount of 50 µL/well, followed by coating for 2 hours at room temperature. After blocking with 1% (w/v) BSA-TBS-T and subsequent washing with TBS-T, each 0.25 ug/mL alkaline phosphatase-labeled clone (detection antibody) and a 10 ng/mL antigen solution (1% (w/v) TBS-T dilution) were each added in an amount of 50 µL/well to perform reaction at room temperature for 2 hours. As an antigen solution, in addition to Sample 1 of the full-length SARS-CoV-2 N protein, Sample 2, in which the total amount of the SARS-CoV-2 N protein was replaced with the equivalent mol amount of NTD and the equivalent mol amount of CTD, as a mimic of the sample in which the SARS-CoV-2 N protein was degraded, was used. After washing with TBS-T, pNPP was added in an amount of 100 µL/well as a chromogenic substrate, and the reaction was performed for 15 minutes at room temperature, after which the absorbance at 405 nm was measured using a microplate reader. Table 20 shows the results. The percentage (%) in Table 20 represents the relative absorbance value to the absorbance measurement value of Sample 1.

**Table 20**

| Measurement No. | Immobilized antibody | Detection antibody | Sample 1 (0% of antigen is degraded.) | Sample 2 (100% of antigen is degraded. ) |
|---|---|---|---|---|
| 1 | N1 | N2 | 100% | 38% |
| 2 | N3 | N16 | 100% | 22% |
| 3 | N13 | N11 | 100% | 77% |
| 4 | N2 | N6 | 100% | 17% |
| 5 | N11 | N7 | 100% | 19% |
| 6 | C1 | C2 | 100% | 45% |
| 7 | C3 | C4 | 100% | 32% |
| 8 | C1 | C16 | 100% | 90% |
| 9 | C9 | C14 | 100% | 25% |
| 10 | C27 | C4 | 100% | 47% |
| 11 | N1 | C2 | 100% | 6% |
| 12 | N1 | C4 | 100% | 5% |
| 13 | N3 | C2 | 100% | 1% |
| 14 | N3 | C4 | 100% | 5% |
| 15 | N13 | C16 | 100% | 0% |
| 16 | N2 | C14 | 100% | 0% |
| 17 | N11 | C4 | 100% | 0% |
| 18 | C1 | N2 | 100% | 3% |
| 19 | C1 | N16 | 100% | 3% |
| 20 | C3 | N2 | 100% | 2% |
| 21 | C3 | N16 | 100% | 3% |
| 22 | C1 | N11 | 100% | 1% |
| 23 | C9 | N6 | 100% | 5% |
| 24 | C27 | N7 | 100% | 0% |

When two types of antibodies recognizing NTD were combined, and when two types of antibodies recognizing CTD were combined, it was assumed that the antigen fragment had a conformational change in Sample 2, which was a mimic of the sample in which the SARS-CoV-2 N protein was degraded, and that the reactivity of the antigen fragment was reduced, and a reduction in the absorbance measurement value was observed; however, the degree of reduction was low. In contrast, when the NTD recognition antibody was combined with the CTD recognition antibody, a significant reduction in the absorbance measurement value was confirmed in Samples 2 to 4.

### Sandwich ELISA using oligoclonal antibody

An immobilized antibody mixed with multiple biotin-labeled clones was diluted to 5 pg/mL with TBS, and added to a 96-well microplate in an amount of 50 pL/well, followed by coating for 2 hours at room temperature. After blocking with 1% (w/v) BSA-TBS-T and subsequent washing with TBS-T, a 0.25 ug/mL detection antibody mixed with multiple clones labeled with alkaline phosphatase, and a 10 ng/mL antigen solution (1% (w/v) TBS-T dilution) were each added in an amount of 50 µL/well to perform reaction at room temperature for 2 hours. When two types of immobilized antibodies were used, the addition amount of each immobilized antibody was halved so that the total amount was 50 µL/well. The same applied to the case in which two types of detection antibodies were used. As an antigen solution, in addition to Sample 1 of the full-length SARS-CoV-2 N protein, Sample 2, in which the total amount of the SARS-CoV-2 N protein was replaced with the equivalent mol amount of NTD and the equivalent mol amount of CTD, as a mimic of the sample in which the SARS-CoV-2 N protein was degraded, was used. After washing with TBS-T, pNPP was added in an amount of 100 µL/well as a chromogenic substrate, and the reaction was performed for 15 minutes at room temperature, after which the absorbance was measured at 405 nm using a microplate reader. Table 21 shows the results.

**Table 21**

| Immobilized antibody 1 | Immobilized antibody 2 | Detection antibody 1 | Detection antibody 2 | Sample 1 (0% of antigen is degraded. ) | Sample 2 (100% of antigen is degraded. ) |
|---|---|---|---|---|---|
| N1 | - | N2 | - | 0.653 | 0.245 |
| C1 | - | C2 | - | 0.292 | 0.132 |
| N1 | C1 | N2 | C2 | 0.886 | 0.320 |

As compared to the use of a single antibody as an immobilized antibody and a detection antibody, it was confirmed that use of the combination of multiple antibodies improved detection sensitivity.

### Epitope mapping of antibody

Epitope mapping was determined using the service of Conformational Epitope mappings produced by PEPperPRINT GmbH. In an amino acid region at positions 200 to 419 from the N-terminus of N protein of SARS-CoV-2, peptides consisting of 7, 10, and 13 amino acids were synthesized on peptide arrays so that they were shifted by 1 amino acid to overlap by 6, 9, and 12 amino acids. Based on the reactivity of antibodies, i.e., detection signals, it was identified which peptide was the epitope of antibody binding.

### Results

By epitope mapping of monoclonal antibodies, of the clones shown in Table 18, it was confirmed that three clone epitopes were in a region of SEQ ID: No.150:LLPAADLDDFSK (at positions 394 to 405 from the N-terminus of the N protein of SARS-CoV-2), a region of SEQ ID: No. 151:KKADETQAL (at positions 374 to 382 from the N-terminus of the N protein of SARS-CoV-2), and a region of SEQ ID: No. 152:SAAEASKK (at positions 250 to 257 from the N-terminus of the N protein of SARS-CoV-2).

## Claims

1. An antibody or fragment thereof specifically bindable to the amino acid sequence of SEQ ID NO: 1 or 2.

2. An antibody or fragment thereof, comprising
a heavy-chain CDR1 containing the amino acid sequence represented by the following formula (A-1) or (A-2):
GFX^{A31}X^{A41}X^{A51}X^{A61}X^{A71}X^{A81} (A-1)
wherein
X^{A31} represents T or S,
X^{A41} represents F, L, or I,
X^{A51} represents D, S, N, or T,
X^{A61} represents D, N, S, I, or T,
X^{A71} represents Y, F, or N,
X^{A81} represents G, W, Y, S, or T, and
GFX^{A33}X^{A43}X^{A53}X^{A63}X^{A73}X^{A83}X^{A93}XA¹⁰³ (A-2)
wherein
X^{A33} represents T or S,
X^{A43} represents F, L, or I,
X^{A53} represents D, S, N, or T,
X^{A63} represents D, N, S, or T,
X^{A73} represents N or S,
X^{A83} represents G or Y,
X^{A93} represents Y, F, or N,
X^{A103} represents T, G, W, or Y;
a heavy-chain CDR2 containing the amino acid sequence represented by the following formula (B-1) or (B-2):
IX^{B21}X^{B31}X^{B41}X^{B51}X^{B61}X^{B71}X^{B81} (B-1)
wherein
X^{B21} represents S, N, D, W, or H,
X^{B31} represents Y, G, P, or S,
X^{B41} represents S, D, or G,
X^{B51} represents G, S, T, or A,
X^{B61} represents G, D, T, N, or R,
X^{B71} represents R, K, T, S, N, G, Y, or D,
X^{B81} represents T, I, or M, and
IX^{B23}X^{B33}X^{B43}X^{B53}X^{B63}X^{B73} (B-2)
wherein
X^{B23} represents S, N, D, W, or H,
X^{B33} represents Y, G, P, or S,
X^{B43} represents S, D, or G,
X^{B53} represents G, S, T, or A,
X^{B63} represents R, K, T, S, N, G, Y, or D,
X^{B73} represents T, I, or M;
a heavy-chain CDR3 containing the amino acid sequence represented by any one of the following formulas (C-1) to (C-8) :
X^{C11}X^{C21}X^{C31}X^{C41}X^{C51}X^{C61}X^{C71}X^{C81}X^{C91}X^{C101}X^{C111}X^{C121}X^{C131}X^{C141}X^{C151} (C-1)
wherein
X^{C11} represents A or S,
X^{C21} represents R, T, or K,
X^{C31} represents D, N, S, or Y,
X^{C41} represents G, S, or Y,
X^{C51} represents G, I, or D,
X^{C61} represents S or I,
X^{C71} represents Y, V, or A,
X^{C81} represents Y, H, S, T, K, or E,
X^{C91} represents S, I, or D,
X^{C101} represents P or Y,
X^{C111} represents I, Y, G, P, T, A, or V,
X^{C121} represents N, S, Y, P, G, or F,
X^{C131} represents F, C, M, or L,
X^{C141} represents Q or D,
X^{C151} represents F, Y, V, or S,
X^{C13}X^{C23}X^{C33}X^{C43}X^{C53}X^{C63}X^{C73}X^{C83} (C-2)
wherein
X^{C13} represents A or S,
X^{C23} represents R, T, or K,
X^{C33} represents D, N, S, or Y,
X^{C43} represents I, Y, G, P, T, A, or V,
X^{C53} represents N, S, Y, P, G, or F,
X^{C63} represents F, C, M, or L,
X^{C73} represents Q or D,
X^{C83} represents F, Y, V, or S,
X^{C15}X^{C25}X^{C35}X^{C45}X^{C55}X^{C65}X^{C75}X^{C85}X^{C95}TIFTFX^{C155}X^{C165}X^{C175}X^{C185}X^{C195} (C-3)
wherein
X^{C15} represents A or S,
X^{C25} represents R, T, or K,
X^{C35} represents G, S, or Y,
X^{C45} represents G, I, or D,
X^{C55} represents S or I,
X^{C65} represents Y, V, or A,
X^{C75} represents Y, H, S, T, K, or E,
X^{C85} represents S, I, or D,
X^{C95} represents P or Y,
X^{C155} represents I, Y, G, P, T, A, or V,
X^{C165} represents N, S, Y, P, G, or F,
X^{C175} represents F, C, M, or L,
X^{C185} represents Q or D,
X^{C195} represents F, Y, V, or S,
ARLWSGYALDI (C-4),
X^{C16}X^{C26}X^{C36}X^{C46}X^{C56}X^{C66}X^{C76}X^{C86}X^{C96}X^{C106}X^{C116}X^{C126}XC¹³⁶ (C-5)
wherein
X^{C16} represents A or S,
X^{C26} represents R, T, or K,
X^{C36} represents D, N, S, or Y,
X^{C46} represents G, S, or Y,
X^{C56} represents G, I, or D,
X^{C66} represents Y, H, S, T, K, or E,
X^{C76} represents S, I, or D,
X^{C86} represents P or Y,
X^{C96} represents I, Y, G, P, T, A, or V,
X^{C106} represents N, S, Y, P, G, or F,
X^{C116} represents F, C, M, or L,
X^{C126} represents Q or D,
X^{C136} represents F, Y, V, or S,
VX^{C27}PLLVX^{C77}HGX^{C107}X^{C117}X^{C127}X^{C137}X^{C147} (C-6)
wherein
X^{C27} represents S or K,
X^{C77} represents V, Y, or L,
X^{C107} represents I, Y, G, P, T, A, or V,
X^{C117} represents N, S, Y, P, G, or F,
X^{C127} represents F, C, M, or L,
X^{C137} represents Q or D,
X^{C147} represents F, Y, V, or S,
VSPSX^{C58}X^{C68}X^{C78}X^{C88}X^{C98}X^{C108} (C-7)
wherein
X^{C58} represents G or A,
X^{C68} represents I, Y, G, P, T, A, or V,
X^{C78} represents N, S, Y, P, G, or F,
X^{C88} represents F, C, M, or L,
X^{C98} represents Q or D,
X^{C108} represents F, Y, V, or S, and
SPSX^{C49}X^{C59}X^{C69}X^{C79}X^{C89}X^{C99} (C-8)
wherein
X^{C49} represents G or A,
X^{C59} represents I, Y, G, P, T, A, or V,
X^{C69} represents N, S, Y, P, G, or F,
X^{C79} represents F, C, M, or L,
X^{C89} represents Q or D,
X^{C99} represents F, Y, V, or S;
a light-chain CDR1 containing the amino acid sequence represented by any one of the following formulas (D-1) to (D-3) :
SX^{D21}X^{D31}X^{D41}X^{D51}X^{D61}X^{D71} (D-1)
wherein
X^{D21} represents E, D, Q, or G,
X^{D31} represents H, Y, G, or S,
X^{D41} represents R, S, I, K, or L,
X^{D51} represents S, H, N, or F,
X^{D61} represents Y or K,
X^{D71} represents Y, S, W, or N,
SX^{D23}X^{D33}X^{D43}X^{D53}X^{D63}X^{D73}GKNKDL (D-2)
wherein
X^{D23} represents E, D, Q, or G,
X^{D33} represents H, Y, G, or S,
X^{D43} represents R, S, I, K, or L,
X^{D53} represents S, H, N, or F,
X^{D63} represents Y or K,
X^{D73} represents Y, S, W, or N, and
NIGGKT (D-3);
a light-chain CDR2 containing the amino acid sequence represented by any one of the following formulas (E-1) to (E-3) :
X^{E11}X^{E21}SX^{E41}GSX^{E71} (E-1)
wherein
X^{E11} represents L, V, or I,
X^{E21} represents K, N, or E,
X^{E41} represents D, Y, or K,
X^{E71} represents H or L,
VGTSDIVG (E-2), and
X^{E12}X^{E22}X^{E32} (E-3)
wherein
X^{E12} represents K, S, or W,
X^{E22} represents A or H,
X^{E32} represents N or S; and
a light-chain CDR3 containing the amino acid sequence represented by any one of the following formulas (F-1) to (F-7):
GX^{F21}X^{F31}X^{F41}X^{F51}X^{F61}X^{F71}X^{F81}X^{F91}X^{F101} (F-1)
wherein
X^{F21} represents V or A,
X^{F31} represents S, D, N, G, or K,
X^{F41} represents Y, F, V, L, or H,
X^{F51} represents S, K, or N,
X^{F61} represents G or N,
X^{F71} represents G, A, E, or K,
X^{F81} represents H, S, Y, or V,
X^{F91} represents N, G, or S,
X^{F101} represents I, V, L, or Y,
GX^{F23}X^{F33}X^{F43}X^{F53}X^{F63}X^{F73}X^{F83}X^{F93}YX^{F113} (F-2)
wherein
X^{F23} represents V or A,
X^{F33} represents S, D, N, or G,
X^{F43} represents Y, F, V, L, or H,
X^{F53} represents S, K, or N,
X^{F63} represents G or N,
X^{F73} represents G, A, E, or K,
X^{F83} represents H, S, or Y,
X^{F93} represents N, G, or S,
X^{F113} represents I, V, L, or Y,
GX^{F24}X^{F34}X^{F44}X^{F54}IX^{F74}X^{F84}X^{F94}YX^{F114}YX^{F134} (F-3)
wherein
X^{F24} represents V or A,
X^{F34} represents S, D, N, or G,
X^{F44} represents Y, F, V, L, or H,
X^{F54} represents S, K, or N,
X^{F74} represents G or N,
X^{F84} represents G, A, E, or K,
X^{F94} represents H, S, or Y,
X^{F114} represents N, G, or S,
X^{F134} represents I, V, L, or Y,
GX^{F25}X^{F35}X^{F45}X^{F55}X^{F65}X^{F75}X^{F85}X^{F95} (F-4)
wherein
X^{F25} represents V or A,
X^{F35} represents S, D, N, G, or K,
X^{F45} represents Y, F, V, L, or H,
X^{F55} represents S, K, or N,
X^{F65} represents G or N,
X^{F75} represents G, A, E, or K,
X^{F85} represents H, S, Y, or V,
X^{F105} represents I, V, L, or Y,
QYDSTPPLT (F-5),
QVWDSYTYV (F-6), and
QFINGPLT (F-7).

3. The antibody or fragment thereof according to claim 2, comprising
a heavy-chain CDR1 containing the amino acid sequence represented by formula (A-1),
a heavy-chain CDR2 containing the amino acid sequence represented by formula (B-1) or (B-2),
a heavy-chain CDR3 containing the amino acid sequence represented by any one of formulas (C-1), (C-2), (C-4), and (C-6) to (C-8),
a light-chain CDR1 containing the amino acid sequence represented by formula (D-1),
a light-chain CDR2 containing the amino acid sequence represented by any one of formulas (E-1) to (E-3), and
a light-chain CDR3 containing the amino acid sequence represented by any one of formulas (F-1) to (F-5).

4. An antibody or fragment thereof, comprising
a heavy-chain CDR1 containing the amino acid sequence represented by the following formula (A-1-1):
GFX^{A32}X^{A42}X^{A52}X^{A62}X^{A72}X^{A82} (A-1-1)
wherein
X^{A32} represents T or S,
X^{A42} represents F or L,
X^{A52} represents D, S, N, or T,
X^{A62} represents D, S, or N,
X^{A72} represents Y or F, and
X^{A82} represents G, W, S, or Y;
a heavy-chain CDR2 containing the amino acid sequence represented by the following formula (B-1-1) or (B-2-1):
IX^{B22}X^{B32}X^{B42}X^{B52}X^{B62}X^{B72}X^{B82} (B-1-1)
wherein
X^{B22} represents S, N, D, or H,
X^{B32} represents Y, G, or P,
X^{B42} represents S or D,
X^{B52} represents G or S,
X^{B62} represents G, D, T, or N,
X^{B72} represents R, K, T, S, G, or Y,
X^{B82} represents T or I, and
IWX^{B34}X^{B44}X^{B54}X^{B64}X^{B74} (B-2-1)
wherein
X^{B34} represents Y or S,
X^{B44} represents D or G,
X^{B54} represents G or A,
X^{B64} represents R or D, and
X^{B74} represents T or I,
a heavy-chain CDR3 containing the amino acid sequence represented by any one of the following formulas (C-1-1), (C-2-1), (C-6-1), (C-7-1), and (C-8-1):
X^{C12}RDGGSYX^{C82}SPIX^{C122}X^{C132}QX^{C152} (C-1-1)
wherein
X^{C12} represents A or S,
X^{C82} represents Y, H, or S,
X^{C122} represents N or S,
X^{C132} represents F or C,
X^{C152} represents F or Y, and
ATX^{C34}X^{C44}X^{C54}X^{C64}X^{C74}X^{C84} (C-2-1)
wherein
X^{C34} represents N or S,
X^{C44} represents Y or G,
X^{C54} represents N or P,
X^{C64} represents F or M,
X^{C74} represents Q or D,
X^{C84} represents Y or V,
VSPLLVVHGPFFQY (C-6-1),
VSPSGAGLDV (C-7-1), and
SPSAVGFDV (C-8-1);
a light-chain CDR1 containing the amino acid sequence represented by any one of the following formulas (D-1-1) to (D-1-7):
SEYKHYN (D-1-1),
SEHRSYY (D-1-2),
SDYSHYS (D-1-3),
SQGINKW (D-1-4),
SGHSSYY (D-1-5),
SEHSSYY (D-1-6), and
SDHSSYY (D-1-7);
a light-chain CDR2 containing the amino acid sequence represented by any one of the following formulas (E-1-1), (E-2), and (E-3-1):
X^{E12}X^{E22}SDGSX^{E72} (E-1-1)
wherein
X^{E12} represents L, V, or I,
X^{E22} represents K, or E,
X^{E72} represents H or L,
VGTSDIVG (E-2), and
KAN (E-3-1); and
a light-chain CDR3 containing the amino acid sequence represented by any one of the following formulas (F-1-1), (F-2-1), (F-3-1), (F-4-1), and (F-5):
GVSX^{F42}SGGHX^{F92}X^{F102} (F-1-1)
wherein
X^{F42} represents Y or F,
X^{F92} represents N or G,
X^{F102} represents I or V,
GADYSGASSYV (F-2-1),
GANHNIGESYGYV (F-3-1),
GVX^{F36}YX^{F56}X^{F66}X^{F76}X^{F86}X^{F96} (F-4-1)
wherein
X^{F36} represents S, G, or K,
X^{F56} represents S or N,
X^{F66} represents G or N,
X^{F76} represents G or K,
X^{F86} represents Y or V,
X^{F96} represents V or Y; and
QYDSTPPLT (F-5).

5. The antibody or fragment thereof according to claim 2, comprising
a heavy-chain CDR1 containing the amino acid sequence of any one of SEQ ID NOs: 3 to 11 or an amino acid sequence formed of any of these amino acid sequences with one to three amino acid mutations,
a heavy-chain CDR2 containing the amino acid sequence of any one of SEQ ID NOs: 12 to 25 or an amino acid sequence formed of any of these amino acid sequences with one to three amino acid mutations,
a heavy-chain CDR3 containing the amino acid sequence of any one of SEQ ID NOs: 26 to 41 or an amino acid sequence formed of any of these amino acid sequences with one to three amino acid mutations,
a light-chain CDR1 containing the amino acid sequence of any one of SEQ ID NOs: 42 to 50 or an amino acid sequence formed of any of these amino acid sequences with one to three amino acid mutations,
a light-chain CDR2 containing the amino acid sequence of any one of SEQ ID NOs: 51 to 58 or an amino acid sequence formed of any of these amino acid sequences with one to three amino acid mutations, or the amino acid sequence represented by KAN, SHN, or WAS, and
a light-chain CDR3 containing the amino acid sequence of any one of SEQ ID NOs: 59 to 73 or an amino acid sequence formed of any of these amino acid sequences with one to three amino acid mutations.

6. The antibody or fragment thereof according to claim 5, wherein the mutations are conservative substitutions.

7. The antibody or fragment thereof according to any one of claims 2 to 6, which is specifically bindable to the amino acid sequence of SEQ ID NO: 1.

8. An antibody or fragment thereof, comprising
a heavy-chain CDR1 containing the amino acid sequence represented by the following formula (G-1) or (G-2):
GX^{G21}X^{G31}X^{G41}X^{G51}X^{G61}X^{G71}X^{G81} (G-1)
wherein
X^{G21} represents F or L,
X^{G31} represents I, T, S, or F,
X^{G41} represents F or L,
X^{G51} represents S, N, T, R, or D,
X^{G61} represents N, T, or D,
X^{G71} represents Y, S, or H,
X^{G81} represents F, T, Y, W, or G,
KLSERY (G-2);
a heavy-chain CDR2 containing the amino acid sequence represented by any one of the following formulas (H-1) to (H-3):
IX^{H21}X^{H31}X^{H41}X^{H51}X^{H61}X^{H71}X^{H81} (H-1)
wherein
X^{H21} represents S, G, K, or N,
X^{H31} represents G, N, S, Y, T, or P,
X^{H41} represents D, S, T, or A,
X^{H51} represents S, G, or D,
X^{H61} represents T, S, A, G, or R,
X^{H71} represents Y, K, N, T, I, or S,
X^{H81} represents I, T, P, or V,
MWSDGDT (H-2), and
NDS (H-3);
a heavy-chain CDR3 containing the amino acid sequence represented by any one of the following formulas (I-1) to (1-11):
X^{I11}X^{I21}X^{I31}X^{I41}X^{I51}X^{I61}X^{I71} (1-1)
wherein
X^{I11} represents S or T,
X^{I21} represents I or T,
X^{I31} represents N, L, D, or T,
X^{I41} represents W or G,
X^{I51} represents G, V, F, or N,
X^{I61} represents D, G, F, or N,
X^{I71} represents V or L,
ARTSRIVADLVTMGYALDF (1-2),
ARDVUKTGTTGLPFDL (1-3),
AREGVITVGTWGDV (I-4),
ATSEY (I-5),
TTATDV (1-6),
TPATDV (1-7),
SPMTIHGLDT (1-8),
GSGWV (1-9),
GSGWY (1-10), and
LSSESDDARV (I-11);
a light-chain CDR1 containing the amino acid sequence represented by any one of the following formulas (J-1) to (J-3):
X^{J11}LX^{J31}X^{J41}X^{J51}Y (J-1)
wherein
X^{J11} represents K or A,
X^{J31} represents S or P,
X^{J41} represents N, E, K, T, or D,
X^{J51} represents Q, R, K, or E,
SX^{J23}SLX^{J53}X^{J63}X^{J73}DGX^{J103}TX^{J123} (J-2),
wherein
X^{J23} represents E or Q,
X^{J53} represents L or V,
X^{J63} represents H, D, or Y,
X^{J73} represents S or G,
X^{J103} represents N, K, or Y,
X^{J123} represents Y or F, and
NIGGKA (J-3),
a light-chain CDR2 containing the amino acid sequence represented by the following formula (K-1):
X^{K11}X^{K21}X^{K31} (K-1)
wherein
X^{K11} represents K, N, L, or E,
X^{K21} represents D, G, V, or I,
X^{K31} represents S, N, or D,
a light-chain CDR3 containing the amino acid sequence represented by any one of the following formulas (L-1) to (L-6):
LSX^{L31}X^{L41}X^{L51}X^{L61}X^{L71}X^{L81}X^{L91}X^{L101}X^{L111} (L-1)
wherein
X^{L31} represents R, S, A, or G,
X^{L41} represents Q, E, or D,
X^{L51} represents S or G,
X^{L61} represents D, S, or N,
X^{L71} represents D, G, or T,
X^{L81} represents A, T, or Y,
X^{L91} represents A, T, or Y,
X^{L101} represents S, R, or W,
X^{L111} represents V, L, or Y,
LSX^{L33}X^{L43}X^{L53}X^{L63}X^{L73}X^{L83}X^{L93}X^{L103} (L-2),
wherein
X^{L33} represents R, S, A, or G,
X^{L43} represents Q, E, or D,
X^{L53} represents S or G,
X^{L63} represents D, S, or N,
X^{L73} represents D, G, or T,
X^{L83} represents A, T, or Y,
X^{L93} represents S, R, or W,
X^{L103} represents V, L, or Y,
X^{L14}QX^{L34}X^{L44}X^{L54}X^{L64}PX^{L84}X^{L94} (L-3),
wherein
X^{L14} represents L or V,
X^{L34} represents A or T,
X^{L44} represents T or S,
X^{L54} represents H or N,
X^{L64} represents D, N, or V,
X^{L84} represents Y, V, I, or L,
X^{L94} represents T or S,
QX^{L27}X^{L37}X^{L47}X^{L57}PX^{L77}X^{L87} (L-4)
wherein
X^{L27} represents A or T,
X^{L37} represents T or S,
X^{L47} represents H or N,
X^{L57} represents D, N, or V,
X^{L77} represents Y, V, I, or L,
X^{L87} represents T or S,
QVYDSSSFV (L-5), and
WQGTDFPR (L-6).

9. The antibody or fragment thereof according to claim 8, comprising
a heavy-chain CDR1 containing the amino acid sequence represented by formula (G-1),
a heavy-chain CDR2 containing the amino acid sequence represented by formula (H-1) or (H-2),
a heavy-chain CDR3 containing the amino acid sequence represented by any one of formulas (I-1) to (1-3) and (1-5) to (1-9),
a light-chain CDR1 containing the amino acid sequence represented by any one of formulas (J-1) to (J-3),
a light-chain CDR2 containing the amino acid sequence represented by formula (K-1), and
a light-chain CDR3 containing the amino acid sequence represented by any one of formulas (L-1) to (L-3), (L-5), and (L-6).

10. An antibody or fragment thereof, comprising
a heavy-chain CDR1 containing the amino acid sequence represented by the following formula (G-1-1):
GX^{G22}X^{G32}X^{G42}X^{G52}X^{G62}X^{G72}X^{G82} (G-1-1)
wherein
X^{G22} represents F or L,
X^{G32} represents I, T, or S,
X^{G42} represents F or L,
X^{G52} represents S, N, T, or R,
X^{G62} represents N or T,
X^{G72} represents Y, S, or H,
X^{G82} represents F, T, Y, or W,
a heavy-chain CDR2 containing the amino acid sequence represented by the following formula (H-1-1) or (H-2):
IX^{H22}X^{H32}X^{H42}X^{H52}X^{H62}X^{H72}X^{H82} (H-1-1)
wherein
X^{H22} represents S, G, K, or N,
X^{H32} represents G, N, S, T, or P,
X^{H42} represents D, S, or A,
X^{H52} represents S, G, or D,
X^{H62} represents T, S, G, or R,
X^{H72} represents Y, K, N, T, I, or S,
X^{H82} represents I, T, P, or V, and
MWSDGDT (H-2),
a heavy-chain CDR3 containing the amino acid sequence represented by any one of the following formulas (I-1-1), (1-2), (1-3), and (1-5) to (1-9):
X^{I12}X^{I22}X^{I32}X^{I42}X^{I52}X^{I62}X^{I72} (I-1-1)
wherein
X^{I12} represents S or T,
X^{I22} represents I or T,
X^{I32} represents N, L, or T,
X^{I42} represents W or G,
X^{I52} represents G, V, F, or N,
X^{I62} represents D, F, or G,
X^{I72} represents V or L,
ARTSRIVADLVTMGYALDF (1-2),
ARDVUKTGTTGLPFDL (1-3),
ATSEY (I-5),
TTATDV (1-6),
TPATDV (1-7),
SPMTIHGLDT (1-8), and
GSGWV (1-9),
a light-chain CDR1 containing the amino acid sequence represented by any one of the following formulas (J-1-1), (J-2-1), and (J-3):
KLSX^{J42}X^{J52}Y (J-1-1)
wherein
X^{J42} represents N, E, or K,
X^{J52} represents Q, R, or E,
SX^{J24}SLX^{J54}X^{J64}SDGX^{J104}TX^{J124} (J-2-1)
wherein
X^{J24} represents E or Q,
X^{J54} represents L or V,
X^{J64} represents H, D, or Y,
X^{J104} represents N, K, or Y,
X^{J124} represents Y or F, and
NIGGKA (J-3),
a light-chain CDR2 containing the amino acid sequence represented by the following formulas (K-1-1) to (K-1-7) :
NDD (K-1-1),
KDS (K-1-2),
NGN (K-1-3),
NDS (K-1-4),
KVS (K-1-5),
LIS (K-1-6), and
EVS (K-1-7), and
a light-chain CDR3 containing the amino acid sequence represented by any one of the following formulas (L-1-1), (L-2-1), (L-3-1), (L-5), and (L-6):
LSX^{L32}X^{L42}SX^{L62}DAAX^{L102}V (L-1-1)
wherein
X^{L32} represents R or S,
X^{L42} represents Q or E,
X^{L62} represents D or N,
X^{L102} represents S, R, or W,
LSGESDDAWV (L-2-1),
X^{L15}QX^{L35}THDPYT (L-3-1)
wherein
X^{L15} represents L or V,
X^{L35} represents A or T,
QVYDSSSFV (L-5), and
WQGTDFPR (L-6).

11. The antibody or fragment thereof according to claim 8, comprising
a heavy-chain CDR1 containing the amino acid sequence of any one of SEQ ID NOs: 74 to 84 or an amino acid sequence formed of any of these amino acid sequences with one to three amino acid mutations,
a heavy-chain CDR2 containing the amino acid sequence of any one of SEQ ID NOs: 85 to 101 or an amino acid sequence formed of any of these amino acid sequences with one to three amino acid mutations, or the amino acid sequence represented by NDS,
a heavy-chain CDR3 containing the amino acid sequence of any one of SEQ ID NOs: 102 to 118 or an amino acid sequence formed of any of these amino acid sequences with one to three amino acid mutations,
a light-chain CDR1 containing the amino acid sequence of any one of SEQ ID NOs: 119 to 133 or an amino acid sequence formed of any of these amino acid sequences with one to three amino acid mutations,
a light-chain CDR2 containing the amino acid sequence represented by NDD, KDS, NGN, NDS, KVS, LIS, or EVS, and
a light-chain CDR3 containing the amino acid sequence of any one of SEQ ID NOs: 134 to 149 or an amino acid sequence formed of any of these amino acid sequences with one to three amino acid mutations.

12. The antibody or fragment thereof according to claim 11, wherein the mutations are conservative substitutions.

13. The antibody or fragment thereof according to any one of claims 8 to 12, which is specifically bindable to the amino acid sequence of SEQ ID NO: 2.

14. An antibody or fragment thereof against a nucleocapsid protein of severe acute respiratory syndrome coronavirus-2 (SARS-CoV-2), whose epitope is present in a region formed of the amino acid sequence of any of SEQ ID NOs: 150 to 152 of the nucleocapsid protein.

15. The antibody or fragment thereof according to any one of claims 1 to 14, wherein the antibody is a monoclonal antibody.

16. A polynucleotide comprising a coding sequence of the antibody or fragment thereof of any one of claims 1 to 15.

17. A cell comprising the polynucleotide of claim 16.

18. A reagent or drug comprising the antibody or fragment thereof of any one of claims 1 to 15, the polynucleotide of claim 16, or the cell of claim 17.

19. A kit for detecting the nucleocapsid protein of severe acute respiratory syndrome coronavirus-2 (SARS-CoV-2), comprising the antibody or fragment thereof of any one of claims 1 to 15.

20. A kit for detecting the nucleocapsid protein of severe acute respiratory syndrome coronavirus-2 (SARS-CoV-2), comprising
two or more antibodies or fragments thereof specifically bindable to the amino acid sequence of SEQ ID NO: 1, the two or more antibodies or fragments thereof each recognizing a different epitope in the amino acid sequence of SEQ ID NO: 1, and/or
two or more antibodies or fragments thereof specifically bindable to the amino acid sequence of SEQ ID NO: 2, the two or more antibodies or fragments thereof each recognizing a different epitope in the amino acid sequence of SEQ ID NO: 2.

21. The kit according to claim 20,
wherein
the two or more antibodies or fragments thereof specifically bindable to the amino acid sequence of SEQ ID NO: 1 are each the antibody or fragment thereof of any one of claims 2 to 7 or an antibody or fragment thereof containing an amino acid sequence with at least 90% identity to the amino acid sequence of the antibody or fragment thereof of any one of claims 2 to 7, and
the two or more antibodies or fragments thereof specifically bindable to the amino acid sequence of SEQ ID NO: 2 are each the antibody or fragment thereof of any one of claims 8 to 14 or an antibody or fragment thereof containing an amino acid sequence with at least 90% identity to the amino acid sequence of the antibody or fragment thereof of any one of claims 8 to 14.

22. The kit according to any one of claims 19 to 21, for detecting the nucleocapsid protein of severe acute respiratory syndrome coronavirus-2 (SARS-CoV-2) by immunochromatography, enzyme immunoassay, chemiluminescent immunoassay, chemiluminescent enzyme immunoassay, radioimmunoassay, electrochemiluminescent immunoassay, immunonephelometry, or latex agglutination.

23. The kit according to any one of claims 19 to 22, wherein a portion of two or more antibodies or fragments thereof specifically bindable to the amino acid sequence of SEQ ID NO: 1 or 2 is immobilized, and the remaining portion is labeled.

24. A method for detecting the nucleocapsid protein of severe acute respiratory syndrome coronavirus-2 (SARS-CoV-2), comprising the following steps (1) to (3):
(1) immobilizing a primary antibody or fragment thereof specifically bindable to the amino acid sequence of SEQ ID NO: 1 or 2 onto a solid phase,
(2) applying a secondary antibody or fragment thereof that is specifically bindable to a nucleocapsid protein of SARS-CoV-2, and that is specifically bindable to the amino acid sequence of SEQ ID NO: 1 or 2 to the solid phase, wherein the secondary antibody or fragment thereof recognizes a different epitope in an amino acid sequence to which the primary antibody or fragment thereof is specifically bindable, and the secondary antibody or fragment thereof is labeled with a labeling substance, and
(3) detecting a label derived from the labeling substance of the secondary antibody or fragment thereof bound to the solid phase via the nucleocapsid protein.

25. The method according to claim 24, wherein the primary antibody or fragment thereof and the secondary antibody or fragment thereof are selected from the group consisting of the antibody or fragment thereof of any one of claims 2 to 15, and an antibody or fragment thereof containing an amino acid sequence with at least 90% identity to the amino acid sequence of the antibody or fragment thereof of any one of claims 2 to 15.
